# EUROPEAN PATENT SPECIFICATION

(11) **EP 4 648 753 B1**
(45) Date of publication and mention of the grant of the patent: **18.02.2026**
(21) Application number: 25708607.4
(22) Date of filing: 06.02.2025
(51) Int. Cl.: A61K 9/16, A61K 31/444, A61P 11/00

(54) **COMPOSITIONS FOR THE TREATMENT OF CFTR-MEDIATED DISEASES**
ZUSAMMENSETZUNGEN ZUR BEHANDLUNG VON CFTR-VERMITTELTEN ERKRANKUNGEN
COMPOSITIONS POUR LE TRAITEMENT DE MALADIES MÉDIÉES PAR CFTR

(30) Priority: 07.02.2024 US 202463550817 P
(43) Date of publication of application: 19.11.2025
(73) Proprietor: Vertex Pharmaceuticals Incorporated, Boston, MA 02210 (US)
(72) Inventor: KARKARE, Radhika, Boston, Massachusetts 02210 (US); MILLER, Jonathan M., Boston, Massachusetts 02210 (US)
(74) Representative: Carpmaels & Ransford LLP
(86) International application number: PCT/US2025/014740
(87) International publication number: WO 2025/171098

(56) References cited:
- EP-B1- 3 424 534
- WO-A1-2021/030552
- US-A1- 2018 125 838
- US-B2- 8 883 206
- ANONYMOUS: "Vertex Announces Positive Results From Pivotal Trials of Vanzacaftor/Tezacaftor/Deutivacaftor, Next-In-Class Triple Combination Treatment for Cystic Fibrosis", VERTEX, 5 February 2024 (2024-02-05), pages 1 - 7, XP093191892, Retrieved from the Internet <URL:https://investors.vrtx.com/news-releases/news-release-details/vertex-announces-positive-results-pivotal-trials> [retrieved on 20250506]
- HOPPE JORDANA E ET AL: "Vanzacaftor-tezacaftor-deutivacaftor for children aged 6-11 years with cystic fibrosis (RIDGELINE Trial VX21-121-105): an analysis from a single-arm, phase 3 trial", THE LANCET RESPIRATORY MEDICINE, 2 January 2025 (2025-01-02), pages 244 - 255, XP093275054, ISSN: 2213-2600, Retrieved from the Internet <URL:https://www.sciencedirect.com/science/article/pii/S2213260024004077?via%3Dihub> [retrieved on 20250507], DOI: 10.1016/S2213-2600(24)00407-7

## Description

This application claims the benefit of U.S. Provisional Application No. 63/550,817, filed on February 7, 2024.

The present invention relates to pharmaceutical compositions in the form of a plurality of granules suitable for pediatric administration containing calcium bis((14*S*)-8-[3-(2-{dispiro[2.0.2⁴.1³]heptan-7-yl}ethoxy)pyrazol-1-yl]-12,12-dimethyl-2,2,4-trioxo-2λ⁶-thia-3,9,11,18,23-pentaazatetracyclo[17.3.1.1¹¹,¹⁴. 0⁵,¹⁰]tetracosa-1(23),5,7,9,19,21-hexaen-3-ide), (R)-1-(2,2-difluorobenzo[*d*][1,3]dioxol-5-yl)-N-(1-(2,3-dihydroxypropyl)-6-fluoro-2-(1-hydroxy-2-methylpropan-2-yl)-1*H*-indol-5-yl)cyclopropanecarboxamide, and *N*-(2-(*tert*-butyl)-5-hydroxy-4-(2-(methyl-d₃)propan-2-yl-1,1,1,3,3,3-d₆)phenyl)-4-oxo-1,4-dihydroquinoline-3-carboxamide, including formulations of the granules, methods for manufacturing and processing the granules, and pharmaceutical compositions for use in methods for treating cystic fibrosis employing the pharmaceutical compositions.

One aspect of the invention provides granules suitable for pediatric administration comprising modulators of Cystic Fibrosis Transmembrane Conductance Regulator (CFTR). Cystic fibrosis (CF) is a recessive genetic disease that affects approximately 70,000 children and adults worldwide. Despite progress in the treatment of CF, there is no cure.

In patients with CF, mutations in CFTR endogenously expressed in respiratory epithelia lead to reduced apical anion secretion causing an imbalance in ion and fluid transport. The resulting decrease in anion transport contributes to enhanced mucus accumulation in the lung and accompanying microbial infections that ultimately cause death in CF patients. In addition to respiratory disease, CF patients typically suffer from gastrointestinal problems and pancreatic insufficiency that, if left untreated, result in death. In addition, the majority of males with cystic fibrosis are infertile, and fertility is reduced among females with cystic fibrosis.

Sequence analysis of the CFTR gene has revealed a variety of disease-causing mutations (Cutting, G. R. et al. (1990) Nature 346:366-369; Dean, M. et al. (1990) Cell 61:863:870; and Kerem, B-S. et al. (1989) Science 245:1073-1080; Kerem, B-S et al. (1990) Proc. Natl. Acad. Sci. USA 87:8447-8451). To date, greater than 2000 mutations in the CF gene have been identified; currently, the CFTR2 database contains information on only 485 of these identified mutations, with sufficient evidence to define 401 mutations as disease-causing. The most prevalent disease-causing mutation is a deletion of phenylalanine at position 508 of the CFTR amino acid sequence, and is commonly referred to as the F508del mutation. This mutation occurs in many of the cases of cystic fibrosis and is associated with severe disease.

The deletion of residue 508 in CFTR prevents the nascent protein from folding correctly. This results in the inability of the mutant protein to exit the endoplasmic reticulum (ER) and traffic to the plasma membrane. As a result, the number of CFTR channels for anion transport present in the membrane is far less than observed in cells expressing wild-type CFTR, i.e., CFTR having no mutations. In addition to impaired trafficking, the mutation results in defective channel gating. Together, the reduced number of channels in the membrane and the defective gating lead to reduced anion and fluid transport across epithelia. (Quinton, P. M. (1990), FASEB J. 4: 2709-2727). The channels that are defective because of the F508del mutation are still functional, albeit less functional than wild-type CFTR channels. (Dalemans et al. (1991), Nature Lond. 354: 526-528; Pasyk and Foskett (1995), J. Cell. Biochem. 270: 12347-50). In addition to F508del, other disease-causing mutations in CFTR that result in defective trafficking, synthesis, and/or channel gating could be up- or down-regulated to alter anion secretion and modify disease progression and/or severity.

CFTR is a cAMP/ATP-mediated anion channel that is expressed in a variety of cell types, including absorptive and secretory epithelia cells, where it regulates anion flux across the membrane, as well as the activity of other ion channels and proteins. In epithelial cells, normal functioning of CFTR is critical for the maintenance of electrolyte transport throughout the body, including respiratory and digestive tissue. CFTR is composed of approximately 1480 amino acids that encode a protein which is made up of a tandem repeat of transmembrane domains, each containing six transmembrane helices and a nucleotide binding domain. The two transmembrane domains are linked by a large, polar, regulatory (R)-domain with multiple phosphorylation sites that regulate channel activity and cellular trafficking.

Chloride transport takes place by the coordinated activity of ENaC and CFTR present on the apical membrane and the Na⁺-K⁺-ATPase pump and Cl- channels expressed on the basolateral surface of the cell. Secondary active transport of chloride from the luminal side leads to the accumulation of intracellular chloride, which can then passively leave the cell via Cl⁻channels, resulting in a vectorial transport. Arrangement of Na⁺/2Cl⁻/K⁺ co-transporter, Na⁺-K⁺-ATPase pump and the basolateral membrane K⁺ channels on the basolateral surface and CFTR on the luminal side coordinate the secretion of chloride via CFTR on the luminal side. Because water is probably never actively transported itself, its flow across epithelia depends on tiny transepithelial osmotic gradients generated by the bulk flow of sodium and chloride.

(14*S*)-8-[3-(2-{Dispiro[2.0.2.1]heptan-7-yl}ethoxy)-1*H*-pyrazol-1-yl]-12,12-dimethyl-2λ⁶-thia-3,9,11,18,23-pentaazatetracyclo [17.3.1. 1^{11,14}.0^{5,10}]tetracosa-1(22),5,7,9,19(23),20-hexaene-2,2,4-trione (vanzacaftor), calcium bis((14*S*)-8-[3-(2-{dispiro[2.0.2⁴.1³]heptan-7-yl}ethoxy)pyrazol-1-yl]-12,12-dimethyl-2,2,4-trioxo-2λ⁶-thia-3,9,11,18,23-pentaazatetracyclo[17.3.1.1^{11,14}.0^{5,10}]tetracosa-1(23),5,7,9,19,21-hexaen-3-ide) (vanzacaftor calcium salt), and (*R*)-1-(2,2-difluorobenzo[d][1,3]dioxol-5-yl)-N-(1-(2,3-dihydroxypropyl)-6-fluoro-2-(1-hydroxy-2-methylpropan-2-yl)-1*H*-indol-5-yl)cyclopropanecarboxamide (tezacaftor) are potent and selective CFTR correctors for treating mutant forms of human CFTR-mediated diseases, such as cystic fibrosis. *N*-(2-(*tert*-butyl)-5-hydroxy-4-(2-(methyl-d₃)propan-2-yl-1,1,1,3,3,3-d₆)phenyl)-4-oxo-1,4-dihydroquinoline-3-carboxamide (deutivacaftor) is a potent and selective CFTR potentiator of wild-type and mutant forms of human CFTR and is useful in treating cystic fibrosis.

Cystic fibrosis may be treated by a combination of vanzacaftor, tezacaftor and deutivacaftor (Anonymous: "Vertex Announces Positive Results From Pivotal Trials of Vanzacaftor/Tezacaftor/Deutivacaftor, Next-In-Class Triple Combination Treatment for Cystic Fibrosis", Vertex, 5 February 2024, pages 1-7, URL: https://investors.vrtx.com/news-releases/news-release-details/vertex-announces-positive-results-pivotal-trials).

Pediatric CF patients may require administration of pharmaceutical compositions in a dosage form that facilitates swallowing or that may be easily mixed with easily digested foods. The use of powders and crushed tablets in the administration of pharmaceutical compositions to children has often presented problems in administration and dosing. Some challenges associated with the development of pediatric formulations comprising CFTR modulators are summarized below.

### Ease of administration

Tablets and capsules are very often difficult for small children to swallow. Therefore, alternative formulations that are easier to swallow such as, e.g., liquid formulations or suspensions, may facilitate administration. However, the feasibility of liquid formulations and suspensions is limited when the aqueous solubility of the active pharmaceutical ingredient is low.

### Accuracy of dosing

Administering crushed tablet formulations to children can lead to absorption problems, fragments that are either too difficult to swallow or fail to solubilize and remain undigested resulting in therapeutic failure or dosage inaccuracies. Additionally, the dosing of crushed tablets can lead to dosing inaccuracies because of difficulties associated with the handling of crushed tablets. The use of powder blends may also result in dosage inaccuracies. In other instances, active powder agents may remain adhered to the interior walls of a capsule, pouch, or packet at the time of administration, resulting in less than the required therapeutic dosage. Such dosing inaccuracies are particularly prevalent when the person administering the dose is inexperienced and when the dose is small, as in those used to treat pediatric patients. Dosage errors involving CF pharmaceutical active agents therefore become critical in pediatric populations, particularly considering that pharmaceutical CF active agents are administered in low doses (e.g., less than 100 mg or less than 50 mg per unit dose). These dosing inaccuracies become critical in pediatric patients having a low threshold for dose deviation. The dose for the pharmaceutical compounds of the invention per administration is very low (the total unit dose of vanzacaftor is at or below about 12 mg, e.g., the total unit dose is about 1 mg, about 2 mg, about 4 mg, about 6 mg, about 8 mg, about 10 mg, or about 12 mg). Administering a low dose of the active pharmaceutical ingredient(s) as a plurality of granules exacerbates the potential for dosing inaccuracies, because each individual granule must contain a specific fraction of the total unit dose. Thus, there is a need to develop pharmaceutical compositions that provide a consistent dose of the very small amount of the medicine with minimal deviation.

### Manufacturing reproducibility

During the manufacturing of compressed solid dosage forms such as granules, physical force is used to eject the dosage form from a die. The composition needs to be formulated such that the granules are hard enough to withstand the ejection forces without breaking. High ejection forces arise when there is a large amount of friction between the compressed dosage form and the walls of the die. They are undesirable because high ejection forces can result in damage to the dosage form during ejection from the die and can also cause premature wear of the compression equipment (which itself could lead to reproducibility problems).

### Dispersibility

A potential method of administering CFTR modulating compounds to pediatric patient is by mixing the dosage form with liquids or soft foods. For this to be feasible, the dosage form must be soft enough to quickly disintegrate and disperse within the mouth or the administration medium.

### Palatability

Palatability (e.g., aroma, taste, texture, and mouthfeel) is an extremely important factor in attaining acceptable dosing compliance of pediatric formulations. CFTR modulators may exhibit an unpleasant or bitter taste. For example, ivacaftor, a CFTR modulator, has a strong bitter taste. Thus, a formulation containing specific amounts of sweeteners and/or flavorings to mask this unpleasant or bitter taste is often required to achieve satisfactory patient compliance. At the same time, sweeteners or blends of sweeteners may impart an undesirable mouthfeel. For example, blends of mannitol and sucrose with aspartame have been found to have a "gritty" texture rendering them unsuitable for pediatric formulations with ivacaftor, despite their favorable flavor profiles.

### Physical and chemical stability

Physical and chemical stability of the pharmaceutical granule composition depends greatly on the specific formulation employed. If the composition is not appropriately formulated, this can lead to undesirable chemical or physical changes such as, for example, the conversion of the active pharmaceutical ingredient into a less bioavailable solid form.

### Bioavailability

All the above factors must be optimized without compromising bioavailability. Bioavailability is highly unpredictable and any changes in the formulation could unexpectedly lead to a reduction in bioavailability.

Accordingly, there is a need for pharmaceutical granule compositions of vanzacaftor, tezacaftor, and deutivacaftor useful for treating pediatric patients with CF where the compositions are physically and chemically stable, bioavailable, consistently deliver the specified doses present in very small amounts, all while being hard enough to be manufactured consistently, and while being able to disperse quickly to provide acceptable dissolution and bioavailability upon administration. The compositions of the invention have been developed to balance all of these factors and meet all of these requirements.

There is a need for stable, bioavailable pharmaceutical granule compositions of vanzacaftor, tezacaftor, and deutivacaftor useful for treating a particular population with an unmet medical need, such as children 12 to 24 months and 2 to 5 years of age, who cannot swallow adult tablets.

There is a need for stable, bioavailable pharmaceutical granule compositions of vanzacaftor, tezacaftor, and deutivacaftor that allow for accurate and flexible dosing in pediatric patients 12 to 24 months and 2 to 5 years of age by changing the number of granules in the unit dose, packet, pouch, or capsule.

### SUMMARY

The present invention relates to pharmaceutical granule compositions comprising vanzacaftor, tezacaftor, and deutivacaftor, and methods of manufacturing and administering pharmaceutical compositions comprising vanzacaftor, tezacaftor, and deutivacaftor. The pharmaceutical granule compositions comprising a vanzacaftor, tezacaftor, and deutivacaftor may also include one or more of the following excipients: one or more fillers, a sweetener, a disintegrant, a wetting agent, a glidant, and a lubricant.

In accordance with the invention, as described in the claims, provided is a pharmaceutical composition, formulated into a unit dosage form comprising a plurality of granules, wherein each of the granules comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant; and
wherein the unit dosage form comprises crystalline vanzacaftor calcium salt hydrate Form D in an amount ranging from 1 mg to 12.7 mg, amorphous tezacaftor in an amount ranging from 10 mg to 50 mg, and amorphous deutivacaftor in an amount ranging from 15 mg to 150 mg.

Another aspect of the present invention provides a pharmaceutical composition as described in the claims for use in a method of treating or lessening the severity of CFTR-mediated disease in a pediatric patient, wherein the CFTR mediated disease is cystic fibrosis.

References to methods of treatment in this application should be interpreted as referring to the pharmaceutical compositions, as described in the claims, for use in the methods of treatment.

The pharmaceutical granule compositions disclosed herein can be reproducibly manufactured and have been found to enable accurate dosing of pediatric patients at unit doses of about 12 mg or less (e.g., a unit dose of about 1 mg, about 2 mg, about 4 mg, about 6 mg, about 8 mg, about 10 mg, or about 12 mg) of vanzacaftor, while maintaining a desirable level of palatability and ease of administration. The pharmaceutical granule compositions disclosed herein have also been found to have beneficial properties such as improved dispersibility, physical and chemical stability, and a high level of bioavailability.

The pharmaceutical compositions of the present invention are formulated into a plurality of granules. The granules can be contained in capsules, pouches, packets, sachets, bottles, or blister packs to provide a unit dosage form. The granules can also be compressed into other solid forms such as pellets, beads, particles, particulates, troches, or other comparable dosage forms. In one embodiment, the pharmaceutical granule compositions described herein contain: (a) crystalline vanzacaftor calcium salt hydrate Form D, a solid dispersion comprising tezacaftor, and a solid dispersion comprising deutivacaftor, and (b) one or more pharmaceutically acceptable excipients (for example, one or more fillers, a sweetener, a disintegrant, optionally a wetting agent, a glidant, and a lubricant), wherein a plurality of the granules is contained within a unit dosage form, and further wherein the unit dosage form contains a unit dose, i.e., a specified amount, of vanzacaftor calcium salt hydrate Form D, ranging from at least about 1 mg to at least about 12.7 mg per unit dosage form, amorphous tezacaftor, ranging from about 10 mg to about 50 mg per unit dosage form, and amorphous deutivacaftor, ranging from about 15 mg to about 150 mg per unit dosage form.

In one embodiment, the present invention provides a unit dosage form of a pharmaceutical composition comprising a plurality of granules formulated to deliver about 0.1 mg to about 12.7 mg (e.g., about 1 mg, about 2.1 mg, about 3 mg, about 4.2 mg, about 6 mg, about 6.4 mg, about 8 mg, about 8.5 mg, about 10 mg, about 10.6 mg, or about 12.7 mg) of crystalline vanzacaftor calcium salt hydrate Form D, a solid dispersion comprising about 10 mg to about 50 mg (e.g., about 10 mg, about 16 mg, about 24 mg, about 32 mg, or about 48 mg) of amorphous tezacaftor, and a solid dispersion comprising about 40 mg to about 150 mg (e.g., about 50 mg, about 75 mg, about 100 mg, or about 150 mg) of amorphous deutivacaftor. In certain embodiments, the present invention provides a pharmaceutical composition formulated to deliver a unit dosage form comprising about 4.2 mg of crystalline vanzacaftor calcium salt hydrate Form D, about 16 mg of amorphous tezacaftor, and about 50 mg of amorphous deutivacaftor. In certain embodiments, the present invention provides a pharmaceutical composition formulated to deliver a unit dosage form comprising about 6.4 mg of crystalline vanzacaftor calcium salt hydrate Form D, about 24 mg of amorphous tezacaftor, and about 75 mg of amorphous deutivacaftor. In certain embodiments, the present invention provides a pharmaceutical composition formulated to deliver a unit dosage form comprising about 8.5 mg of crystalline vanzacaftor calcium salt hydrate Form D, about 32 mg of amorphous tezacaftor, and about 100 mg of amorphous deutivacaftor. In certain embodiments, the present invention provides a pharmaceutical composition formulated to deliver a unit dosage form comprising about 12.7 mg of crystalline vanzacaftor calcium salt hydrate Form D, about 48 mg of amorphous tezacaftor, and about 150 mg of amorphous deutivacaftor.

In some embodiments, the pharmaceutical granule compositions disclosed herein comprise a first solid dispersion comprising tezacaftor and a second solid dispersion comprising deutivacaftor. In some embodiments one or both solid dispersions are spray-dried dispersions. In some embodiments, the solid dispersions and the spray dried dispersions of the disclosure can comprise excipients, such as polymers and/or surfactants.

In some embodiments, sodium lauryl sulfate (SLS) is used as a surfactant in the solid dispersion of deutivacaftor. The amount of the surfactant (e.g., SLS) relative to the total weight of the solid dispersion may be between 0.1% w/w and 15% w/w. For example, it is from 0.5% w/w to 10% w/w, such as from 0.5% w/w to 5% w/w, e.g., 0.5% w/w to 4% w/w, 0.5% w/w to 3% w/w, 0.5% w/w to 2% w/w, 0.5% w/w to 1% w/w, or 0.5% w/w. In certain embodiments, the amount of the surfactant relative to the total weight of the solid dispersion is at least 0.1% w/w or at least 0.5% w/w. The amount of the surfactant (e.g., SLS) relative to the total weight of the solid dispersion may also be between about 0.1% w/w and about 15% w/w. For example, it is from about 0.5% w/w to about 10% w/w, such as from about 0.5% w/w to about 5% w/w, e.g., about 0.5% w/w to about 4% w/w, about 0.5% w/w to about 3% w/w, about 0.5% w/w to about 2% w/w, about 0.5% w/w to about 1% w/w, or about 0.5% w/w. In certain embodiments, the amount of the surfactant relative to the total weight of the solid dispersion is at least about 0.1% w/w or at least about 0.5% w/w. In some embodiments, the surfactant is SLS. In some embodiments, the solid dispersion of deutivacaftor comprises about 0.5% SLS by weight of the solid dispersion of deutivacaftor.

In some embodiments, tezacaftor or deutivacaftor is present in an amount greater than half of the percentage weight of the dispersions. In some embodiments, a first solid dispersion comprises from about 70 wt% to about 90 wt% (e.g., from about 75 wt% to about 85 wt%) of tezacaftor. In some embodiments, a second solid dispersion comprises from about 70 wt% to about 90 wt% (e.g., from about 75 wt% to about 85 wt%) of deutivacaftor. For example, in some embodiments, in a solid dispersion comprising tezacaftor, the polymer is present in about 20 wt% by weight of the solid dispersion and amorphous tezacaftor is present in about 80 wt% by weight of the solid dispersion.

In one embodiment, the solid dispersion of tezacaftor comprises about 80 wt% of amorphous tezacaftor by weight of the solid dispersion, and about 20 wt% of HPMC by weight of the solid dispersion.

In one embodiment, the solid dispersion of deutivacaftor comprises about 80 wt% of amorphous deutivacaftor by weight of the solid dispersion, and about 19.5 wt% of HPMCAS by weight of the solid dispersion, and about 0.5 wt% SLS by weight of the dispersion.

In some embodiments, each of the first and second solid dispersions independently comprise a plurality of particles having a mean particle diameter of 5 to 100 microns. In some embodiments, each of the first and second solid dispersions independently comprise a plurality of particles having a mean particle diameter of 15 to 40 microns. In some embodiments, each of the first and second solid dispersions independently comprise a plurality of particles having a mean particle diameter of 15 microns. In some embodiments, each of the first and second solid dispersions independently comprise a plurality of particles having a mean particle diameter of about 5 to about 100 microns. In some embodiments, each of the first and second solid dispersions independently comprise a plurality of particles having a mean particle diameter of about 15 to 40 about microns. In some embodiments, each of the first and second solid dispersions independently comprise a plurality of particles having a mean particle diameter of about 15 microns.

In still further embodiments, in addition to the active pharmaceutical agents (APIs), the pharmaceutical compositions disclosed herein also comprise one or more fillers (e.g., mannitol, celluloses, calcium carbonate, starches, sugars (e.g., dextrose, lactose, or the like)) in concentrations of at least about 10 wt% (e.g., about 10 to about 50 wt%, about 20 to about 60 wt%, about 30 to about 70 wt%), by weight of the composition; a sweetener (e.g., sucralose, sorbitol, saccharin, fructose, aspartame, or a combination thereof) in a concentration of about 10 wt% or less (e.g., about 0 to about 10 wt%, about 0 to about 5 wt%, about 0 to about 3 wt%) by weight of this composition; a disintegrant (e.g., croscarmellose sodium, sodium starch glycolate, or a combination thereof) in concentrations of about 10 wt% or less (e.g., about 0 to about 10 wt%, about 0 to about 8 wt%, about 0 to about 6 wt%) by weight of the composition; optionally a wetting agent (e.g., sodium lauryl sulfate, SLS) in concentrations of about 10 wt% or less (e.g., about 0 to about 10 wt%, about 0 to about 5 wt%, about 0 to about 2 wt%) by weight of the composition; a glidant (e.g., colloidal silicon dioxide, talc, or a combination thereof) in concentrations of about 3 wt% or less (e.g., about 0 to about 3 wt%, about 0 to about 2 wt%, about 0 to about 1 wt%) by weight of the composition; and a lubricant (e.g., magnesium stearate, stearic acid, hydrogenated oil, sodium stearyl fumarate, or any combination thereof) in concentrations of about 3 wt% or less (e.g., about 0 to about 3 wt%, about 0 to about 2 wt%, about 0 to about 1 wt%) by weight of the composition.

Such pharmaceutical compositions can optionally comprise one or more colorants, fragrances, and/or flavors to enhance its visual appeal, taste, and scent.

In one aspect, the invention includes a pharmaceutical composition comprising crystalline vanzacaftor calcium salt hydrate Form D, a first solid dispersion comprising amorphous tezacaftor, a second solid dispersion comprising amorphous deutivacaftor, one or more fillers, a sweetener, a disintegrant, a glidant and a lubricant, and optionally a wetting agent.

In some embodiments, the filler comprises mannitol which is present in an amount from about 10 to about 45 percent by weight of the composition. In some embodiments, the filler comprises lactose in an amount from about 25 to about 45 percent by weight of the composition. In some embodiments, the filler is binary, comprising both mannitol and lactose. In some embodiments, the binary filler comprises mannitol which is present in an amount from about 10 to about 45 percent (e.g., about 10 to about 15 percent, about 11 to about 13 percent, about 12 percent, about 12.5 percent) by weight of the composition, and lactose which is present in an amount from about 25 to about 45 percent (e.g., about 30 to about 40 percent, about 34 to about 38 percent, about 35 percent, about 35.5 percent, about 36 percent) by weight of the composition. In some embodiments, the binary filler comprises mannitol and lactose in a ratio of about 1 to 5 by weight. In some embodiments, the binary filler comprises mannitol and lactose in a ratio of about 1 to 4 by weight. In some embodiments, the binary filler comprises mannitol and lactose in a ratio of about 1 to 3 by weight. In some embodiments, the binary filler comprises mannitol and lactose in a ratio of about 1 to 2 by weight. In some embodiments, the binary filler comprises mannitol and lactose in a ratio of about 1 to 1.5 by weight. In some embodiments, the binary filler comprises mannitol and lactose in a ratio of about 1 to about 5 by weight. In some embodiments, the binary filler comprises mannitol and lactose in a ratio of about 1 to about 4 by weight. In some embodiments, the binary filler comprises mannitol and lactose in a ratio of about 1 to about 3 by weight. In some embodiments, the binary filler comprises mannitol and lactose in a ratio of about 1 to about 2 by weight. In some embodiments, the binary filler comprises mannitol and lactose in a ratio of about 1 to about 1.5 by weight.

In one embodiment, the sweetener comprises sucralose which is present in an amount from about 0.1 to about 5 percent (e.g., about 1 to about 4 percent, about 2 to about 3 percent, about 2 percent, about 2.2 percent, about 2.3 percent, about 2.4 percent) by weight of the composition.

In one embodiment, the disintegrant comprises croscarmellose sodium which is present in an amount from about 1 to about 8 percent (e.g., about 2 to about 7.5 percent, about 3.5 to about 7 percent, about 5 to about 6.5 percent) by weight of the composition.

In one embodiment, the wetting agent comprises sodium lauryl sulfate which is present in an amount of about 2 or less percent (e.g., about 0 to about 2 percent, about 0 to about 1 percent) by weight of the composition.

In one embodiment, the glidant comprises: talc, colloidal silica (i.e., colloidal silicon dioxide), precipitated silica, magnesium oxide, magnesium silicate, leucine, and starch. In a further embodiment, the glidant comprises colloidal silica which is present in an amount from about 0.1 to about 5 percent (e.g., about 0.1 to about 4 percent, about 0.5 to about 3 percent, about 0.5 percent, about 1 percent, about 1.5 percent) by weight of the composition.

In one embodiment, the lubricant comprises magnesium stearate which is present in an amount from about 0.5 to about 2.0 percent (e.g., about 0.75 to about 1.25 percent, about 0.5 to about 1.5 percent, about 0.5 percent, about 1 percent, about 1.5 percent, about 2 percent) by weight of the composition.

The present invention contemplates stable, solid dosage forms such as granules which overcome the problems described above with respect to dosing inaccuracies, in particular, for pediatric patients. These stable, solid unit dose forms can have any shape, including oval, spherical, cylindrical, elliptical, cubical, square, or rectangular among others. Granules may have flat, shallow, standard, deep convex, or double deep convex faces or combinations thereof.

In one aspect, the pharmaceutical granule composition can be formulated into a unit dosage form, for example, a capsule, a sachet, and the like, containing at least one or more granules to simplify the administration of the pharmaceutical composition. In some embodiments, the unit dosage form can include a capsule or a packet containing at least one granule, or a plurality of granules. In another embodiment, the unit dose can include a pouch, a packet or sachet containing a specific dose of crystalline vanzacaftor calcium salt hydrate Form D, a solid dispersion comprising amorphous tezacaftor, and a solid dispersion comprising deutivacaftor, in granular form.

Such pharmaceutical compositions as described herein can be in the form of a plurality of granules made up of any number of granules (e.g., at least 10, at least 20, at least 40, at least 60, at least 80, at least 90, at least 100, or any number greater than 100, at least about 10, at least about 20, at least about 40, at least about 60, at least about 80, at least about 90, at least about 100, or any number greater than about 100, inclusive of all of the ranges in between). In one embodiment, the plurality of granules contains about 20 to about 40 (e.g., about 25, about 30, about 35) granules. In one embodiment, the plurality of granules contains 30 granules. In one embodiment, the plurality of granules contains about 30 granules. Thus, pharmaceutical compositions as described herein can be in the form of an individual granule, or a plurality of granules.

In one embodiment, the plurality of granules contains 30 granules, wherein the plurality of granules contains a unit dose of 4 mg of vanzacaftor. In one embodiment, the plurality of granules contains 45 granules, wherein the plurality of granules contains a unit dose of 6 mg of vanzacaftor. In one embodiment, the plurality of granules contains 60 granules, wherein the plurality of granules contains a unit dose of 8 mg of vanzacaftor. In one embodiment, the plurality of granules contains 90 granules, wherein the plurality of granules contains a unit dose of 12 mg of vanzacaftor. In one embodiment, the plurality of granules contains about 30 granules, wherein the plurality of granules contains a unit dose of about 4 mg of vanzacafter. In one embodiment, the plurality of granules contains about 45 granules, wherein the plurality of granules contains a unit dose of about 6 mg of vanzacaftor. In one embodiment, the plurality of granules contains about 60 granules, wherein the plurality of granules contains a unit dose of about 8 mg of vanzacaftor. In one embodiment, the plurality of granules contains about 90 granules, wherein the plurality of granules contains a unit dose of about 12 mg of vanzacaftor.

In another aspect, the granules described herein are colored, such as by incorporating a colorant in the granule formulation or by coloring the surface of the granule.

In other embodiments, the pharmaceutical composition comprising a plurality of granules can be in pouches, sachets, packets, bottles, or blister packs, or optionally further compressed into different solid unit dose forms that can be easily administered to patients that have difficulty in swallowing adult-sized tablet formulations. As such, these novel pharmaceutical compositions and unit dose forms containing said pharmaceutical compositions are organoleptically acceptable to said patients, are sprinkled into liquids or soft food and disintegrated or dispersed in those various liquids and soft foods or food compositions such as milk (including breast milk), baby formula or infant formula, apple sauce, water, plain yogurt, ice cream, or baby food, ensuring that the entire prescribed dose has been disintegrated or dispersed and are capable of administration to patients having difficulty swallowing adult tablets. Baby food includes, but is not limited to, carrots or carrot puree. The pharmaceutical composition can also be administered in strawberry preserves, rice pudding, chocolate pudding and the like. In one embodiment, the unit dose form is sprinkled into soft food and administered. In another embodiment, the unit dose form is sprinkled into liquid and administered. In one embodiment, the unit dose form is sprinkled into soft food, mixed, and administered. In another embodiment, the unit dose form is sprinkled into liquid, mixed, and administered. Liquids may include, but are not limited to, baby formula, infant formula, milk, or breast milk. In some instances, for smaller sized granules, the contents of packets, pouches, capsules, bottles, or sachets may be administered directly to the mouth followed by breast milk or formula. Methods of administration of the present invention can optionally also include, for smaller sized granules, administering the contents of packets, pouches, capsules, bottles, or sachets directly to the mouth followed by a liquid or beverage. In some embodiments, any methods of administration of the present invention can optionally include orally administering with fat-containing food such as a standard CF high-calorie, high-fat meal or snack. In other embodiments, any methods of administration of the present invention can optionally include orally administering concurrently with, before, or after fat-containing food such as a standard CF high-calorie, high-fat meal or snack. In one embodiment, the pharmaceutical compositions of the present invention and solid unit dose forms thereof find particular utility in the treatment of CFTR mediated disease in the pediatric patient population.

In one embodiment, the pharmaceutical composition is a unit dose form comprising a plurality of granules, and wherein the unit dose form comprises from about 1 mg to about 12.7 mg of crystalline vanzacaftor calcium salt hydrate Form D. In some embodiments, the pharmaceutical composition is a unit dose form comprising a plurality of granules, and wherein the unit dose form comprises about 4.2 mg of crystalline vanzacaftor calcium salt hydrate Form D. In some embodiments, the pharmaceutical composition is a unit dose form comprising a plurality of granules, and wherein the unit dose form comprises about 6.4 mg of crystalline vanzacaftor calcium salt hydrate Form D. In some embodiments, the pharmaceutical composition is a unit dose form comprising a plurality of granules, and wherein the unit dose form comprises about 8.5 mg of crystalline vanzacaftor calcium salt hydrate Form D. In some embodiments, the pharmaceutical composition is a unit dose form comprising a plurality of granules, and wherein the unit dose form comprises about 12.7 mg of crystalline vanzacaftor calcium salt hydrate Form D.

In one embodiment, the pharmaceutical composition is a unit dose form comprising a plurality of granules, and wherein the unit dose form comprises from about 10 mg to about 50 mg of amorphous or substantially amorphous tezacaftor. In some embodiments, the pharmaceutical composition is a unit dose form comprising a plurality of granules, and wherein the unit dose form comprises about 16 mg of amorphous or substantially amorphous tezacaftor. In some embodiments, the pharmaceutical composition is a unit dose form comprising a plurality of granules, and wherein the unit dose form comprises about 24 mg of amorphous or substantially amorphous tezacaftor. In some embodiments, the pharmaceutical composition is a unit dose form comprising a plurality of granules, and wherein the unit dose form comprises about 32 mg of amorphous or substantially amorphous tezacaftor. In some embodiments, the pharmaceutical composition is a unit dose form comprising a plurality of granules, and wherein the unit dose form comprises about 48 mg of amorphous or substantially amorphous tezacaftor.

In one embodiment, the pharmaceutical composition is a unit dose form comprising a plurality of granules, and wherein the unit dose form comprises from about 15 mg to about 150 mg of amorphous or substantially amorphous deutivacaftor. In some embodiments, the pharmaceutical composition is a unit dose form comprising a plurality of granules, and wherein the unit dose form comprises about 50 mg of amorphous or substantially amorphous deutivacaftor. In some embodiments, the pharmaceutical composition is a unit dose form comprising a plurality of granules, and wherein the unit dose form comprises about 75 mg of amorphous or substantially amorphous deutivacaftor. In some embodiments, the pharmaceutical composition is a unit dose form comprising a plurality of granules, and wherein the unit dose form comprises about 100 mg of amorphous or substantially amorphous deutivacaftor. In some embodiments, the pharmaceutical composition is a unit dose form comprising a plurality of granules, and wherein the unit dose form comprises about 150 mg of amorphous or substantially amorphous deutivacaftor.

In another embodiment, the solid dispersion(s) comprising tezacaftor and deutivacaftor are present in the pharmaceutical composition of the invention in a combined amount of about 20 to about 50 percent by weight of the pharmaceutical composition and the unit dose form is a granule having a shape that is cylinder-like, oval-like, cone-like, sphere-like, ellipsis-like, polygon-like, or combinations thereof, wherein the granule has as its longest dimension or diameter a length of from about 1.5 mm to about 4.0 mm.

In a further embodiment, the combined solid dispersions are present in an amount of about 20 to about 50 percent by weight of the pharmaceutical composition and the unit dose form is a granule having a shape that is cylinder-like, oval-like, cone-like, sphere-like, ellipsis-like, polygon-like or combinations thereof, wherein the granule has as its longest dimension or diameter a length of about 2.0 mm.

In some embodiments, individual granules are about 5.0 mg to about 10 mg in weight. In some embodiments, individual granules are about 6.0 mg to about 9.0 mg in weight. In some embodiments, individual granules are about 6.5 mg to about 8.0 mg in weight. In some embodiments, each granule is about 7.0 mg in weight. In some embodiments, each granule is about 2.0 mm in diameter and about 7.0 mg in weight.

Another aspect of the present invention provides a method of administering a pharmaceutical composition by orally administering to a patient, for example, a human pediatric patient, once per day, a plurality of granules, comprising crystalline vanzacaftor calcium salt hydrate Form D, a solid dispersion comprising amorphous tezacaftor, a solid dispersion comprising amorphous deutivacaftor, one or more fillers, a sweetener, a disintegrant, optionally a wetting agent, a glidant, and a lubricant, wherein the unit dose comprises about 4.2 mg of crystalline vanzacaftor calcium salt hydrate Form D, about 16 mg of substantially amorphous or amorphous tezacaftor, and about 50 mg of amorphous or substantially amorphous deutivacaftor.

Another aspect of the present invention provides a method of administering a pharmaceutical composition by orally administering to a patient, for example, a human pediatric patient, once per day, a plurality of granules, comprising crystalline vanzacaftor calcium salt hydrate Form D, a solid dispersion comprising amorphous tezacaftor, a solid dispersion comprising amorphous deutivacaftor, one or more fillers, a sweetener, a disintegrant, optionally a wetting agent, a glidant, and a lubricant, wherein the unit dose comprises about 6.4 mg of crystalline vanzacaftor calcium salt hydrate Form D, about 24 mg of substantially amorphous or amorphous tezacaftor, and about 75 mg of amorphous or substantially amorphous deutivacaftor.

Another aspect of the present invention provides a method of administering a pharmaceutical composition by orally administering to a patient, for example, a human pediatric patient, once per day, a plurality of granules, comprising crystalline vanzacaftor calcium salt hydrate Form D, a solid dispersion comprising amorphous tezacaftor, a solid dispersion comprising amorphous deutivacaftor, one or more fillers, a sweetener, a disintegrant, optionally a wetting agent, a glidant, and a lubricant, wherein the unit dose comprises about 8.5 mg of crystalline vanzacaftor calcium salt hydrate Form D, about 32 mg of substantially amorphous or amorphous tezacaftor, and about 100 mg of amorphous or substantially amorphous deutivacaftor.

Another aspect of the present invention provides a method of administering a pharmaceutical composition by orally administering to a patient, for example, a human pediatric patient, once per day, a plurality of granules, comprising crystalline vanzacaftor calcium salt hydrate Form D, a solid dispersion comprising amorphous tezacaftor, a solid dispersion comprising amorphous deutivacaftor, one or more fillers, a sweetener, a disintegrant, optionally a wetting agent, a glidant, and a lubricant, wherein the unit dose comprises about 12.7 mg of crystalline vanzacaftor calcium salt hydrate Form D, about 48 mg of substantially amorphous or amorphous tezacaftor, and about 150 mg of amorphous or substantially amorphous deutivacaftor.

Another aspect of the present invention provides a method of administering a pharmaceutical composition by orally administering to a patient, for example, a human pediatric patient, once per day, a plurality of granules, comprising crystalline vanzacaftor calcium salt hydrate Form D, a solid dispersion comprising substantially amorphous or amorphous tezacaftor, a solid dispersion comprising substantially amorphous or amorphous deutivacaftor, one or more fillers, a sweetener, a disintegrant, optionally a wetting agent, a glidant, and a lubricant, wherein the unit dose comprises about 4.2 mg of crystalline vanzacaftor calcium salt hydrate Form D, about 16 mg of substantially amorphous or amorphous tezacaftor, and about 50 mg of amorphous or substantially amorphous deutivacaftor.

Another aspect of the present invention provides a method of administering a pharmaceutical composition by orally administering to a patient, for example, a human pediatric patient, once per day, a plurality of granules, comprising crystalline vanzacaftor calcium salt hydrate Form D, a solid dispersion comprising substantially amorphous or amorphous tezacaftor, a solid dispersion comprising substantially amorphous or amorphous deutivacaftor, one or more fillers, a sweetener, a disintegrant, optionally a wetting agent, a glidant, and a lubricant, wherein the unit dose comprises about 6.4 mg of crystalline vanzacaftor calcium salt hydrate Form D, about 24 mg of substantially amorphous or amorphous tezacaftor, and about 75 mg of amorphous or substantially amorphous deutivacaftor.

Another aspect of the present invention provides a method of administering a pharmaceutical composition by orally administering to a patient, for example, a human pediatric patient, once per day, a plurality of granules, comprising crystalline vanzacaftor calcium salt hydrate Form D, a solid dispersion comprising substantially amorphous or amorphous tezacaftor, a solid dispersion comprising substantially amorphous or amorphous deutivacaftor, one or more fillers, a sweetener, a disintegrant, optionally a wetting agent, a glidant, and a lubricant, wherein the unit dose comprises about 8.5 mg of crystalline vanzacaftor calcium salt hydrate Form D, about 32 mg of substantially amorphous or amorphous tezacaftor, and about 100 mg of amorphous or substantially amorphous deutivacaftor.

Another aspect of the present invention provides a method of administering a pharmaceutical composition by orally administering to a patient, for example, a human pediatric patient, once per day, a plurality of granules, comprising crystalline vanzacaftor calcium salt hydrate Form D, a solid dispersion comprising substantially amorphous or amorphous tezacaftor, a solid dispersion comprising substantially amorphous or amorphous deutivacaftor, one or more fillers, a sweetener, a disintegrant, optionally a wetting agent, a glidant, and a lubricant, wherein the unit dose comprises about 12.7 mg of crystalline vanzacaftor calcium salt hydrate Form D, about 48 mg of substantially amorphous or amorphous tezacaftor, and about 150 mg of amorphous or substantially amorphous deutivacaftor.

### DEFINITIONS

As used herein, the term "active pharmaceutical ingredient" or "API" refers to a biologically active compound. APIs employed in the pharmaceutical compositions of the invention include two CF corrector compounds, calcium bis((14*S*)-8-[3-(2-{dispiro[2.0.2⁴.1³]heptan-7-yl}ethoxy)pyrazol-1-yl]-12,12-dimethyl-2,2,4-trioxo-2λ⁶-thia-3,9,11,18,23-pentaazatetracyclo[17.3.1.1^{11,14}.0^{5,10}]tetracosa-1(23),5,7,9,19,21-hexaen-3-ide), (vanzacaftor calcium salt) and (*R*)-1-(2,2-difluorobenzo[*d*][1,3]dioxol-5-yl)-*N*-(1-(2,3-dihydroxypropyl)-6-fluoro-2-(1-hydroxy-2-methylpropan-2-yl)-1*H*-indol-5-yl)cyclopropanecarboxamide (tezacaftor), and a CF potentiator, *N*-(2-(*tert*-butyl)-5-hydroxy-4-(2-(methyl-d₃)propan-2-yl-1,1,1,3,3,3-d₆)phenyl)-4-oxo-1,4-dihydroquinoline-3-carboxamide (deutivacaftor).

### Vanzacaftor

As used herein, the term "vanzacaftor" is used interchangeably with (14*S*)-8-[3-(2-{dispiro[2.0.2.1]heptan-7-yl}ethoxy)-1*H*-pyrazol-1-yl]-12,12-dimethyl-2λ⁶-thia-3,9,11,18,23-pentaazatetracyclo[17.3.1.1^{11,14}.0^{5,10}]tetracosa-1(22),5,7,9,19(23),20-hexaene-2,2,4-trione, which has the following structure:

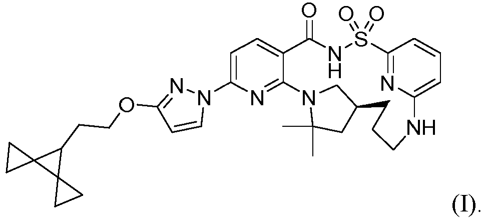

"Vanzacaftor calcium salt" as used herein refers to calcium bis((14*S*)-8-[3-(2-{dispiro[2.0.2⁴.1³]heptan-7-yl}ethoxy)pyrazol-1-yl]-12,12-dimethyl-2,2,4-trioxo-2λ⁶-thia-3,9,11,18,23-pentaazatetracyclo[17.3.1.1^{11,14}.0⁵,¹⁰]tetracosa-1(23),5,7,9,19,21-hexaen-3-ide), a calcium salt of vanzacaftor.

Vanzacaftor is described in International Patent Publications WO 2019/161078, WO 2020/102346, WO 2021/030554, WO 2021/030552, WO 2022/036060, WO 2022/125826,. These publications also describe methods of making vanzacaftor, and demonstrate that vanzacaftor is a CFTR corrector therapeutic. WO 2021/030552 also describes methods of making vanzacaftor calcium salt hydrate Form D.

In some embodiments, vanzacaftor calcium salt hydrate Form D is characterized by an X-ray powder diffractogram having signals at 6.1 ± 0.2 degrees two-theta, 16.2 ± 0.2 degrees two-theta, and 22.8 ± 0.2 degrees two-theta. In some embodiments, vanzacaftor calcium salt hydrate Form D is characterized by an X-ray powder diffractogram having (a) signals at 6.1 ± 0.2 degrees two-theta, 16.2 ± 0.2 degrees two-theta, and 22.8 ± 0.2 degrees two-theta; and (b) one or more signals selected from 5.5 ± 0.2 degrees two-theta, 15.5 ± 0.2 degrees two-theta, 19.7 ± 0.2 degrees two-theta, 21.5± 0.2 degrees two-theta, 22.1 ± 0.2 degrees two-theta, 23.0 ± 0.2 degrees two-theta, and 27.6 ± 0.2 degrees two-theta.

In some embodiments, vanzacaftor calcium salt hydrate Form D is characterized by an X-ray powder diffractogram having (a) signals at 6.1 ± 0.2 degrees two-theta, 16.2 ± 0.2 degrees two-theta, and 22.8 ± 0.2 degrees two-theta; and (b) two or more signals selected from 5.5 ± 0.2 degrees two-theta, 15.5 ± 0.2 degrees two-theta, 19.7 ± 0.2 degrees two-theta, 21.5± 0.2 degrees two-theta, 22.1 ± 0.2 degrees two-theta, 23.0 ± 0.2 degrees two-theta, and 27.6 ± 0.2 degrees two-theta. In some embodiments, vanzacaftor calcium salt hydrate Form D is characterized by an X-ray powder diffractogram having (a) signals at 6.1 ± 0.2 degrees two-theta, 16.2 ± 0.2 degrees two-theta, and 22.8 ± 0.2 degrees two-theta; and (b) three or more signals selected from 5.5 ± 0.2 degrees two-theta, 15.5 ± 0.2 degrees two-theta, 19.7 ± 0.2 degrees two-theta, 21.5± 0.2 degrees two-theta, 22.1 ± 0.2 degrees two-theta, 23.0 ± 0.2 degrees two-theta, and 27.6 ± 0.2 degrees two-theta. In some embodiments, vanzacaftor calcium salt hydrate Form D is characterized by an X-ray powder diffractogram having (a) signals at 6.1 ± 0.2 degrees two-theta, 16.2 ± 0.2 degrees two-theta, and 22.8 ± 0.2 degrees two-theta; and (b) four or more signals selected from 5.5 ± 0.2 degrees two-theta, 15.5 ± 0.2 degrees two-theta, 19.7 ± 0.2 degrees two-theta, 21.5± 0.2 degrees two-theta, 22.1 ± 0.2 degrees two-theta, 23.0 ± 0.2 degrees two-theta, and 27.6 ± 0.2 degrees two-theta.

In some embodiments, vanzacaftor calcium salt hydrate Form D is characterized by an X-ray powder diffractogram having signals at 6.1 ± 0.2 degrees two-theta, 16.2 ± 0.2 degrees two-theta, and 22.8 ± 0.2 degrees two-theta, and 27.6 ± 0.2 degrees two-theta. In some embodiments, vanzacaftor calcium salt hydrate Form D is characterized by an X-ray powder diffractogram having signals at 6.1 ± 0.2 degrees two-theta, 15.5 ± 0.2 degrees two-theta, 16.2 ± 0.2 degrees two-theta, 19.7 ± 0.2 degrees two-theta, 22.8 ± 0.2 degrees two-theta, and 27.6 ± 0.2 degrees two-theta.

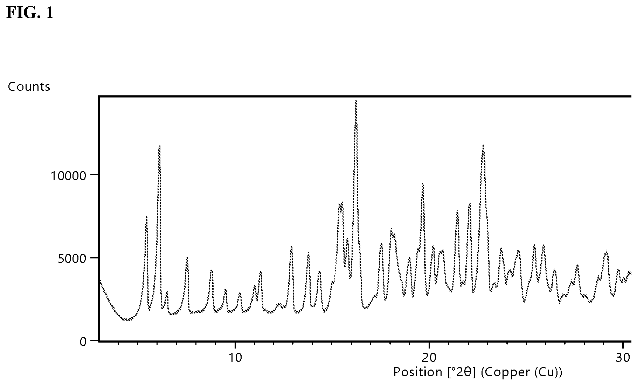

In some embodiments, vanzacaftor calcium salt hydrate Form D is characterized by an X-ray powder diffractogram substantially similar to FIG. 1.

### Tezacaftor

As used herein, the term "tezacaftor" is used interchangeably with (*R*)-1-(2,2-difluorobenzo[d][1,3]dioxol-5-yl)-*N*-(1-(2,3-dihydroxypropyl)-6-fluoro-2-(1-hydroxy-2-methylpropan-2-yl)-1*H*-indol-5-yl)cyclopropanecarboxamide. Tezacaftor can be depicted as having the following structure: 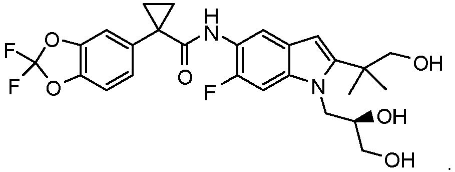 Tezacaftor has been previously described in United States Patent Publication US 2009/0131492, and International Patent Publications WO 2011/119984 and WO 2015/160787.

### Deutivacaftor

As used herein, the term "deutivacaftor" is used interchangeably with *N-*(2-(*tert-*butyl)-5-hydroxy-4-(2-(methyl-d₃)propan-2-yl-1,1,1,3,3,3-d₆)phenyl)-4-oxo-1,4-dihydroquinoline-3-carboxamide. deutivacaftor has the following structure: 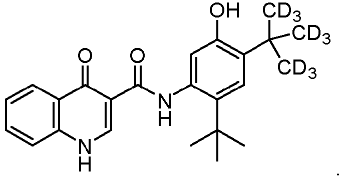 Deutivacaftor has been previously described in International Patent Publications WO 2012/158885, WO 2017/053455, WO 2017/053711, WO 2018/080591, and WO 2019/109021.

The terms "about" and "approximately", when used in connection with doses, amounts, or weight percent of ingredients of a composition or a dosage form, include the value of a specified dose, amount, or weight percent or a range of the dose, amount, or weight percent that is recognized by one of ordinary skill in the art to provide a pharmacological effect equivalent to that obtained from the specified dose, amount, or weight percent. The terms "about" and "approximately" may refer to an acceptable error for a particular numerical value (e.g., weight, size, amount, weight percent, or duration of time) as determined by one of skill in the art, which depends in part on how the values is measured or determined. In some embodiments, the terms "about" and "approximately" mean within 25%, 20%, 15%, 10%, 5%, 4%, 3%, 2%, 1%, or 0.5% of a given value or range. In some embodiments, the terms "about" and "approximately" mean within 10% of a given value or range. In some embodiments, the terms "about" and "approximately" mean within 15% of a given value or range. In some embodiments, the terms "about" and "approximately" mean within 20% of a given value or range. In some embodiments, the terms "about" and "approximately" mean within 25% of a given value or range. As used herein, the symbol "~" appearing immediately before a numerical value has the same meaning as the terms "about" and "approximately."

As used herein, "CFTR" or "CFTR protein" stands for cystic fibrosis transmembrane conductance regulator protein.

As used herein, the term "modulator" refers to a compound that increases the activity of a biological compound such as a protein. For example, a CFTR modulator is a compound that increases the activity of CFTR. The increase in activity resulting from a CFTR modulator includes but is not limited to compounds that correct, potentiate, stabilize and/or amplify CFTR.

As used herein, a "CF potentiator" refers to a compound that exhibits biological activity characterized by increasing gating functionality of the mutant CFTR protein present in the cell surface to approximately wild-type levels (i.e., a compound that augments or induces the channel activity of CFTR protein located at the cell surface, resulting in increased functional activity). Deutivacaftor disclosed herein is a CFTR potentiator.

As used herein, the term "CFTR corrector" refers to a compound that augments or induces the amount of functional CFTR protein to the cell surface, resulting in increased functional activity. Vanzacaftor and tezacaftor disclosed herein are CFTR correctors.

The terms "patient" and "subject" are used interchangeably and refer to an animal including humans.

As used herein, the "terms," "treatment," "treating," and the like generally mean the improvement of CF or its symptoms or lessening the severity of CF or one or more of the symptoms of CF in a subject. "Treatment," as used herein, includes, but is not limited to, the following: increased growth of the subject, increased weight gain, reduction of mucus in the lungs, improved pancreatic and/or liver function, reduction of chest infections, and/or reductions in coughing or shortness of breath. Improvements in or lessening the severity of any of these symptoms can be readily assessed according to standard methods and techniques known in the art.

As used herein, the term "in combination with," when referring to two or more compounds, agents, or additional active pharmaceutical ingredients, means the administration of two or more compounds, agents, or active pharmaceutical ingredients to the patient prior to, concurrently with, or subsequent to each other.

As used herein, the term "amorphous" refers to a solid material having no long-range order in the position of its molecules. Amorphous solids are generally supercooled liquids in which the molecules are arranged in a random manner so that there is no well-defined arrangement, e.g., molecular packing, and no long-range order. Amorphous solids are generally isotropic, i.e. exhibit similar properties in all directions and do not have definite melting points. For example, an amorphous material is a solid material having no sharp characteristic crystalline peak(s) in its X-ray power diffraction (XRPD) pattern (i.e., is not crystalline as determined by XRPD). Instead, one or several broad peaks (e.g., halos) appear in its XRPD pattern. Broad peaks are characteristic of an amorphous solid. *See,* US 2004/0006237 for a comparison of XRPDs of an amorphous material and crystalline material. In some embodiments, a solid material may comprise an amorphous compound, and the material may, for example, be characterized by a lack of sharp characteristic crystalline peak(s) in its XRPD spectrum (i.e. the material is not crystalline, but is amorphous, as determined by XRPD). Instead, one or several broad peaks (e.g. halos) may appear in the XRPD pattern of the material. *See,* US 2004/0006237 for a comparison of XRPDs of an amorphous material and crystalline material. A solid material, comprising an amorphous compound, may be characterized by, for example, a wider temperature range for the melting of the solid material, as compared to the range for the melting of a pure crystalline solid. Other techniques, such as, for example, Raman spectroscopy, infrared spectroscopy, and solid-state NMR may be used to characterize crystalline or amorphous forms.

In some embodiments, a solid material may comprise a mixture of crystalline solids and amorphous solids. A solid material prepared to comprise an amorphous compound may also, for example, contain up to about 30% of a crystalline solid. A solid material prepared to comprise an amorphous compound may also, for example, contain up to 30% of a crystalline solid. In some embodiments, a solid material prepared to comprise an amorphous compound may also, for example, contain up to 25%, 20%, 15%, 10%, 5%, 2%, about 25%, about 20%, about 15%, about 10%, about 5%, or about 2% of a crystalline solid. In embodiments wherein the solid material contains a mixture of crystalline solids and amorphous solids, the characterizing data, such as XRPD, may contain indicators of both crystalline and amorphous solids.

As used herein, the term "substantially amorphous" refers to a solid material having little or no long-range order in the position of its molecules. For example, substantially amorphous materials have less than about 15% crystallinity (e.g., less than about 10% crystallinity or less than about 5% crystallinity). It is also noted that the term "substantially amorphous" includes the descriptor, "amorphous," which refers to materials having no (0%) crystallinity.

As used herein, the term "dispersion" refers to a disperse system in which one substance, the dispersed phase, is distributed, in discrete units, throughout a second substance (the continuous phase or vehicle). The size of the dispersed phase can vary considerably (e.g., single molecules, colloidal particles of nanometer dimension, to multiple microns in size). In general, the dispersed phases can be solids, liquids, or gases. In the case of a solid dispersion, the dispersed and continuous phases are both solids. In this invention, a solid dispersion can include an amorphous drug in an amorphous polymer or an amorphous drug in crystalline polymer. In some embodiments, a solid dispersion includes the drug constituting the dispersed phase, and the polymer constitutes the continuous phase.

As used herein, the term "solid dispersion" generally refers to a solid dispersion of two or more components. In some embodiments, a solid dispersion comprises a single API (e.g., tezacaftor or deutivacaftor). In some embodiments, the solid dispersion comprises two APIs (e.g., tezacaftor and deutivacaftor). In some embodiments, the solid dispersion contains a polymer, but possibly containing other components such as surfactants or other pharmaceutical excipients, where the drug(s) (e.g., tezacaftor and/or deutivacaftor) is substantially amorphous (e.g., having about 15% or less (e.g., about 10% or less, or about 5% or less)) of crystalline drug or amorphous (i.e., having no crystalline drug), and the physical stability and/or dissolution and/or solubility of the substantially amorphous or amorphous drug is enhanced by the other components. Solid dispersions typically include a compound dispersed in an appropriate carrier medium, such as a solid-state carrier. For example, a carrier comprises a polymer (e.g., a water-soluble polymer or a partially water-soluble polymer) and can include optional excipients such as functional excipients (e.g., one or more surfactants) or nonfunctional excipients (e.g., one or more fillers). Another exemplary solid dispersion is a co-precipitate or a co-melt of tezacaftor and/or deutivacaftor, optionally comprising at least one polymer.

As used herein, "crystallinity" refers to the degree of structural order in a solid. For example, a substantially amorphous material has less than about 15% crystallinity, or its solid-state structure is less than about 15% crystalline. In another example, a fully amorphous material has zero (0%) crystallinity.

As used herein, the terms "X-ray powder diffractogram," "X-ray powder diffraction pattern," "XRPD pattern," "XRPD spectrum" interchangeably refer to an experimentally obtained pattern plotting signal positions (on the abscissa) versus signal intensities (on the ordinate). For an amorphous material, an X-ray powder diffractogram may include one or more broad signals; and for a crystalline material, an X-ray powder diffractogram may include one or more signals, each identified by its angular value as measured in degrees 2θ (° 2θ), depicted on the abscissa of an X-ray powder diffractogram, which may be expressed as "a signal at ... degrees two-theta," "a signal at [a] two-theta value(s)of ..." and/or "a signal at at least ... two-theta value(s) selected from ...."

A "signal" or "peak" as used herein refers to a point in the XRPD pattern where the intensity as measured in counts is at a local maximum. One of ordinary skill in the art would recognize that one or more signals (or peaks) in an XRPD pattern may overlap and may, for example, not be apparent to the naked eye. Indeed, one of ordinary skill in the art would recognize that some art-recognized methods are capable of and suitable for determining whether a signal exists in a pattern, such as Rietveld refinement.

As used herein, "a signal at ... degrees two-theta" refer to X-ray reflection positions as measured and observed in X-ray powder diffraction experiments (° 2θ).

The repeatability of the measured angular values is in the range of ± 0.2° 2θ, i.e., the angular value can be at the recited angular value + 0.2 degrees two-theta, the angular value - 0.2 degrees two-theta, or any value between those two end points (angular value + 0.2 degrees two-theta and angular value - 0.2 degrees two-theta).

As used herein, the term "granule" refers to solid particulate pharmaceutical compositions suitable for administration to patient who have difficulty swallowing large tablets or capsules such as, e.g., pediatric patients. As used herein, "granule" is equivalent to similar terms such as minitablets, sprinkles, pellets, beads, particles, particulates, and troches, a plurality of which can be contained in capsules, pouches, packets, sachets, bottles, or blister packs to provide a unit dosage form.

As used herein, "unit dose form" and "unit dosage form" are used interchangeably to refer to a package (e.g., a capsule, packet, sachet, or pouch) containing a plurality of granules, the solid dose form containing a specified amount (i.e., a unit dose) of the API (e.g., vanzacaftor, tezacaftor, and/or deutivacaftor) per administration.

As used herein, an "excipient" is an inactive ingredient in a pharmaceutical composition. Examples of excipients include a filler, a sweetener, a disintegrant, a glidant, a lubricant, and the like.

As used herein, a "diluent" or "filler" is an excipient that adds bulkiness to a pharmaceutical composition. Examples of fillers include mannitol, lactose, celluloses, such as microcrystalline cellulose and ethyl cellulose, cellulose acetate, calcium carbonate, potato starch, sorbitol, polyhydric alcohols, dextrose, or combinations thereof.

As used herein, a "disintegrant" is an excipient that hydrates a pharmaceutical composition and aids in tablet dispersion. Examples of disintegrants include sodium croscarmellose and/or sodium starch glycolate.

As used herein, a "sweetener" is an excipient that imparts a pharmaceutical composition with a sweet taste and/or masks other unpleasant tastes. Examples of sweeteners include sucralose, sorbitol, xylitol, and combinations thereof.

As used herein, a "glidant" is an excipient that imparts a pharmaceutical composition with enhanced flow properties. Examples of glidants include colloidal silica, precipitated silica and/or talc.

As used herein, a "colorant" is an excipient that imparts a pharmaceutical composition with a desired color. Examples of colorants include commercially available pigments such as FD&C Blue # 1 Aluminum Lake, FD&C Blue #2, other FD&C Blue colors, titanium dioxide, iron oxide, and/or combinations thereof.

As used herein, a "lubricant" is an excipient that is added to pharmaceutical compositions to minimize adherence to surfaces, especially for pharmaceutical compositions that are pressed into granules. The lubricant aids in ejection of a granule of a pharmaceutical composition from a compression die. Examples of lubricants include magnesium stearate, stearic acid (stearin), hydrogenated oil, sodium stearyl fumarate, or any combination thereof.

As used herein, "mean particle diameter" is the average particle diameter as measured using techniques such as laser light scattering, image analysis, or sieve analysis.

As used herein, "bulk density" is the mass of particles of material divided by the total volume the particles occupy. The total volume includes particle volume, inter-particle void volume and internal pore volume. Bulk density is not an intrinsic property of a material; it can change depending on how the material is processed.

As used herein, a "wetting agent" or "surfactant" is an excipient that imparts pharmaceutical compositions with enhanced solubility and/or wettability. Examples of wetting agents include sodium lauryl sulfate (SLS), sodium stearyl fumarate (SSF), polyoxyethylene 20 sorbitan mono-oleate (e.g., Tween^{™}), or any combination thereof.

Deutivacaftor is deuterium (²H)-labelled. In chemical structures, deuterium is represented as "D."

The concentration of the isotope(s) (e.g., deuterium) incorporated into ivacaftor may be defined by the isotopic enrichment factor. The term "isotopic enrichment factor" as used herein means the ratio between the isotopic abundance and the natural abundance of a specified isotope. In some embodiments, if a substituent in a compound of the disclosure is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

### DETAILED DESCRIPTION

The present invention relates to pharmaceutical compositions containing calcium bis((14*S*)-8-[3-(2-{dispiro[2.0.2⁴.1³]heptan-7-yl}ethoxy)pyrazol-1-yl]-12,12-dimethyl-2,2,4-trioxo-2λ⁶-thia-3,9,11,18,23-pentaazatetracyclo[17.3.1.1^{11,14}.0⁵,¹⁰]tetracosa-1(23),5,7,9,19,21-hexaen-3-ide) (vanzacaftor calcium salt), (*R*)-1-(2,2-difluorobenzo[*d*][1,3]dioxol-5-yl)-N-(1-(2,3-dihydroxypropyl)-6-fluoro-2-(1-hydroxy-2-methylpropan-2-yl)-1*H*-indol-5-yl)cyclopropanecarboxamide (tezacaftor), and *N*-(2-(*tert*-butyl)-5-hydroxy-4-(2-(methyl-d₃)propan-2-yl-1,1,1,3,3,3-d₆)phenyl)-4-oxo-1,4-dihydroquinoline-3-carboxamide (deutivacaftor), including formulations of the solid compositions into granules, methods for manufacturing and processing the granules, and methods for treating cystic fibrosis employing the pharmaceutical compositions. In some embodiments, vanzacaftor calcium salt is crystalline vanzacaftor calcium salt hydrate Form D. In some embodiments, tezacaftor is amorphous tezacaftor in a solid dispersion. In some embodiments, deutivacaftor is amorphous tezacaftor in a solid dispersion.

### SOLID DISPERSIONS

In some embodiments, the pharmaceutical compositions disclosed herein comprise a first solid dispersion comprising amorphous tezacaftor and a second solid dispersion comprising amorphous deutivacaftor. In some embodiments one or both solid dispersions are spray-dried dispersions.

In some embodiments, each of the first and second solid dispersions independently comprise a plurality of particles having a mean particle diameter of 5 to 100 microns. In some embodiments, each of the first and second solid dispersions independently comprise a plurality of particles having a mean particle diameter of 15 to 40 microns. In some embodiments, each of the first and second solid dispersions independently comprise a plurality of particles having a mean particle diameter of 15 microns. In some embodiments, each of the first and second solid dispersions independently comprise a plurality of particles having a mean particle diameter of about 5 to about 100 microns. In some embodiments, each of the first and second solid dispersions independently comprise a plurality of particles having a mean particle diameter of about 15 to about 40 microns. In some embodiments, each of the first and second solid dispersions independently comprise a plurality of particles having a mean particle diameter of about 15 microns.

In some embodiments, the solid dispersions and the spray dried dispersions of the disclosure can comprise other excipients, such as polymers and/or surfactants. Any suitable polymers and surfactants known in the art can be used. Certain exemplary polymers and surfactants are as described below.

Solid dispersions of amorphous tezacaftor, and/or amorphous deutivacaftor, may be prepared by any suitable method known in the art, e.g., spray drying, lyophilizing, hot melting, or cryogrinding/cryomilling techniques. Typically, such spray drying, lyophilizing, hot melting or cryogrinding/cryomilling techniques results in an amorphous form of API (e.g., tezacaftor and deutivacaftor).

Spray drying is a process that converts a liquid feed to a dried particulate form. Optionally, a secondary drying process such as fluidized bed drying or vacuum drying may be used to reduce residual solvents to pharmaceutically acceptable levels. Typically, spray drying involves contacting a highly dispersed liquid suspension or solution, and a sufficient volume of hot gas to produce evaporation and drying of the liquid droplets. The preparation to be spray dried can be any solution, coarse suspension, slurry, colloidal dispersion, or paste that may be atomized using the selected spray drying apparatus. In one procedure, the preparation is sprayed into a current of warm filtered gas that evaporates the solvent and conveys the dried product to a collector (e.g., a cyclone). The spent gas is then exhausted with the solvent, or alternatively the spent air is sent to a condenser to capture and potentially recycle the solvent. Commercially available types of apparatus may be used to conduct the spray drying. For example, commercial spray dryers are manufactured by Buchi Ltd. And Niro (e.g., the PSD line of spray driers manufactured by Niro) (*see,* US 2004/0105820; US 2003/0144257).

Techniques and methods for spray drying may be found in Perry's Chemical Engineering Handbook, 6th Ed., R. H. Perry, D. W. Green & J. O. Maloney, eds., McGraw-Hill book co. (1984); and Marshall, "Atomization and Spray-Drying," 50, Chem. Eng. Prog. Monogr. Series 2 (1954).

Removal of the solvent may require a subsequent drying step, such as tray drying, fluid bed drying, vacuum drying, microwave drying, rotary drum drying or biconical vacuum drying. In one embodiment, the solid dispersions and the spray dried dispersions of the disclosure are fluid bed dried. In one process, the solvent includes a volatile solvent, for example a solvent having a boiling point of less than 100 °C. In some embodiments, the solvent includes a mixture of solvents, for example a mixture of volatile solvents or a mixture of volatile and non-volatile solvents. Where mixtures of solvents are used, the mixture can include one or more non-volatile solvents, for example, where the non-volatile solvent is present in the mixture at less than 15%, e.g., less than 12%, less than 10%, less than 8%, less than 5%, less than 3%, or less than 2%. Where mixtures of solvents are used, the mixture can also include one or more non-volatile solvents, for example, where the non-volatile solvent is present in the mixture at less than about 15%, e.g., less than about 12%, less than about 10%, less than about 8%, less than about 5%, less than about 3%, or less than about 2%.

The particle size and the temperature drying range may be modified to prepare an optimal solid dispersion. As would be appreciated by skilled practitioners, a small particle size would lead to improved solvent removal. It has been found, however, that smaller particles may result in low bulk density that, under some circumstances, do not provide optimal solid dispersions for downstream processing.

A solid dispersion (e.g., a spray dried dispersion) disclosed herein may optionally include a surfactant. A surfactant or surfactant mixture would generally decrease the interfacial tension between the solid dispersion and an aqueous medium. An appropriate surfactant or surfactant mixture may also enhance aqueous solubility and bioavailability of the API(s) (e.g., amorphous tezacaftor and/or amorphous deutivacaftor) from a solid dispersion. The surfactants for use in connection with the disclosure include, but are not limited to, sorbitan fatty acid esters (e.g., Spans^{®}), polyoxyethylene sorbitan fatty acid esters (e.g., Tweens^{®}), sodium lauryl sulfate (SLS), sodium dodecylbenzene sulfonate (SDBS) dioctyl sodium sulfosuccinate (Docusate sodium), dioxycholic acid sodium salt (DOSS), Sorbitan Monostearate, Sorbitan Tristearate, hexadecyltrimethyl ammonium bromide (HTAB), sodium N-lauroylsarcosine, sodium oleate, sodium myristate, sodium stearate, sodium palmitate, Gelucire 44/14, ethylenediamine tetraacetic acid (EDTA), vitamin E d-alpha tocopheryl polyethylene glycol 1000 succinate (TPGS), lecithin, glutanic acid monosodium monohydrate, labrasol, PEG 8 caprylic/capric glycerides, transcutol, diethylene glycol monoethyl ether, Solutol HS-15, polyethylene glycol/hydroxystearate, taurocholic Acid, Pluronic F68, Pluronic F108, and Pluronic F127 (or any other polyoxyethylene-polyoxypropylene co-polymers (Pluronics^{®}) or saturated polyglycolized glycerides (Gelucirs^{®})). Specific examples of such surfactants that may be used in connection with this disclosure include, but are not limited to, Span 65, Span 25, Tween 20, Capryol 90, Pluronic F108, sodium lauryl sulfate (SLS), Vitamin E TPGS, pluronics and copolymers.

In some embodiments, SLS is used as a surfactant in the solid dispersion of amorphous deutivacaftor. The amount of the surfactant (e.g., SLS) relative to the total weight of the solid dispersion may be between 0.1% w/w and 15% w/w. For example, it is from 0.5% w/w to 10% w/w, such as from 0.5% w/w to 5% w/w, e.g., 0.5% w/w to 4% w/w, 0.5% w/w to 3% w/w, 0.5% w/w to 2% w/w, 0.5% w/w to 1% w/w, or 0.5% w/w. In certain embodiments, the amount of the surfactant relative to the total weight of the solid dispersion is at least 0.1% w/w or at least 0.5% w/w. In some embodiments, the surfactant is SLS in an amount of 0.5% by weight. The amount of the surfactant (e.g., SLS) relative to the total weight of the solid dispersion may also be between about 0.1% w/w and about 15% w/w. For example, it is from about 0.5% w/w to about 10% w/w, such as from about 0.5% w/w to about 5% w/w, e.g., about 0.5% w/w to about 4% w/w, about 0.5% w/w to about 3% w/w, about 0.5% w/w to about 2% w/w, about 0.5% w/w to about 1% w/w, or about 0.5% w/w. In certain embodiments, the amount of the surfactant relative to the total weight of the solid dispersion is at least about 0.1% w/w or at least about 0.5% w/w. In some embodiments, the surfactant is SLS in an amount of about 0.5% by weight.

Another aspect of the disclosure provides a single spray dried dispersion comprising amorphous tezacaftor and amorphous deutivacaftor, wherein the dispersion is prepared without a polymer, and wherein the spray dried dispersion is generated by (i) providing a mixture that consists essentially of amorphous tezacaftor and amorphous deutivacaftor and a solvent; and (ii) forcing the mixture through a nozzle under spray drying conditions to generate the spray dried dispersion.

In some embodiments, solid dispersions for inclusion in a pharmaceutically acceptable composition of the disclosure may be prepared by non-spray drying techniques, such as, for example, cryogrinding/cryomilling techniques. A solid dispersion comprising amorphous tezacaftor or amorphous deutivacaftor may also be prepared by hot melt extrusion techniques.

In some embodiments, the solid dispersions (e.g., spray dried dispersions) of the disclosure comprise a polymer(s). Any suitable polymers known in the art can be used in the disclosure. Exemplary suitable polymers include polymers selected from cellulose-based polymers, polyoxyethylene-based polymers, polyethylene-propylene glycol copolymers, vinyl-based polymers, PEO-polyvinyl caprolactam-based polymers, and polymethacrylate-based polymers.

The cellulose-based polymers include a methylcellulose, a hydroxypropyl methylcellulose (HPMC) (hypromellose), a hypromellose phthalate (HPMC-P), a hypromellose acetate succinate, and co-polymers thereof. The polyoxyethylene-based polymers include a polyethylene-propylene glycol, a polyethylene glycol, a poloxamer, and co-polymers thereof. The vinyl-based polymers include a polyvinylpyrrolidine (PVP), and PVP/VA. The PEO-polyvinyl caprolactam-based polymers include a polyethylene glycol, polyvinyl acetate, and polyvinylcaprolactame-based graft copolymer (e.g., Soluplus^{®}). The polymethacrylate-based polymers are synthetic cationic and anionic polymers of dimethylaminoethyl methacrylates, methacrylic acid, and methacrylic acid esters in varying ratios. Several types are commercially available and may be obtained as the dry powder, aqueous dispersion, or organic solution. Examples of such polymethacrylate-based polymers include a poly(methacrylic acid, ethyl acrylate) (1:1), a dimethylaminoethyl methacrylate-methylmethacrylate copolymer, and Eudragit^{®}.

In some embodiments, the cellulose-based polymer is a hypromellose acetate succinate (also known as hydroxypropyl methylcellulose acetate succinate or HMPCAS) and a hypromellose (also known as hydroxypropyl methylcellulose or HPMC), or a combination of hypromellose acetate succinate and a hypromellose. HPMCAS is available in various grades based on the content of acetyl and succinoyl groups (wt%) in the HPMCAS molecule and on particle size. For example, HPMCAS grades L, M, and H are available. HPMCAS-H is a grade that contains about 10-14 wt% of acetyl groups and about 4-8 wt% of succinoyl groups. Each HPMCAS grade is available in two particle sizes, F (fine) and G (granular). HPMC comes in various types (for example, HPMC E, F, J, and K-types). HPMC E type means that there are about 28-30% methoxy groups and about 7-12% hydroxpropoxy groups. There are various E grades ranging from low to high viscosity. For example, E3 means the viscosity is about 2.4-3.6 millipascal seconds (mPa·s) for HPMC measured at 2% in water at 20 °C; E15 means the viscosity is about 12-18 mPa·s for the HPMC measured at 2% in water at 20 °C; and E50 means the viscosity is about 40-60 mPa·s for the HPMC measured at 2% in water at 20 °C.

In some embodiments, the cellulose-based polymer is a hypromellose acetate succinate and a hypromellose, or a combination of hypromellose acetate succinate and a hypromellose. In some embodiments, the cellulose-based polymer is hypromellose E15, hypromellose acetate succinate L or hypromellose acetate succinate H. In some embodiments, the polyoxyethylene-based polymer or polyethylene-propylene glycol copolymer is a polyethylene glycol or a pluronic. In some embodiments, the polyoxyethylene-based polymer or polyethylene-propylene glycol copolymer is polyethylene glycol 3350 or poloxamer 407. In some embodiments, the vinyl-based polymer is a vinylpolyvinylpyrrolidine-based polymer, such as polyvinylpyrrolidine K30 or polyvinylpyrrolidine VA 64. In some embodiments, the polymethacrylate polymer is Eudragit^{®} L100-55 or Eudragit^{®} E PO. In some embodiments, the polymer(s) is selected from cellulosic polymers such as HPMC and/or HPMCAS.

In one embodiment, a polymer is able to dissolve in aqueous media. The solubility of the polymers may be pH independent or pH dependent. The latter include one or more enteric polymers. The term "enteric polymer" refers to a polymer that is preferentially soluble in the less acidic environment of the intestine relative to the more acid environment of the stomach, for example, a polymer that is insoluble in acidic aqueous media but soluble when the pH is above 5-6. An appropriate polymer is chemically and biologically inert. In order to improve the physical stability of the solid dispersions, the glass transition temperature (T_{g}) of the polymer is as high as possible. For example, polymers that have a glass transition temperature at least equal to or greater than the glass transition temperature of the API. Other polymers have a glass transition temperature that is within about 10 °C to about 15 °C of the API.

Additionally, the hygroscopicity of the polymers is as low, e.g., less than about 10%. For the purpose of comparison in this application, the hygroscopicity of a polymer or composition is characterized at about 60% relative humidity. In some preferred embodiments, the polymer has less than 10% water absorption, for example less than 9%, less than 8%, less than 7%, less than 6%, less than 5%, less than 4%, less than 3%, or less than 2% water absorption. The hygroscopicity can also affect the physical stability of the solid dispersions. Generally, moisture adsorbed in the polymers can greatly reduce the T_{g} of the polymers as well as the resulting solid dispersions, which will further reduce the physical stability of the solid dispersions as described above.

In one embodiment, the polymer is one or more water-soluble polymer(s) or partially water-soluble polymer(s). Water-soluble or partially water-soluble polymers include, but are not limited to, cellulose derivatives (e.g., hydroxypropylmethylcellulose (HPMC), hydroxypropylcellulose (HPC)) or ethylcellulose; polyvinylpyrrolidones (PVP); polyethylene glycols (PEG); polyvinyl alcohols (PVA); acrylates, such as polymethacrylate (e.g., Eudragit^{®} E); cyclodextrins (e.g., β-cyclodextin) and copolymers and derivatives thereof, including for example PVP-VA (polyvinylpyrollidone-vinyl acetate).

In some embodiments, the polymer is hydroxypropylmethylcellulose (HPMC), such as HPMC E50, HPMC E15, or HPMC E3.

As discussed herein, the polymer can be a pH-dependent enteric polymer. Such pH-dependent enteric polymers include, but are not limited to, cellulose derivatives (e.g., cellulose acetate phthalate (CAP)), hydroxypropyl methyl cellulose phthalates (HPMCP), hydroxypropyl methyl cellulose acetate succinate (HPMCAS), carboxymethylcellulose (CMC) or a salt thereof (e.g., a sodium salt such as (CMC-Na)); cellulose acetate trimellitate (CAT), hydroxypropylcellulose acetate phthalate (HPCAP), hydroxypropylmethyl-cellulose acetate phthalate (HPMCAP), and methylcellulose acetate phthalate (MCAP), or polymethacrylates (e.g., Eudragit^{®} S). In some embodiments, the polymer is hydroxypropyl methyl cellulose acetate succinate (HPMCAS). In some embodiments, the polymer is hydroxypropyl methyl cellulose acetate succinate HG grade (HPMCAS-HG).

In yet another embodiment, the polymer is a polyvinylpyrrolidone co-polymer, for example, a vinylpyrrolidone/vinyl acetate co-polymer (PVP/VA).

In embodiments where tezacaftor or deutivacaftor forms a solid dispersion with a polymer, for example with an HPMC, HPMCAS, or PVP/VA polymer, the amount of polymer relative to the total weight of the solid dispersion ranges from 0.1% to 99% by weight. Unless otherwise specified, percentages of drug, polymer, and other excipients as described within a dispersion are given in weight percentages. The amount of polymer is typically at least 20%, and preferably at least 30%, for example, at least 35%, at least 40%, at least 45%, or 50% (e.g., 49.5%). The amount is typically 99% or less, and preferably 80% or less, for example 75% or less, 70% or less, 65% or less, 60% or less, or 55% or less. In one embodiment, the polymer is in an amount of up to 50% of the total weight of the dispersion (and even more specifically, between 40% and 50%, such as 49%, 49.5%, or 50%). In one embodiment, the polymer is in an amount of up to about 50% of the total weight of the dispersion (and even more specifically, between about 40% and about 50%, such as about 49%, about 49.5%, or about 50%).

In some embodiments, tezacaftor (or deutivacaftor) and polymer are present in roughly equal amounts in weight, for example each of the polymer and the drug make up half of the percentage weight of the dispersion. For example, the polymer is present in 49.5 wt % and amorphous tezacaftor (or amorphous deutivacaftor) is present in 50 wt%. In another embodiment, amorphous tezacaftor (or amorphous deutivacaftor) is present in an amount greater than half of the percentage weight of the dispersions. In some embodiments, a first solid dispersion comprises from 70 wt% to 90 wt% (e.g., from 75 wt% to 85 wt%) of amorphous tezacaftor. In some embodiments, a second solid dispersion comprises from 70 wt% to 90 wt% (e.g., from 75 wt% to 85 wt%) of amorphous deutivacaftor. For example, in some embodiments, in a solid dispersion comprising tezacaftor, the polymer is present in 20 wt% and amorphous tezacaftor is present in 80 wt%. In other embodiments, a solid dispersion of amorphous deutivacaftor comprises 19.5 wt% of polymer, 80 wt% of amorphous deutivacaftor, and 0.5 wt% of SLS. In some embodiments, a first solid dispersion comprises from about 70 wt% to about 90 wt% (e.g., from about 75 wt% to about 85 wt%) of amorphous tezacaftor. In some embodiments, a second solid dispersion comprises from about 70 wt% to about 90 wt% (e.g., from about 75 wt% to about 85 wt%) of amorphous deutivacaftor. For example, in some embodiments, in a solid dispersion comprising tezacaftor, the polymer is present in about 20 wt% and amorphous tezacaftor is present in about 80 wt%. In other embodiments, a solid dispersion of amorphous deutivacaftor comprises about 19.5 wt% of polymer, about 80 wt% of amorphous deutivacaftor, and about 0.5 wt% of SLS.

In one embodiment, the solid dispersion of amorphous tezacaftor comprises about 80 percent of amorphous tezacaftor by weight of the solid dispersion, and about 20 percent of HPMC by weight of the solid dispersion.

In one embodiment, the solid dispersion of amorphous deutivacaftor comprises about 80 percent of amorphous deutivacaftor by weight of the solid dispersion, and about 19.5 percent of HPMCAS by weight of the solid dispersion, and about 0.5 percent SLS by weight of the dispersion.

Any suitable spray dried dispersion(s) of amorphous tezacaftor and amorphous deutivacaftor can be used for the pharmaceutical compositions disclosed herein. Some examples for spray-dried dispersions of amorphous tezacaftor and its pharmaceutically acceptable salts can be found in WO 2015/160787 and WO 2011/119984. Solid dispersions of a non-deuterated analog of deutivacaftor and methods of preparing such dispersions are disclosed in PCT Publication No. WO 2007/079139.

### PHARMACEUTICAL COMPOSITIONS

In one aspect, the present invention provides pharmaceutical granule compositions comprising an admixture of two CF corrector APIs (e.g., crystalline vanzacaftor calcium salt hydrate Form D and amorphous tezacaftor) and a CF potentiator API (e.g., amorphous deutivacaftor). As exemplified herein, the pharmaceutical granule compositions of the present invention can be an admixture of vanzacaftor, tezacaftor, and deutivacaftor and one or more excipients described herein. The pharmaceutical composition is capable of being packaged into a unit dosage form, for example, a tablet, capsule, sachet, troches, blister pack, and the like, containing the granules of the present invention in specified dosage amounts.

In some embodiments, the present invention provides a pharmaceutical granule composition, each granule comprising from about 0.05 to about 0.2 mg of crystalline vanzacaftor calcium salt hydrate Form D. For example, each granule may comprise about 0.05 mg, about 0.075 mg, about 0.1 mg, about 0.15 mg, about 0.2 mg of crystalline vanzacaftor calcium salt hydrate Form D. Each unit dosage form (e.g., package of granules) provides about 1 mg to about 12.7 mg of crystalline vanzacaftor calcium salt hydrate Form D. In some embodiments, the unit dose form comprises about 4.2 mg of crystalline vanzacaftor calcium salt hydrate Form D. In some embodiments, the unit dose form comprises about 6.4 mg of crystalline vanzacaftor calcium salt hydrate Form D. In some embodiments, the unit dose form comprises about 8.5 mg of crystalline vanzacaftor calcium salt hydrate Form D. In some embodiments, the unit dose form comprises about 12.7 mg of crystalline vanzacaftor calcium salt hydrate Form D.

In some embodiments, the present invention provides a pharmaceutical granule composition, each granule comprising from about 0.25 to about 0.75 mg of amorphous tezacaftor, or about 0.25 to about 0.75 mg of a solid dispersion comprising amorphous tezacaftor. For example, each granule may comprise about 0.25 mg, about 0.5 mg, or about 0.75 mg of amorphous tezacaftor, or about 0.3 mg, about 0.5 mg, or about 0.7 mg of a solid dispersion comprising amorphous tezacaftor. In some embodiments, the unit dose form comprises about 16 mg of amorphous tezacaftor. In some embodiments, the unit dose form comprises about 24 mg of amorphous tezacaftor. In some embodiments, the unit dose form comprises about 32 mg of amorphous tezacaftor. In some embodiments, the unit dose form comprises about 48 mg of amorphous tezacaftor. In some embodiments, the unit dose form comprises about 20 mg of a solid dispersion comprising amorphous tezacaftor. In some embodiments, the unit dose form comprises about 30 mg of a solid dispersion comprising amorphous tezacaftor. In some embodiments, the unit dose form comprises about 40 mg of a solid dispersion comprising amorphous tezacaftor. In some embodiments, the unit dose form comprises about 60 mg of a solid dispersion comprising amorphous tezacaftor.

In some embodiments, the present invention provides a pharmaceutical granule composition, each granule comprising from about 1.0 to about 3.0 mg of amorphous deutivacaftor, and/or about 1.0 to about 3.0 mg of a solid dispersion comprising amorphous deutivacaftor. For example, each granule may comprise about 1.0 mg, about 1.5 mg, or about 2.0 mg of deutivacaftor, or about 1.0 mg, about 2.0 mg, or about 3.0 mg of a solid dispersion comprising amorphous deutivacaftor. In some embodiments, the unit dose form comprises about 50 mg of amorphous deutivacaftor. In some embodiments, the unit dose form comprises about 75 mg of amorphous deutivacaftor. In some embodiments, the unit dose form comprises about 100 mg of amorphous deutivacaftor. In some embodiments, the unit dose form comprises about 150 mg of amorphous deutivacaftor. In some embodiments, the unit dose form comprises about 62.5 mg of a solid dispersion comprising amorphous deutivacaftor. In some embodiments, the unit dose form comprises about 94 mg of a solid dispersion comprising amorphous deutivacaftor. In some embodiments, the unit dose form comprises about 125 mg of a solid dispersion comprising amorphous deutivacaftor. In some embodiments, the unit dose form comprises about 188 mg of a solid dispersion comprising amorphous deutivacaftor.

In some embodiments, the present invention provides a pharmaceutical granule composition, each granule comprising from about 0.05 to about 0.5 mg of crystalline vanzacaftor calcium salt hydrate Form D, from about 0.25 to about 0.75 mg of amorphous tezacaftor and from about 0.5 to about 1.0 mg of amorphous deutivacaftor. In some embodiments, each granule comprises about 0.14 mg of crystalline vanzacaftor calcium salt hydrate Form D, about 0.5 mg of amorphous tezacaftor, and about 1.7 mg of amorphous deutivacaftor. In some embodiments, the unit dose form comprises about 4.2 mg of crystalline vanzacaftor calcium salt hydrate Form D, about 16 mg of amorphous tezacaftor, and about 50 mg of amorphous deutivacaftor. In some embodiments, the unit dose form comprises about 6.4 mg of crystalline vanzacaftor calcium salt hydrate Form D, about 24 mg of amorphous tezacaftor, and about 75 mg of amorphous deutivacaftor. In some embodiments, the unit dose form comprises about 8.5 mg of crystalline vanzacaftor calcium salt hydrate Form D, about 36 mg of amorphous tezacaftor, and about 100 mg of amorphous deutivacaftor. In some embodiments, the unit dose form comprises about 12.7 mg of crystalline vanzacaftor calcium salt hydrate Form D, about 48 mg of amorphous tezacaftor, and about 150 mg of amorphous deutivacaftor.

In some embodiments, the present invention provides a pharmaceutical granule composition, each granule comprising from about 0.05 to about 0.2 mg of crystalline vanzacaftor calcium salt hydrate Form D, from about 0.25 to about 0.75 mg of a solid dispersion comprising amorphous tezacaftor, and from about 0.5 to about 1.0 mg of a solid dispersion comprising amorphous deutivacaftor. In some embodiments, each granule comprises about 0.1 mg of crystalline vanzacaftor calcium salt hydrate Form D, about 0.7 mg of a solid dispersion comprising amorphous tezacaftor, and about 2.0 mg of a solid dispersion comprising deutivacaftor. In some embodiments, the unit dose form comprises about 4.2 mg of crystalline vanzacaftor calcium salt hydrate Form D, about 20 mg of a solid dispersion comprising amorphous tezacaftor, and about 60 mg of a solid dispersion comprising amorphous deutivacaftor. In some embodiments, the unit dose form comprises about 6.4 mg of crystalline vanzacaftor calcium salt hydrate Form D, about 30 mg of a solid dispersion comprising amorphous tezacaftor, and about 94 mg of a solid dispersion comprising amorphous deutivacaftor. In some embodiments, the unit dose form comprises about 8.5 mg of crystalline vanzacaftor calcium salt hydrate Form D, about 40 mg of a solid dispersion comprising amorphous tezacaftor, and about 125 mg of a solid dispersion comprising amorphous deutivacaftor. In some embodiments, the unit dose form comprises about 12.7 mg of crystalline vanzacaftor calcium salt hydrate Form D, about 60 mg of a solid dispersion comprising amorphous tezacaftor, and about 188 mg of a solid dispersion comprising amorphous deutivacaftor.

In addition to the solid dispersion of tezacaftor and deutivacaftor, pharmaceutical granule compositions of the present invention also comprise one or more excipients such as fillers, sweeteners, disintegrants, wetting agents, glidants, lubricants, colorants, flavoring agents, or combinations thereof. It is noted that some excipients may serve more than one function, such as some fillers can also be sweeteners and some disintegrants can also be wetting agents (e.g., mannitol can be a filler and a sweetener).

Fillers suitable for the present invention are compatible with the ingredients of the pharmaceutical granule composition, i.e., they do not substantially reduce the solubility, the chemical stability, the physical stability, or the biological activity of the pharmaceutical composition. Examples of the filler(s) can include, but are not limited to, mannitol, lactose, sucrose, dextrose, maltodextrin, sorbitol, xylitol, powdered cellulose, polyhydric alcohols, microcrystalline cellulose, silicified microcrystalline cellulose, cellulose acetate, methylcellulose, ethylcellulose, hydroxyethylcellulose, methylhydroxyethylcellulose, talc, starch (i.e., potato starch), pregelatinized starch, dibasic calcium phosphate, calcium sulfate, and calcium carbonate, or combinations thereof.

In one embodiment, the filler comprises microcrystalline cellulose.

In a further embodiment, the one or more fillers in the pharmaceutical granule composition of the invention is a binary filler comprising a mixture of two fillers. In another further embodiment, the binary filler is a mixture of mannitol and another filler. In another embodiment, the binary filler is a mixture of lactose, e.g., lactose monohydrate, and another filler. In certain embodiments, the binary filler is a mixture of mannitol and lactose, e.g., lactose monohydrate.

In some embodiments, the pharmaceutical granule composition comprises a binary filler, wherein the binary filler comprises mannitol and lactose in a ratio of about 3:1 mannitol to lactose. In certain embodiments of the disclosure, a 1 to about 3 mannitol to lactose binary filler results in improved properties of the pharmaceutical granule composition including, e.g., improved palatability and improved processability, without negatively impacting, e.g., dissolution, dispersibility, and/or bioavailability. For example, in certain embodiments, pharmaceutical granule compositions comprising a 1 to about 3 mannitol to lactose binary filler were observed to require low ejection forces during manufacturing even when compression pressure was high, as compared to pharmaceutical compositions comprising mannitol as a sole filler. The ability to use lower ejection force allows the composition to be more reliably manufactured, because the composition is less likely to be damaged during ejection from the die following compression.

The pharmaceutical granule composition also comprises a sweetener to mask and enhance the taste of the composition. In some embodiments, one or more sweeteners include, but are not limited to, monosaccharides, disaccharides, and polysaccharides. Examples of suitable sweeteners include both natural and artificial sweeteners. Examples can include, but are not limited to, glucose, sucrose, maltose, mannose, dextrose, fructose, lactose, trehalose, maltitol, lactitol, xylitol, sorbitol, mannitol, tagatose, glycerin, erythritol, isomalt, maltose, sucralose, aspartame, neotame, alitame, neohesperidin dihydrochalcone, cyclamate (i.e. sodium cyclamate), thaumatin, acesulfame potassium, saccharin, and saccharin sodium. In certain embodiments, the sweetener comprises sucralose in a concentration of about 1 wt% to about 2 wt%.

Disintegrants suitable for the present invention enhance the dispersal of the pharmaceutical granule composition and are compatible with the ingredients of the pharmaceutical composition, i.e., they do not substantially reduce the chemical stability, the physical stability, or the biological activity of the pharmaceutical composition. Exemplary disintegrants include: croscarmellose sodium (e.g., AcDiSol), sodium alginate, calcium alginate, alginic acid, starch, pregelatinized starch, sodium starch glycolate, polyvinylpyrrolidone, copolymers of polyvinylpyrrolidone, crospovidone, carboxymethylcellulose calcium, cellulose and its derivatives, carboxymethylcellulose sodium, soy polysaccharide, clays, gums (i.e. guar gum), an ion exchange resin, an effervescent system based on food acids and an alkaline carbonate component, and sodium bicarbonate. In some embodiments, the pharmaceutical composition comprises about 4 wt% to about 8% of croscarmellose sodium, by weight of the composition. In another example, the pharmaceutical composition comprises about 6 wt% of croscarmellose sodium, by weight of the composition.

Wetting agents and/or surfactants suitable for the present invention can enhance the solubility or the wettability of the pharmaceutical granule composition and are compatible with the ingredients of the pharmaceutical composition, i.e., they do not substantially reduce the chemical stability, the physical stability, or the biological activity of the pharmaceutical composition. In some embodiments, the one or more wetting agents include one or more surfactants. Examples of wetting agents/surfactants may include, but are not limited to the following: sodium lauryl sulfate (also called sodium dodecyl sulfate (SDS)), cetostearyl alcohol, cetomacrogol emulsifying wax, gelatin, casein, docusate sodium, benzalkonium chloride, calcium stearate, polyethylene glycols, phosphates, polyoxyethylene sorbitan fatty acid esters (e.g. Polysorbate 80, Polysorbate 20), gum acacia, cholesterol, tragacanth, polyoxyethylene 20 stearyl ethers, polyoxyethylene alkyl ethers, polyoxyethylene castor oil derivatives, pegylated hydrogenated castor oils, sorbitan esters of fatty acids, vitamin E or tocopherol derivatives, vitamin E TPGS, tocopheryl esters, lecithin, phospholipids and their derivatives, poloxamers, stearic acid, oleic acid, oleic alcohol, cetyl alcohol, mono and diglycerides, propylene glycol esters of fatty acids, glycerol esters of fatty acids (i.e. glycerol monostearate), ethylene glycol palmitostearate, polyoxylglycerides, propylene glycol monocaprylate, propylene glycol monolaurate, alkyl aryl polyether alcohols (Triton^{®}) and polyglyceryl oleate. The use of wetting agents in the pharmaceutical compositions of the invention is optional.

Glidants suitable for the present invention enhance the flow properties of the pharmaceutical composition and are compatible with the ingredients of the pharmaceutical composition, i.e., they do not substantially reduce the solubility, the chemical stability, the physical stability, or the biological activity of the pharmaceutical composition. A "glidant" is a substance to promote powder flow by reducing interparticle friction and cohesion. In certain embodiments, the one or more excipients can include one or more glidants. Examples of the glidants may include, but are not limited to, talc, colloidal silica (e.g., Cabosil M-5P), precipitated silica, magnesium oxide, magnesium silicate, leucine, and starch. In some embodiments, the compositions of the invention contain colloidal silicon dioxide as a glidant. In some embodiments, the pharmaceutical granule compositions comprise about 0.5 to about 1.5 wt% of colloidal silicon dioxide, by weight of the composition. In certain embodiments, the pharmaceutical granule compositions comprise about 1.0 wt% of colloidal silicon dioxide, by weight of the composition.

Lubricants suitable for the present invention improve the compression and ejection of compressed pharmaceutical compositions from a die. Lubricants may further have anti-sticking or anti-tacking properties, and minimize sticking in various operations of the present invention, including operations such as encapsulation, and are compatible with the ingredients of the pharmaceutical composition, i.e., they do not substantially reduce the solubility, or the biological activity of the pharmaceutical composition. Examples of the lubricants may include, but are not limited to, talc, fatty acid, stearic acid, magnesium stearate, calcium stearate, sodium stearate, stearic acid, glyceryl monostearate, sodium lauryl sulfate, sodium stearyl fumarate, hydrogenated oils (i.e., hydrogenated vegetable oil), polyethylene glycol, fatty alcohol, fatty acid ester, glyceryl behenate, mineral oil, vegetable oil, leucine, sodium benzoate, or a combination thereof. In some embodiments, the pharmaceutical granule compositions of the invention comprise magnesium stearate as a glidant. In some embodiments, the pharmaceutical granule composition comprises about 0.5 to about 2.0 wt% of magnesium stearate, by weight of the composition. In certain embodiments, the pharmaceutical granule composition of the invention comprises about 1.0 wt% of magnesium stearate, by weight of the composition.

Pharmaceutical granule compositions of the present invention can optionally comprise one or more colorants, flavors, and/or fragrances to enhance the visual appeal, taste, and/or scent of the composition. Suitable colorants, flavors, or fragrances are compatible with the ingredients of the pharmaceutical composition, i.e., they do not substantially reduce the solubility, the chemical stability, the physical stability, or the biological activity of the pharmaceutical composition. In one embodiment, the pharmaceutical granule composition comprises a colorant, a flavor, and/or a fragrance.

Suitable flavoring agents can include, for example, flavors, which are known to those of skill in the art, such as, for example, natural flavors, artificial flavors, and combinations thereof. Flavoring agents are compatible with the ingredients of the pharmaceutical composition, i.e., they do not substantially reduce the chemical stability, the physical stability, or the biological activity of the pharmaceutical composition. Flavoring agents may be chosen, e.g., from synthetic flavor oils and flavoring aromatics and/or oils, oleoresins, extracts derived from plants, leaves, flowers, fruits, and the like, and combinations thereof. Non-limiting examples of flavor oils include spearmint oil, cinnamon oil, oil of wintergreen (methyl salicylate), peppermint oil, clove oil, bay oil, anise oil, eucalyptus oil, thyme oil, cedar leaf oil, oil of nutmeg, allspice, oil of sage, mace, oil of bitter almonds, and cassia oil. Suitable flavoring agents also include, for example, artificial, natural, and synthetic flower-derived or fruit flavors such as vanilla, ethyl vanillin, citrus oils (e.g., lemon, orange, tangerine, lime, and grapefruit), and fruit essences (e.g., natural and/or artificial flavor of apple, pear, peach, orange, grape, strawberry, raspberry, cherry, plum, pineapple, and apricot), and the like, and combinations thereof. The flavoring agents may be used in liquid or solid form and, as indicated above, may be used individually or in admixture. Other flavoring agents can include, for example, certain aldehydes and esters, e.g., cinnamyl acetate, cinnamaldehyde, citral diethylacetal, dihydrocarvyl acetate, eugenyl formate, 4-methylanisole, and the like, and combinations thereof.

In some embodiments, the present invention provides a pharmaceutical granule composition that can be used to treat a patient who possesses mutant forms of human CFTR.

In other embodiments, the present invention provides unit dose forms comprising a plurality of granules s. The unit dose forms comprise compressed powder pharmaceutical granule compositions as described above with the addition of one or more functional excipients, for example, a disintegrant, glidant, lubricant, filler and/or a wetting agent to facilitate compression of the powder pharmaceutical composition into a compressed pharmaceutical composition, and to facilitate disintegration and dissolution of the compressed powder. The compressed pharmaceutical granule composition can be formulated into unit dose forms such as tablets, capsules, pouches, packets, sachets, bottles, and blister packs containing a plurality of such granules. The number of granules required for each unit dose form will depend on the concentration of vanzacaftor, tezacaftor, and deutivacaftor in each granule, and the required final amount of vanzacaftor required by the unit dose form. For purposes of illustration only, a unit dose form comprising 30 granules, wherein each granule comprises 0.14 mg of crystalline vanzacaftor calcium salt hydrate Form D, 0.54 mg of amorphous tezacaftor, and 1.66 mg of amorphous deutivacaftor, can deliver a unit dose of 4.2 mg crystalline vanzacaftor calcium salt hydrate Form D, 16.1 mg of amorphous tezacaftor, and 49.9 mg of amorphous deutivacaftor.

In some embodiments, the invention includes a pharmaceutical composition, formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;
wherein the unit dosage form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion comprising tezacaftor and a first polymer;
c. a solid dispersion comprising deutivacaftor and a second polymer;
d. one or more fillers;
e. a disintegrant;
f. a sweetener;
g. a glidant; and
h. a lubricant;

wherein the unit dosage form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg; and
wherein tezacaftor and/or deutivacaftor are in substantially amorphous form;

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;

wherein the unit dose form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg; and
wherein vanzacaftor calcium salt hydrate Form D is characterized by an X-ray powder diffractogram having signals at 6.1 ± 0.2 degrees two-theta, 16.2 ± 0.2 degrees two-theta, and 22.8 ± 0.2 degrees two-theta.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;

wherein the unit dose form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg; and
wherein vanzacaftor calcium salt hydrate Form D is characterized by an X-ray powder diffractogram having (a) signals at 6.1 ± 0.2 degrees two-theta, 16.2 ± 0.2 degrees two-theta, and 22.8 ± 0.2 degrees two-theta; and (b) one or more signals selected from 5.5 ± 0.2 degrees two-theta, 15.5 ± 0.2 degrees two-theta, 19.7 ± 0.2 degrees two-theta, 21.5± 0.2 degrees two-theta, 22.1 ± 0.2 degrees two-theta, 23.0 ± 0.2 degrees two-theta, and 27.6 ± 0.2 degrees two-theta.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;

wherein the unit dose form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg; and
wherein vanzacaftor calcium salt hydrate Form D is characterized by an X-ray powder diffractogram having (a) signals at 6.1 ± 0.2 degrees two-theta, 16.2 ± 0.2 degrees two-theta, and 22.8 ± 0.2 degrees two-theta; and (b) two or more signals selected from 5.5 ± 0.2 degrees two-theta, 15.5 ± 0.2 degrees two-theta, 19.7 ± 0.2 degrees two-theta, 21.5± 0.2 degrees two-theta, 22.1 ± 0.2 degrees two-theta, 23.0 ± 0.2 degrees two-theta, and 27.6 ± 0.2 degrees two-theta.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;

wherein the unit dose form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg; and
wherein vanzacaftor calcium salt hydrate Form D is characterized by an X-ray powder diffractogram substantially similar to FIG. 1.

In another aspect, the invention includes a pharmaceutical composition, formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;
wherein the unit dose form comprises about 4.2 mg of crystalline vanzacaftor calcium salt hydrate Form D, about 16 mg of amorphous tezacaftor, and about 50 mg of amorphous deutivacaftor.

In another aspect, the invention includes a pharmaceutical composition, formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;
wherein the unit dose form comprises about 6.4 mg of crystalline vanzacaftor calcium salt hydrate Form D, about 24 mg of amorphous tezacaftor, and about 75 mg of amorphous deutivacaftor.

In another aspect, the invention includes a pharmaceutical composition, formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;
wherein the unit dose form comprises about 8.5 mg of crystalline vanzacaftor calcium salt hydrate Form D, about 32 mg of amorphous tezacaftor, and about 100 mg of amorphous deutivacaftor.

In another aspect, the invention includes a pharmaceutical composition, formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;
wherein the unit dose form comprises about 12.7 mg of crystalline vanzacaftor calcium salt hydrate Form D, about 48 mg of amorphous tezacaftor, and about 150 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion comprising about 80 wt% of amorphous tezacaftor and about 20 wt% of HPMC by weight of the dispersion;
c. a solid dispersion comprising about 80 wt% of amorphous deutivacaftor, about 19.5 wt% of HPMCAS, and about 0.5 wt% of SLS by weight of the dispersion;
d. a binary filler composed of mannitol and lactose;
e. croscarmellose sodium;
f. sucralose;
g. colloidal silicon dioxide; and
h. magnesium stearate;
wherein the unit dose form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion containing about 80 wt% of amorphous tezacaftor and about 20 wt% of HPMC;
c. a solid dispersion containing about 80 wt% of amorphous deutivacaftor, about 19.5 wt% of HPMCAS, and about 0.5 wt% of SLS;
d. a binary filler composed of mannitol and lactose;
e. croscarmellose sodium;
f. sucralose;
g. colloidal silicon dioxide; and
h. magnesium stearate;
wherein the unit dose form comprises about 4.2 mg of crystalline vanzacaftor calcium salt hydrate Form D, about 16 mg of amorphous tezacaftor, and about 50 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion containing about 80 wt% of amorphous tezacaftor and about 20 wt% of HPMC;
c. a solid dispersion containing about 80 wt% of amorphous deutivacaftor, about 19.5 wt% of HPMCAS, and about 0.5 wt% of SLS;
d. a binary filler composed of mannitol and lactose;
e. croscarmellose sodium;
f. sucralose;
g. colloidal silicon dioxide; and
h. magnesium stearate;
wherein the unit dose form comprises about 6.4 mg of crystalline vanzacaftor calcium salt hydrate Form D, about 24 mg of amorphous tezacaftor, and about 75 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion containing about 80 wt% of amorphous tezacaftor and about 20 wt% of HPMC;
c. a solid dispersion containing about 80 wt% of amorphous deutivacaftor, about 19.5 wt% of HPMCAS, and about 0.5 wt% of SLS;
d. a binary filler composed of mannitol and lactose;
e. croscarmellose sodium;
f. sucralose;
g. colloidal silicon dioxide; and
h. magnesium stearate;
wherein the unit dose form comprises about 8.5 mg of crystalline vanzacaftor calcium salt hydrate Form D, about 32 mg of amorphous tezacaftor, and about 100 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion containing about 80 wt% of amorphous tezacaftor and about 20 wt% of HPMC;
c. a solid dispersion containing about 80 wt% of amorphous deutivacaftor, about 19.5 wt% of HPMCAS, and about 0.5 wt% of SLS;
d. a binary filler composed of mannitol and lactose;
e. croscarmellose sodium;
f. sucralose;
g. colloidal silicon dioxide; and
h. magnesium stearate;
wherein the unit dose form comprises about 12.7 mg of crystalline vanzacaftor calcium salt hydrate Form D, about 48 mg of amorphous tezacaftor, and about 150 mg of amorphous deutivacaftor.

In another aspect, the invention includes a pharmaceutical composition formulated into a unit dosage form comprising a plurality of granules, wherein each granule comprises:
a. about 2 wt% of crystalline vanzacaftor calcium salt hydrate Form D by weight of the composition;
b. about 10 wt% of a solid dispersion by weight of the composition, wherein the solid dispersion comprises about 80 wt% of amorphous tezacaftor and about 20 wt% of HPMC by weight of the dispersion;
c. about 30 wt% of a solid dispersion by weight of the composition, wherein the solid dispersion comprises about 80 wt% of amorphous deutivacaftor, about 19.5 wt% of HPMCAS, and about 0.5 wt% of SLS by weight of the dispersion;
d. about 48 wt% of one or more fillers by weight of the composition;
e. about 6 wt% of a disintegrant by weight of the composition;
f. about 2 wt% of a sweetener by weight of the composition;
g. about 1 wt% of a glidant by weight of the composition; and
h. about 1 wt% of a lubricant by weight of the composition;
wherein the unit dose form comprises about 4.2 mg of crystalline vanzacaftor calcium salt hydrate Form D, about 16 mg of amorphous tezacaftor, and about 50 mg of amorphous deutivacaftor.

In another aspect, the invention includes a pharmaceutical composition formulated into a unit dosage form comprising a plurality of granules, wherein each granule comprises:
a. about 2 wt% of crystalline vanzacaftor calcium salt hydrate Form D by weight of the composition;
b. about 10 wt% of a solid dispersion by weight of the composition, wherein the solid dispersion comprises about 80 wt% of amorphous tezacaftor and about 20 wt% of HPMC by weight of the dispersion;
c. about 30 wt% of a solid dispersion by weight of the composition, wherein the solid dispersion comprises about 80 wt% of amorphous deutivacaftor, about 19.5 wt% of HPMCAS, and about 0.5 wt% of SLS by weight of the dispersion;
d. about 48 wt% of one or more fillers by weight of the composition;
e. about 6 wt% of a disintegrant by weight of the composition;
f. about 2 wt% of a sweetener by weight of the composition;
g. about 1 wt% of a glidant by weight of the composition; and
h. about 1 wt% of a lubricant by weight of the composition;
wherein the unit dose form comprises about 6.4 mg of crystalline vanzacaftor calcium salt hydrate Form D, about 24 mg of amorphous tezacaftor, and about 75 mg of amorphous deutivacaftor.

In another aspect, the invention includes a pharmaceutical composition formulated into a unit dosage form comprising a plurality of granules, wherein each granule comprises:
a. about 2 wt% of crystalline vanzacaftor calcium salt hydrate Form D by weight of the composition;
b. about 10 wt% of a solid dispersion by weight of the composition, wherein the solid dispersion comprises about 80 wt% of amorphous tezacaftor and about 20 wt% of HPMC by weight of the dispersion;
c. about 30 wt% of a solid dispersion by weight of the composition, wherein the solid dispersion comprises about 80 wt% of amorphous deutivacaftor, about 19.5 wt% of HPMCAS, and about 0.5 wt% of SLS by weight of the dispersion;
d. about 48 wt% of one or more fillers by weight of the composition;
e. about 6 wt% of a disintegrant by weight of the composition;
f. about 2 wt% of a sweetener by weight of the composition;
g. about 1 wt% of a glidant by weight of the composition; and
h. about 1 wt% of a lubricant by weight of the composition;
wherein the unit dose form comprises about 8.5 mg of crystalline vanzacaftor calcium salt hydrate Form D, about 32 mg of amorphous tezacaftor, and about 100 mg of amorphous deutivacaftor.

In another aspect, the invention includes a pharmaceutical composition formulated into a unit dosage form comprising a plurality of granules, wherein each granule comprises:
a. about 2 wt% of crystalline vanzacaftor calcium salt hydrate Form D by weight of the composition;
b. about 10 wt% of a solid dispersion by weight of the composition, wherein the solid dispersion comprises about 80 wt% of amorphous tezacaftor and about 20 wt% of HPMC by weight of the dispersion;
c. about 30 wt% of a solid dispersion by weight of the composition, wherein the solid dispersion comprises about 80 wt% of amorphous deutivacaftor, about 19.5 wt% of HPMCAS, and about 0.5 wt% of SLS by weight of the dispersion;
d. about 48 wt% of one or more fillers by weight of the composition;
e. about 6 wt% of a disintegrant by weight of the composition;
f. about 2 wt% of a sweetener by weight of the composition;
g. about 1 wt% of a glidant by weight of the composition; and
h. about 1 wt% of a lubricant by weight of the composition;
wherein the unit dose form comprises about 12.7 mg of crystalline vanzacaftor calcium salt hydrate Form D, about 48 mg of amorphous tezacaftor, and about 150 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion containing about 80 wt% of amorphous tezacaftor and about 20 wt% of HPMC;
c. a solid dispersion containing about 80 wt% of amorphous deutivacaftor, about 19.5 wt% of HPMCAS, and about 0.5 wt% of SLS;
d. a binary filler composed of mannitol and lactose;
e. croscarmellose sodium;
f. sucralose;
g. colloidal silicon dioxide; and
h. magnesium stearate;
wherein the unit dose form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg; and

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion containing about 80 wt% of amorphous tezacaftor and about 20 wt% of HPMC;
c. a solid dispersion containing about 80 wt% of amorphous deutivacaftor, about 19.5 wt% of HPMCAS, and about 0.5 wt% of SLS;
d. a binary filler composed of mannitol and lactose;
e. croscarmellose sodium;
f. sucralose;
g. colloidal silicon dioxide; and
h. magnesium stearate;

wherein the unit dose form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg; and
wherein mannitol is present in about 12.1 wt%.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant;
wherein each granule comprises about 0.14 mg of crystalline vanzacaftor calcium salt hydrate Form D, about 0.54 mg of amorphous tezacaftor, and about 1.66 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D;
b. a solid dispersion containing about 80 wt% of amorphous tezacaftor and about 20 wt% of HPMC;
c. a solid dispersion containing about 80 wt% of amorphous deutivacaftor, about 19.5 wt% of HPMCAS, and about 0.5 wt% of SLS;
d. a binary filler composed of mannitol and lactose;
e. croscarmellose sodium;
f. sucralose;
g. colloidal silicon dioxide; and
h. magnesium stearate;

wherein the unit dose form comprises crystalline vanzacaftor calcium salt hydrate Form D in an amount ranging from about 1 mg to about 12.7 mg, amorphous tezacaftor in an amount ranging from about 10 mg to about 50 mg, and amorphous deutivacaftor in an amount ranging from about 15 mg to about 150 mg; and
wherein the unit dosage form comprises about 30 granules.

In another aspect, the invention includes a pharmaceutical composition formulated into a unit dosage form comprising a plurality of granules, wherein each granule comprises:
a. crystalline vanzacaftor calcium salt hydrate Form D in an amount of about 1 to about 5 percent by weight of the pharmaceutical composition,
b. a solid dispersion of amorphous tezacaftor in an amount of about 8 to about 12 percent by weight of the pharmaceutical composition;
c. a solid dispersion of amorphous deutivacaftor in an amount of about 25 to about 35 percent by weight of the pharmaceutical composition;
d. sucralose in an amount of about 1 to about 3 percent by weight of the pharmaceutical composition;
e. croscarmellose sodium in an amount of about 1 to about 10 percent by weight of the pharmaceutical composition;
f. colloidal silicon dioxide in an amount of about 0.1 to about 5 percent by weight of the pharmaceutical composition;
g. magnesium stearate in an amount of about 0.5 to about 2.0 percent by weight of the pharmaceutical composition;
h. mannitol in an amount of about 10 to about 15 percent by weight of the pharmaceutical composition; and
i. lactose monohydrate in an amount of about 30 to about 40 percent by weight of the pharmaceutical composition;
and wherein the unit dosage form comprises about 0.1 to about 12.7 mg of crystalline vanzacaftor calcium salt hydrate Form D, about 10 to about 50 mg of amorphous tezacaftor, and about 15 to about 150 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D in an amount of about 2 percent by weight of the pharmaceutical composition,
b. a solid dispersion of amorphous tezacaftor in an amount of about 10 percent by weight of the pharmaceutical composition;
c. a solid dispersion of amorphous deutivacaftor in an amount of about 30 percent by weight of the pharmaceutical composition;
d. sucralose in an amount of about 2.3 percent by weight of the pharmaceutical composition;
e. croscarmellose sodium in an amount of about 6 percent by weight of the pharmaceutical composition;
f. colloidal silicon dioxide in an amount of about 1 percent by weight of the pharmaceutical composition;
g. magnesium stearate in an amount of about 1 percent by weight of the pharmaceutical composition;
h. mannitol in an amount of about 12 percent by weight of the pharmaceutical composition; and
i. lactose monohydrate in an amount of about 36 percent by weight of the pharmaceutical composition;
and wherein the unit dosage form comprises about 4.2 mg of crystalline vanzacaftor calcium salt hydrate Form D, about 16 mg of amorphous tezacaftor, and about 50 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D in an amount of about 2 percent by weight of the pharmaceutical composition,
b. a solid dispersion of amorphous tezacaftor in an amount of about 10 percent by weight of the pharmaceutical composition;
c. a solid dispersion of amorphous deutivacaftor in an amount of about 30 percent by weight of the pharmaceutical composition;
d. sucralose in an amount of about 2.3 percent by weight of the pharmaceutical composition;
e. croscarmellose sodium in an amount of about 6 percent by weight of the pharmaceutical composition;
f. colloidal silicon dioxide in an amount of about 1 percent by weight of the pharmaceutical composition;
g. magnesium stearate in an amount of about 1 percent by weight of the pharmaceutical composition;
h. mannitol in an amount of about 12 percent by weight of the pharmaceutical composition; and
i. lactose monohydrate in an amount of about 36 percent by weight of the pharmaceutical composition;
and wherein the unit dosage form comprises about 6.4 mg of crystalline vanzacaftor calcium salt hydrate Form D, about 24 mg of amorphous tezacaftor, and about 75 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D in an amount of about 2 percent by weight of the pharmaceutical composition,
b. a solid dispersion of amorphous tezacaftor in an amount of about 10 percent by weight of the pharmaceutical composition;
c. a solid dispersion of amorphous deutivacaftor in an amount of about 30 percent by weight of the pharmaceutical composition;
d. sucralose in an amount of about 2.3 percent by weight of the pharmaceutical composition;
e. croscarmellose sodium in an amount of about 6 percent by weight of the pharmaceutical composition;
f. colloidal silicon dioxide in an amount of about 1 percent by weight of the pharmaceutical composition;
g. magnesium stearate in an amount of about 1 percent by weight of the pharmaceutical composition;
h. mannitol in an amount of about 12 percent by weight of the pharmaceutical composition; and
i. lactose monohydrate in an amount of about 36 percent by weight of the pharmaceutical composition;
and wherein the unit dosage form comprises about 8.5 mg of crystalline vanzacaftor calcium salt hydrate Form D, about 32 mg of amorphous tezacaftor, and about 100 mg of amorphous deutivacaftor.

In another aspect, the invention includes a pharmaceutical composition formulated into a unit dosage form comprising a plurality of granules, wherein each granule comprises:
a. crystalline vanzacaftor calcium salt hydrate Form D in an amount of about 2 percent by weight of the pharmaceutical composition,
b. a solid dispersion of amorphous tezacaftor in an amount of about 10 percent by weight of the pharmaceutical composition;
c. a solid dispersion of amorphous deutivacaftor in an amount of about 30 percent by weight of the pharmaceutical composition;
d. sucralose in an amount of about 2.3 percent by weight of the pharmaceutical composition;
e. croscarmellose sodium in an amount of about 6 percent by weight of the pharmaceutical composition;
f. colloidal silicon dioxide in an amount of about 1 percent by weight of the pharmaceutical composition;
g. magnesium stearate in an amount of about 1 percent by weight of the pharmaceutical composition;
h. mannitol in an amount of about 12 percent by weight of the pharmaceutical composition; and
i. lactose monohydrate in an amount of about 36 percent by weight of the pharmaceutical composition;
and wherein the unit dosage form comprises about 12.7 mg of crystalline vanzacaftor calcium salt hydrate Form D, about 48 mg of amorphous tezacaftor, and about 150 mg of amorphous deutivacaftor.

### ADMINISTRATION OF A PHARMACEUTICAL COMPOSITION

In another aspect, the invention also provides a method of treating or lessening the severity of a disease in a patient comprising administering to said patient one of the pharmaceutical compositions as defined herein. In some embodiments, the disease is cystic fibrosis.

In some embodiments, the methods of administration of the present invention includes orally administering a liquid or beverage including, but not limited to, milk (including breast milk), baby formula or infant formula, or a soft food including, but not limited to, apple sauce, plain yogurt, ice cream, baby food (including carrots and carrot puree) into which the unit dose form of a pharmaceutical composition of the invention has been sprinkled. It is noted that any of the methods of administration of the present invention can optionally include orally administering with fat-containing food such as a standard CF high-calorie, high-fat meal or snack. In some embodiments, any of the methods of administration of the present invention can optionally include orally administering concurrently with, before or after fat-containing food such as a standard CF high-calorie, high-fat meal or snack.

It is also noted that the methods of administration of the present invention include administering the compositions of the present invention to a patient according to age or weight. In some embodiments, the method of administering a pharmaceutical composition includes orally administering to a patient every 24 hours at least one capsule or packet containing a plurality of granules, wherein each granule comprises a pharmaceutical composition as described herein, wherein the patient is 12 to 24 months or 2 to 5 years of age. In still other embodiments, the method of administering a pharmaceutical composition includes orally administering to a patient every 24 hours at least one capsule or packet containing a plurality of granules, wherein each granule comprises a pharmaceutical composition as described herein, wherein the patient includes, but not limited to, those weighing greater than or equal to about 14 kilograms. In other embodiments, the method of administering a pharmaceutical composition includes orally administering to a patient every 24 hours at least one capsule or packet containing a plurality of granules, wherein each granule comprises a pharmaceutical composition as described herein, wherein the patient includes, but not limited to, those weighing less than 14 kilograms. In still other embodiments, the method of administering a pharmaceutical composition includes orally administering to a patient every 24 hours at least one capsule or packet containing a plurality of granules, wherein each granule comprises a pharmaceutical composition as described herein, wherein the patient includes those weighing about 7.5 kilograms to less than 14 kilograms. In other embodiments, the method of administering a pharmaceutical composition includes orally administering to a patient every 24 hours at least one capsule or packet containing a plurality of granules, wherein each granule comprises a pharmaceutical composition as described herein, wherein the patient includes those weighing about 5 kilograms to less than 7.5 kilograms. In other embodiments, the method of administering a pharmaceutical composition includes orally administering to a patient every 24 hours at least one capsule or packet containing a plurality of granules, wherein each granule comprises a pharmaceutical composition as described herein, wherein the patient includes those weighing about 2.5 kilograms to less than 5 kilograms. In other embodiments, the method of administering a pharmaceutical composition includes orally administering to a patient every 24 hours at least one capsule or packet containing a plurality of granules, wherein each granule comprises a pharmaceutical composition as described herein, wherein the patient includes those weighing less than 7.5 kilograms. In other embodiments, the method of administering a pharmaceutical composition includes orally administering to a patient every 24 hours at least one capsule or packet containing a plurality of granules, wherein each granule comprises a pharmaceutical composition as described herein, wherein the patient includes those weighing about 5 kilograms to more than 5 kilograms. In other embodiments, the method of administering a pharmaceutical composition includes orally administering to a patient every 24 hours at least one capsule or packet containing or plurality of granules, wherein each granule comprises a pharmaceutical composition as described herein, wherein the patient includes those weighing less than 5 kilograms. In other embodiments, the method of administering a pharmaceutical composition includes orally administering to a patient every 24 hours at least one capsule or packet containing a plurality of granules, wherein each granule comprises a pharmaceutical composition as described herein, wherein the patient includes those weighing about 2.5 kilograms to more than 2.5 kilograms.

It is also noted that the methods of administration of the present invention include administering the compositions of the present invention to a patient according to age or weight. In some embodiments, the method of administering a pharmaceutical composition includes orally administering to a patient once a day at least one capsule or packet containing a plurality of granules, wherein each granule comprises a pharmaceutical composition as described herein, wherein the patient is 12 to 24 months or 2 to 5 years of age. In still other embodiments, the method of administering a pharmaceutical composition includes orally administering to a patient once a day at least one capsule or packet containing a plurality of granules, wherein each granule comprises a pharmaceutical composition as described herein, wherein the patient includes, but not limited to, those weighing greater than or equal to about 14 kilograms. In other embodiments, the method of administering a pharmaceutical composition includes orally administering to a patient once a day at least one capsule or packet containing a plurality of granules, wherein each granule comprises a pharmaceutical composition as described herein, wherein the patient includes, but is not limited to, those weighing less than 14 kilograms. In still other embodiments, the method of administering a pharmaceutical composition includes orally administering to a patient once a day at least one capsule or packet containing a plurality of granules, wherein each granule comprises a pharmaceutical composition as described herein, wherein the patient includes those weighing about 7.5 kilograms to less than 14 kilograms. In other embodiments, the method of administering a pharmaceutical composition includes orally administering to a patient once a day at least one capsule or packet containing a plurality of granules, wherein each granule comprises a pharmaceutical composition as described herein, wherein the patient includes to those weighing about 5 kilograms to less than 7.5 kilograms. In other embodiments, the method of administering a pharmaceutical composition includes orally administering to a patient once a day at least one capsule or packet containing a plurality of granules, wherein each granule comprises a pharmaceutical composition as described herein, wherein the patient includes those weighing about 2.5 kilograms to less than 5 kilograms. In other embodiments, the method of administering a pharmaceutical composition includes orally administering to a patient once a day at least one capsule or packet containing a plurality of granules, wherein each granule comprises a pharmaceutical composition as described herein, wherein the patient includes those weighing less than 7.5 kilograms. In other embodiments, the method of administering a pharmaceutical composition includes orally administering to a patient once a day at least one capsule or packet containing a plurality of granules, wherein each granule comprisees a pharmaceutical composition as described herein, wherein the patient includes those weighing about 5 kilograms to more than 5 kilograms. In other embodiments, the method of administering a pharmaceutical composition includes orally administering to a patient once a day at least one capsule or packet containing a plurality of granules, wherein each granule comprises a pharmaceutical composition as described herein, wherein the patient includes those weighing less than 5 kilograms. In other embodiments, the method of administering a pharmaceutical composition includes orally administering to a patient once a day at least one capsule or packet containing a plurality of granules, wherein each granule comprises a pharmaceutical composition as described herein, wherein the patient includes those weighing about 2.5 kilograms to more than 2.5 kilograms.

In some embodiments, the patient has at least one F508del mutation in the CFTR gene. In some embodiments, the patient has a CFTR gene mutation that is responsive to a compound, tautomer, deuterated derivative, or pharmaceutically acceptable salt of the invention based on in vitro data. In some embodiments, the patient has a CFTR gene mutation that is responsive to a compound, tautomer, deuterated derivative, or pharmaceutically acceptable salt of the invention based on clinical data. In some embodiments, the patient is heterozygous and has an F508del mutation on one allele and a mutation on the other allele selected from Table 1:

**Table 1: CFTR Mutations**

| **CFTR Mutations That Are Responsive to Triple Combination Therapy** | | | | | |
|---|---|---|---|---|---|
| 3141del9 | E822K | G1069R | L967S | R117L | S912L |
| 546insCTA | F191V | G1244E | L997F | R117P | S945L |
| A46D | F311del | G1249R | L1077P | R170H | S977F |
| A120T | F311L | G1349D | L1324P | R258G | S1159F |
| A234D | F508C | H139R | L1335P | R334L | S1159P |
| A349V | F508C;S1251N^{†} | H199Y | L1480P | R334Q | S1251N |
| A455E | F508del* | H939R | M152V | R347H | S1255P |
| A554E | F575Y | H1054D | M265R | R347L | T3381 |
| A1006E | F1016S | H1085P | M9521 | R347P | T1036N |
| A1067T | F1052V | H1085R | M952T | R352Q | T10531 |
| D110E | F1074L | H1375P | M1101K | R352W | V201M |
| D110H | F1099L | 1148T | P5L | R553Q | V232D |
| D192G | G27R | 1175V | P67L | R668C | V456A |
| D443Y | G85E | 1336K | P205S | R751L | V456F |
| D443Y;G576A; R668C^{†} | G126D | 1502T | P574H | R792G | V5621 |
| D579G | G178E | I601F | Q98R | R933G | V754M |
| D614G | G178R | 1618T | Q237E | R1066H | V1153E |
| D836Y | G194R | 1807M | Q237H | R1070Q | V1240G |
| D924N | G194V | 1980K | Q359R | R1070W | V1293G |
| D979V | G314E | 11027T | Q1291R | R1162L | W361R |
| D1152H | G463V | 11139V | R31L | R1283M | W1098C |
| D1270N | G480C | 11269N | R74Q | R1283S | W1282R |
| E56K | G551D | 11366N | R74W | S13F | Y109N |
| E60K | G551S | K1060T | R74W;D1270N^{†} | S341P | Y161D |
| E92K | G576A | L15P | R74W;V201M^{†} | S364P | Y161S |
| E116K | G576A;R668C^{†} | L165S | R74W;V201M;D1270N^{†} | S492F | Y563N |
| E193K | G622D | L206W | R75Q | S549N | Y1014C |
| E403D | G628R | L320V | R117C | S549R | Y1032C |
| E474K | G970D | L346P | R117G | S589N | |
| E588V | G1061R | L453S | R117H | S737F | |

| | | | | | |
|---|---|---|---|---|---|
| * F508del is a responsive *CFTR* mutation based on both clinical and in vitro data. ^{†} Complex/compound mutations where a single allele of the *CFTR* gene has multiple mutations; these exist independent of the presence of mutations on the other allele. | | | | | |

In some embodiments, the method includes treating or lessening the severity of cystic fibrosis in a patient comprising administering to said patient a pharmaceutical composition comprising a compressed pharmaceutical composition. In one embodiment, a capsule pharmaceutical composition comprising a unit dosage form of about 1 mg to about 12.7 mg of vanzacaftor calcium salt hydrate Form D, about 10 mg to about 50 mg of amorphous tezacaftor, and about 15 mg to about 150 mg of amorphous deutivacaftor is administered to the patient.

In some embodiments, the method includes treating or lessening the severity of cystic fibrosis in a patient comprising administering to said patient a compressed pharmaceutical composition of the invention, i.e., a capsule or a packet containing a plurality of granules comprising a unit dosage form of about 4.2 mg of vanzacaftor calcium salt hydrate form D, about 20 mg of tezacaftor and about 60 mg of deutivacaftor. In some embodiments, the method includes treating or lessening the severity of cystic fibrosis in a patient comprising administering to said patient a compressed pharmaceutical composition of the invention, i.e., a capsule or a packet containing a plurality of granules comprising a unit dosage form of about 6.4 mg of vanzacaftor calcium salt hydrate form D, about 24 mg of tezacaftor and about 75 mg of deutivacaftor. In some embodiments, the method includes treating or lessening the severity of cystic fibrosis in a patient comprising administering to said patient a compressed pharmaceutical composition of the invention, i.e., a capsule or a packet containing a plurality of granules comprising a unit dosage form of about 8.5 mg of vanzacaftor calcium salt hydrate form D, about 32 mg of tezacaftor and about 100 mg of deutivacaftor. In some embodiments, the method includes treating or lessening the severity of cystic fibrosis in a patient comprising administering to said patient a compressed pharmaceutical composition of the invention, i.e., a capsule or a packet containing a plurality of granules comprising a unit dosage form of about 12.7 mg of vanzacaftor calcium salt hydrate form D, about 48 mg of tezacaftor and about 150 mg of deutivacaftor.

It is also noted that the methods of administration of the present invention can optionally include orally administering a pharmaceutical composition as described herein in the absence of food or beverage. In one embodiment of the invention, the oral administration is performed directly after, or shortly after (e.g., within about 30 minutes) the patient eats or drinks. In another embodiment, the oral administration is performed at least 1 hour (e.g., at least 2 hours, at least 3 hours, at least 4 hours, at least 5 hours, at least 8 hours, at least 12 hours or at least 24 hours) after eating or drinking. For instance, in one example, the method of administering a pharmaceutical composition includes orally administering to a patient once per day at least one capsule or the contents of a packet or a pouch comprising a plurality of granules, each granule containing vanzacaftor, tezacaftor, and deutivacaftor, one or more fillers, a sweetener, a disintegrant, optionally a wetting agent, a glidant; and a lubricant, and another therapeutic or medical procedure. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate for the disease, or condition, being treated."

In one embodiment, the additional therapeutic agent is selected from a mucolytic agent, bronchodilator, an anti-biotic, an anti-infective agent, an anti-inflammatory agent, or a nutritional agent.

In one embodiment, the additional agent is an antibiotic. Exemplary antibiotics useful herein include tobramycin, including tobramycin inhaled powder (TIP), azithromycin, aztreonam, including the aerosolized form of aztreonam, amikacin, including liposomal formulations thereof, ciprofloxacin, including formulations thereof suitable for administration by inhalation, levofloxacin, including aerosolized formulations thereof, and combinations of two antibiotics, e.g., fosfomycin and tobramycin.

In another embodiment, the additional agent is a mucolyte. Exemplary mucolytes useful herein includes Pulmozyme^{®}.

In another embodiment, the additional agent is a bronchodilator. Exemplary bronchodilators include albuterol, metaproterenol sulfate, pirbuterol acetate, salmeterol, or terbutaline sulfate.

In another embodiment, the additional agent is effective in restoring lung airway surface liquid. Such agents improve the movement of salt in and out of cells, allowing mucus in the lung airway to be more hydrated and, therefore, cleared more easily. Exemplary such agents include hypertonic saline, denufosol tetrasodium ([[(3*S*,5*R*)-5-(4-amino-2-oxopyrimidin-1-yl)-3-hydroxyoxolan-2-yl]methoxy-hydroxyphosphoryl][[[(2*R*,3*S*,4*R*,5*R*)-5-(2,4-dioxopyrimidin-1-yl)-3,4-dihydroxyoxolan-2-yl]methoxy-hydroxyphosphoryl]oxy-hydroxyphosphoryl]hydrogen phosphate), or bronchitol (inhaled formulation of mannitol).

In another embodiment, the additional agent is an anti-inflammatory agent, i.e., an agent that can reduce the inflammation in the lungs. Exemplary such agents useful herein include ibuprofen, docosahexaenoic acid (DHA), sildenafil, inhaled glutathione, pioglitazone, hydroxychloroquine, or simavastatin.

In another embodiment, the additional agent is a CFTR modulator other than vanzacaftor, i.e., an agent that has the effect of modulating CFTR activity. Exemplary such agents include ataluren ("PTC124^{®}"; 3-[5-(2-fluorophenyl)-1,2,4-oxadiazol-3-yl]benzoic acid), sinapultide, lancovutide, depelestat (a human recombinant neutrophil elastase inhibitor), cobiprostone (7-{(2*R*,4a*R*,5*R*,7a*R*)-2-[(3*S*)-1,1-difluoro-3-methylpentyl]-2-hydroxy-6-oxooctahydrocyclopenta[*b*]pyran-5-yl}heptanoic acid), or (3-(6-(1-(2,2-difluorobenzo[*d*][1,3]dioxol-5-yl) cyclopropanecarboxamido)-3-methylpyridin-2-yl)benzoic acid. In another embodiment, the additional agent is (3-(6-(1-(2,2-difluorobenzo[*d*][1,3]dioxol-5-yl) cyclopropanecarboxamido)-3-methylpyridin-2-yl)benzoic acid.

In another embodiment, the additional agent is a nutritional agent. Exemplary such agents include pancrelipase (pancreating enzyme replacement), including Pancrease^{®}, Pancreacarb^{®}, Ultrase^{®}, or Creon^{®}, Liprotamase^{®} (formerly Trizytek^{®}), Aquadeks^{®}, or glutathione inhalation. In one embodiment, the additional nutritional agent is pancrelipase.

### EXAMPLES

In order that the invention described herein may be more fully understood, the following examples are set forth. It should be understood that these examples are for illustrative purposes only and are not to be construed as limiting this invention in any manner.

### Abbreviations

ACN = acetonitrile
AUC = area under curve
Boc = *tert*-butyloxycarbonyl
DCM = dichloromethane
DMF = dimethylformamide
DMSO = dimethylsulfoxide
ESI = electrospray ionization
FTNMR = Fourier transform nuclear magnetic resonance
HPLC = high-performance liquid chromatography
HPMC = hydroxypropyl methylcellulose
HPMCAS = hydroxypropylmethylcellulose acetate succinate
IPA = isopropyl alcohol
IPAc = isopropyl acetate
KF = Karl Fischer titration
LC = liquid chromatography
LCMS = liquid chromatography-mass spectrometry
MeTHF = 2-methyltetrahydrofuran
MS = mass spectrometry
MTBE = methyl *tert-*butyl ether
NMR = nuclear magnetic resonance
pn = part number
SDD = spray dried dispersion
SFC = supercritical fluid chromatography
SLS = sodium lauryl sulfate
THF = tetrahydrofuran
TLC = thin layer chromatography
UPLC = ultra-high performance liquid chromatography
UV = ultraviolet
wt% = weight percent

### General UPLC/HPLC Analytical Methods

Unless indicated, yields of enantiomers separated by chiral SFC are given as a percentage of the theoretical yield for a single enantiomer of the racemate.

LC Method A: Analytical reverse phase UPLC using an Acquity UPLC BEH C₁₈ column (30 × 2.1 mm, 1.7 µm particle) made by Waters (pn: 186002349), and a dual gradient run from 1-99% mobile phase B over 1.2 minutes. Mobile phase A = water (0.05% trifluoroacetic acid). Mobile phase B = acetonitrile (0.035% trifluoroacetic acid). Flow rate = 1.5 mL/min, injection volume = 1.5 µL, and column temperature = 60 °C.

LC Method B: Analytical reverse phase UPLC using an Acquity UPLC BEH C₁₈ column (50 × 2.1 mm, 1.7 µm particle) made by Waters (pn: 186002350), and a dual gradient run from 1-99% mobile phase B over 3.0 minutes. Mobile phase A = water (0.05% trifluoroacetic acid). Mobile phase B = acetonitrile (0.035% trifluoroacetic acid). Flow rate = 1.2 mL/min, injection volume = 1.5 µL, and column temperature = 60 °C.

LC Method D: Analytical reverse phase UPLC using an Acquity UPLC BEH C₁₈ column (50 × 2.1 mm, 1.7 µm particle) made by Waters (pn: 186002350), and a dual gradient run from 1-99% mobile phase B over 5.0 minutes. Mobile phase A = water (0.05% trifluoroacetic acid). Mobile phase B = acetonitrile (0.035% trifluoroacetic acid). Flow rate = 1.2 mL/min, injection volume = 1.5 µL, and column temperature = 60 °C.

LC Method F: Analytical reverse phase UPLC using an Acquity UPLC BEH C₁₈ column (50 × 2.1 mm, 1.7 µm particle) made by Waters (pn: 186002350), and a dual gradient run from 1-99% mobile phase B over 15.0 minutes. Mobile phase A = water (0.05% trifluoroacetic acid). Mobile phase B = acetonitrile (0.035% trifluoroacetic acid). Flow rate = 1.2 mL/min, injection volume = 1.5 µL, and column temperature = 60 °C.

LC Method Q: Merck Millipore Chromolith SpeedROD C₁₈ column (50 x 4.6 mm) and a dual gradi-nt run from 5 - 100% mobile phase B over 12 minutes. Mobile phase A = water (0.1 % trifluoroacetic acid). Mobile phase B = acetonitrile (0.1 % trifluoroacetic acid).

### Example 1: Synthesis of vanzacaftor calcium salt hydrate Form D

### Part A. Synthesis of (14S)-8-bromo-12,12-dimethyl-2λ⁶-thia-3,9,11,18,23-pentaazatetracyclo[17.3.1.1^{11,14}.0^{5,10}]tetracosa-1(23),5,7,9,19,21-hexaene-2,2,4-trione

### Step 1: (E)-(2-oxotetrahydropyran-3-ylidene)methanolate (sodium salt)

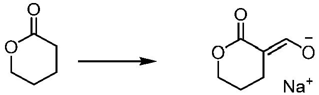

A 5 L, 3-neck round bottom flask was fitted with a mechanical stirrer, a heating mantle, an addition funnel, a J-Kem temperature probe/controller and a nitrogen inlet/outlet. The vessel was charged under a nitrogen atmosphere with sodium hydride (59.91 g of 60% w/w, 1.498 mol) followed by heptane (1.5 L) which provided a grey suspension. Stirring was commenced and the pot temperature was recorded at 19 °C. The vessel was then charged with ethyl alcohol (3.451 g, 74.91 mmol) added *via* syringe which resulted in gas evolution. The addition funnel was charged with a clear pale yellow solution of tetrahydropyran-2-one (150 g, 1.498 mol) and ethyl formate (111 g, 1.50 mol). The solution was added dropwise over 1 h which resulted in gas evolution and a gradual exotherm to 45 °C. The resulting thick white suspension was then heated to 65 °C for 2 h and then allowed to cool to room temperature. The mixture was continued to stir at room temperature overnight (about 10 h). The reaction mixture was vacuum filtered through a glass frit Buchner funnel (medium porosity) under a stream of nitrogen. The filter cake was displacement washed with heptane (2 X 250 mL) and pulled for a few min. The slightly heptane wet cake was transferred to a glass tray and dried in a vacuum oven at 45 °C for 15 h to provide a white solid (205 g, 1.36 mol, 91% yield) as the desired product, (*E*)-(2-oxotetrahydropyran-3-ylidene)methanolate (sodium salt).

### Step 2: 3-Methylenetetrahydropyran-2-one

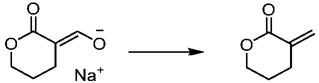

A 5 L, 3-neck round bottom flask was fitted with a mechanical stirrer, a heating mantle, an addition funnel, a J-Kem temperature probe/controller and a nitrogen inlet/outlet. The vessel was charged under a nitrogen atmosphere with (*E*)-(2-oxotetrahydropyran-3-ylidene)methanolate (sodium salt) (205 g, 1.366 mol) (205 g, 1.366 mol) and tetrahydrofuran (1640 mL) which provided a white suspension. Stirring was commenced and the pot temperature was recorded at 19 °C. The vessel was then charged with paraformaldehyde (136.6 g, 4.549 mol) added as a solid in one portion. The resulting suspension was heated to 63 °C and the condition was maintained for 15 h. Upon heating the reaction mixture became slightly gelatinous. The white gelatinous mixture was concentrated under reduced pressure to remove most of the tetrahydrofuran. The remaining residue was partitioned with ethyl acetate (1000 mL), saturated sodium chloride (500 mL) and saturated sodium hydrogen carbonate (500 mL) in a separatory funnel. The organic was removed and the residual aqueous was extracted with ethyl acetate (5 X 300 mL). The combined organic was dried over sodium sulfate (500 g) and then vacuum filtered through a glass frit Buchner funnel with a 20 mm layer of celite. The filter cake was displacement washed with ethyl acetate (250 mL). The clear filtrate was concentrated under reduced pressure to provide a clear pale yellow oil (135 g) as the desired crude product. The material was purified by silica gel column flash chromatography (liquid load) eluting with a gradient of 100% hexane to 60% ethyl acetate in hexane over 1 h collecting 450 mL fractions. The product was detected by TLC analysis on silica gel eluting with 3:1 hexanes/ethyl acetate and visualized under UV. The product fractions were combined and concentrated under reduced pressure to provide a clear, colorless oil (132 g, 1.18 mol, 72% yield containing 16 wt% residual ethyl acetate by NMR) as the desired product, 3-methylenetetrahydropyran-2-one. 1H NMR (400 MHz, dimethyl sulfoxide-d₆) δ 6.18 (q, *J =* 1.9 Hz, 1H), 5.60 (q, *J =* 1.9 Hz, 1H), 4.40 - 4.26 (m, 2H), 2.61 (ddt, *J =* 7.0*,* 6.3, 2.0 Hz, 2H), 1.90 - 1.75 (m, 2H).

### Step 3: 3-(2-Methyl-2-nitro-propyl)tetrahydropyran-2-one

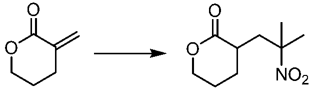

A 5000 mL, 3-neck round bottom flask was fitted with a mechanical stirrer, a cooling bath used as secondary containment, a J-Kem temperature probe, an addition funnel and a nitrogen inlet/outlet. The vessel was charged under a nitrogen atmosphere with 2-nitropropane (104.9 g, 1.177 mol) . Stirring was commenced and the pot temperature was recorded at 19 °C. The vessel was then charged with 1,8-diazabicyclo[5.4.0]undec-7-ene (22.41 g, 147.2 mmol) added neat in one portion which resulted in a clear light yellow solution. No exotherm was observed. The addition funnel was charged with a solution of 3-methylenetetrahydropyran-2-one (110 g, 981.0 mmol) in acetonitrile (1100 mL) which was added dropwise over 1 h which resulted in a clear light yellow solution and a gradual exotherm to 24 °C. The reaction mixture was continued to stir at room temperature for 3.5 h and then concentrated under reduced pressure. The remaining residue was dissolved in dichloromethane (1000 mL) and partitioned with 500 mL of a 3:2 mixture of 1 molar citric acid solution/saturated sodium chloride solution. The resulting organic phase was a clear pale blue solution and the aqueous phase was a slightly cloudy very pale blue solution. The organic was removed and the residual aqueous was extracted with dichloromethane (300 mL). The combined organic was washed with saturated sodium chloride solution (300 mL), dried over sodium sulfate (250 g), and then filtered through a glass frit Buchner funnel. The filtrate was concentrated under reduced pressure to a volume of about 200 mL. The clear pale blue dichloromethane solution was diluted with methyl *tert*-butyl ether (1500 mL) and the cloudy solution was concentrated under reduced pressure to a volume of about 200 mL which provided a suspension. The mixture was again diluted with methyl *tert-*butyl ether (1500 mL) and concentrated under reduced pressure to a volume of about 250 mL. The resulting suspension was allowed to stand at room temperature overnight (about 12 h). The solid was collected by vacuum filtration in a glass frit Buchner funnel and the filter cake was displacement washed with cold methyl tert-butyl ether (2 X 150 mL) and then pulled for 30 min. The material was further dried in a vacuum oven at 45 °C for 5 h to provide (160 g, 0.795 mol, 81% yield) of a white solid as the desired product, 3-(2-methyl-2-nitro-propyl)tetrahydropyran-2-one. 1H NMR (400 MHz, dimethyl sulfoxide-d₆) δ 4.34 (ddd, *J =* 11.1, 9.3, 4.3 Hz, 1H), 4.20 (dt, *J =* 11.1, 5.1 Hz, 1H), 2.75 - 2.62 (m, 1H), 2.56 (dd, *J =* 14.9, 5.2 Hz, 1H), 2.01 - 1.89 (m, 2H), 1.89 - 1.67 (m, 2H), 1.55 (d, *J=* 6.0 Hz, 6H), 1.44 (dddd, *J* = 12.8, 11.5, 8.1, 6.6 Hz, 1H).

### Step 4: Preparation of (S)-3-(2-methyl-2-nitropropyl)tetrahydro-2H-pyran-2-one

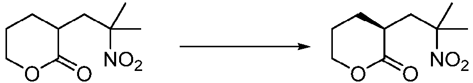

Racemic 3-(2-methyl-2-nitropropyl)tetrahydro-2*H*-pyran-2-one was dissolved in 80 g/L +/- 8 g/L in MeOH/ACN 70/30 v/v (target 80 +/- 2 g/L) and separated on Chiralpak AD 20 µm as the stationary phase using MeOH/ACN 70/30 v/v as the mobile phase. (*S*)-3-(2-Methyl-2-nitropropyl)tetrahydro-2*H*-pyran-2-one is Peak 2.

### Optional recrystallization of (S)-3-(2-methyl-2-nitropropyl)tetrahydro-2H-pyran-2-one :

(*S*)-3-(2-Methyl-2-nitropropyl)tetrahydro-2*H*-pyran-2-one (9.5 kg, 1.0 equiv) was stirred in isopropanol (76 L, 8 vol) then heated to >70 °C to dissolve the solid. The mixture was then cooled to 20 °C over 4-5 h, the solid isolated by filtration, and the cake washed with isopropanol (4.75 L, 0.5 vol) and pulled dry. The material was dried under vacuum to afford (*S*)-3-(2-methyl-2-nitropropyl)tetrahydro-2*H*-pyran-2-one in ~90% recovery.

### Step 5: Synthesis of (S)-3-(3-hydroxypropyl)-5,5-dimethylpyrrolidin-2-one

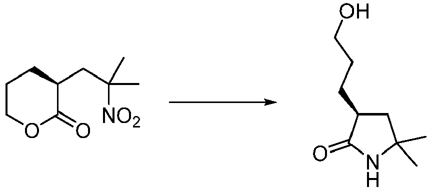

A suspension of Raney Nickel 2400 (77 wt%, 2.8 kg) was allowed to settle for 2 days. The standing liquid was decanted to waste and the remaining catalyst was charged to a reactor with the aid of water (2.6 kg), then degassed with N₂. In a second reactor, a mixture of (*S*)-3-(2-methyl-2-nitropropyl)tetrahydro-2*H*-pyran-2-one (13.9 kg) and EtOH (170.8 kg) was heated to 30 °C, then degassed with N₂, then transferred to the reactor containing Raney Nickel. The transfer was completed with the aid of an EtOH (29.8 kg) rinse. The mixture was purged three times with nitrogen and purged three times with hydrogen. The contents of the reactor were heated to 60-65 °C and stirred under H₂ (4-8 psi) until the reaction was completed (18 h). The mixture was cooled to 15-20 °C, then purged with nitrogen three times, then filtered through a pad of Celite (3.0 kg) wetted with EtOH (3.2 kg). The reactor and Celite cake were washed with EtOH (2 × 14.0 kg). The filtrate was distilled to a final volume of approx. 25 L then heated to 45 °C. MTBE (269.4 kg) was then charged maintaining a temperature of 48-50 °C and then distilled at ambient pressure at 48-55 °C to a final volume of approx. 30 L. Two further portions of MTBE (269.4 kg then 187.4 kg) were sequentially added then concentrated to approx. 30 L volume.

The contents of the reactor were seeded with (*S*)-3-(3-hydroxypropyl)-5,5-dimethylpyrrolidin-2-one (70.1 g) at 40 °C. The seeded crystal slurry was cooled to 15 °C over a period of 3.5 h, then stirred for 16.5 h between 12-15 °C then filtered. The reactor and filter cake were then washed with cold (-2 to -10 °C) MTBE (2 × 10 kg). The filter-cake was dried to a constant weight which afforded (*S*)-3-(3-hydroxypropyl)-5,5-dimethylpyrrolidin-2-one (10.4 kg; 88%) as a white, crystalline solid.

Recrystallization of (*S*)-3-(3-Hydroxypropyl)-5,5-dimethylpyrrolidin-2-one: A mixture of (*S*)-3-(3-hydroxypropyl)-5,5-dimethylpyrrolidin-2-one (10.3 kg) and DCM (28.2 kg) was stirred and heated to 25 °C for 2 h then transferred to another reactor through an in-line filter (45 um). The initial reactor was rinsed with DCM (6.8 kg) at 21 °C for 10 min then transferred to the reactor through the in-line filter. MTBE (38.1 kg) was charged to the solution at 25-30 °C then the mixture was distilled over a period of 2.5 h at 35-52 °C at atmospheric pressure to a final volume of approx. 30 L. MTBE (38.2 kg) was charged to the reactor at 45-50 °C. The resulting suspension was distilled over a period of 3.25 h at 49-55 °C at atmospheric pressure to a final volume of approx. 30 L. The contents of the reactor were cooled to 21 °C over a period of 2.5 h and stirred for 16 h at 20 °C. The suspension was filtered. The reactor and filter cake were rinsed with MTBE (7.7 kg, 0.0 °C). The filter cake was dried over a period of 2 days. Yield: 9.1 kg (88.3 %) of an off-white solid. ¹H NMR (400 MHz, DMSO-d₆) δ 7.63 (s, 1H), 4.38 (s, 1H), 3.38 (t, *J =* 6.5 Hz, 2H), 2.37 (qd, *J =* 9.5, 4.4 Hz, 1H), 2.02 (dd, *J =* 12.4, 8.6 Hz, 1H), 1.78 - 1.63 (m, 1H), 1.50 - 1.33 (m, 3H), 1.16 (d, *J =* 17.9 Hz, 7H). ESI-MS m/z calc. 171.12593, found 172.0 [M+1]⁺. GCMS:100 % (AUC). Chiral HPLC: 100 % (AUC).

### Step 6: Synthesis of (S)-3-(5,5-dimethylpyrrolidin-3-yl)propan-1-ol

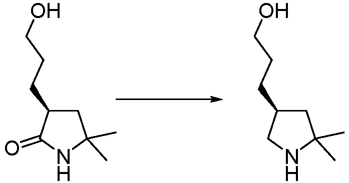

LiAlH₄ pellets (332.5 g, 8.760 mol, 1.50 equiv) were slowly added to a reactor with 2-MeTHF (10.00 L, 10 vol) at 30-40 °C. The mixture was then heated to 75 °C. A mixture of (S)-3-(3-hydroxypropyl)-5,5-dimethylpyrrolidin-2-one (1,000 g, 5.840 mol, 1.00 equiv) and 2-MeTHF (10.00 L, 10 vol) was prepared in a separate reactor and heated to 65 °C, which was then carefully transferred to the reactor containing the LiAlH₄ mixture over 2 h. The mixture was stirred at 70 °C until the reaction was complete (18-24 h) then cooled to 0-10 °C. Water (400.0 mL, 1 x LiAlH₄ wt) was then carefully added while maintaining the mixture temperature at <30 °C. A solution of aq 15% NaOH (400.0 mL, 1x LiAlH₄ wt) was then added followed by water (400.0 mL, 1x LiAlH₄ wt) while maintaining the mixture temperature at <30 °C. The resulting mixture was then heated to 60 °C and held at that temperature for at least 30 min. The mixture was cooled to 20-30 °C then Celite (200 grams, 20 wt%) was added. The mixture was then filtered through a pad of Celite. The reactor and filter cake were rinsed with 2-MeTHF (4.0 L, 4.0 vol). The filtrate was concentrated under vacuum to afford pyrrolidine compound (S)-3-(5,5-dimethylpyrrolidin-3-yl)propan-1-ol (872 g; 94.95 % yield) as a clear oil.

¹H NMR (400 MHz, dimethyl sulfoxide-d₆) δ 3.36 (t, *J =* 6.3 Hz, 3H), 2.95 (dd, *J* = 10.6, 7.6 Hz, 1H), 2.40 (dd, J = 10.6, 7.7 Hz, 1H), 2.12 - 1.97 (m, 1H), 1.69 (dd, *J =* 12.1, 8.2 Hz, 1H), 1.47 - 1.25 (m, 5H), 1.08 (s, 3H), 1.02 (s, 3H).

### Step 7: Synthesis of (S)-6-bromo-2-(4-(3-hydroxypropyl)-2,2-dimethylpyrrolidin-1-yl)nicotinamide

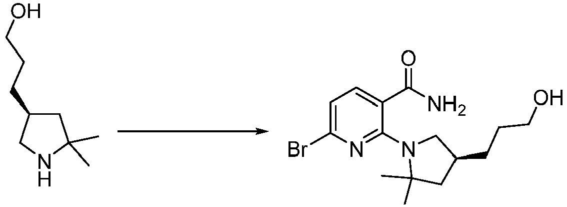

A mixture of (*S*)-3-(5,5-dimethylpyrrolidin-3-yl)propan-1-ol (2325 g, 14.8 mol) and 6-bromo-2-fluoropyridine-3-carboxamide (3400 g, 15.5 mol) in 2-methyltetrahydrofuran (23 L) was stirred, then potassium carbonate (2650 g, 19.2 mol) and deionized water (7 L) were added. The mixture was stirred at <25 °C until the reaction was complete (≥16 h).

The aqueous phase was removed and the upper organic phase was washed with water (7 L) and 2% aqueous sodium chloride (7 L). The organic layer was concentrated under reduced pressure to about 19 L. 2-Methyltetrahydrofuran was chased from the mixture by two sequential additions and concentrations of acetonitrile (2 x 20 L) followed by distillation. To the remaining solution was added acetonitrile (20 L) and the reaction was warmed to 85 °C for 2 h and then cooled at 10 °C/h to 25 °C. The slurry was cooled to 10 °C and stirred for 4 h then filtered. The cake was rinsed two times with acetonitrile (2 x 3 L) then the solid was dried under vacuum to afford (*S*)-6-bromo-2-(4-(3-hydroxypropyl)-2,2-dimethylpyrrolidin-1-yl)nicotinamide as a crystalline white solid (3850 g, 73 % yield).

1H NMR (400 MHz, DMSO-d₆) δ 7.78 (s, 1H), 7.39 (s, 1H), 7.34 (dd, *J* = 7.7*,* 1.0 Hz, 1H), 6.67 (d, *J =* 7.6 Hz, 1H), 4.42 (t, *J =* 5.1 Hz, 1H), 3.39 (q, *J =* 5.7 Hz, 2H), 3.29 - 3.12 (m, 2H), 2.19 (dt, *J =* 10.9, 5.8 Hz, 1H), 1.92 (dd, *J =* 11.9, 5.7 Hz, 1H), 1.53 (s, 3H), 1.50 (s, 3H), 1.48 - 1.29 (m, 5H).

### Step 8: Synthesis of (S)-6-bromo-2-(4-(3-(1,3-dioxoisoindolin-2-yl)propyl)-2,2-dimethylpyrrolidin-1-yl)nicotinamide

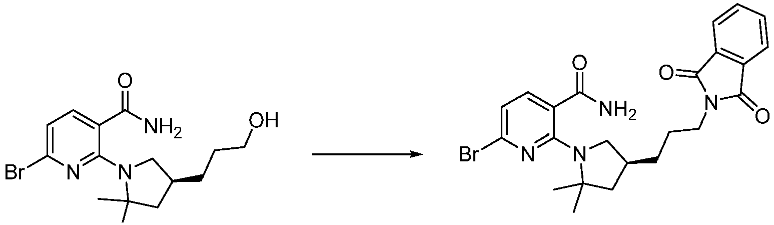

A mixture of (*S*)-6-bromo-2-(4-(3-hydroxypropyl)-2,2-dimethylpyrrolidin-1-yl)nicotinamide (2.65 kg, 7.4 mol), 2-methyltetrahydrofuran (16 L), and triethylamine (900 g, 8.88 mol) was stirred at 20 °C, then methanesulfonyl chloride (933 g, 8.14 mol) was added over 2 h. The mixture was stirred at 20 °C until the reaction was completed (typically 16 h). The resulting mixture was filtered and the filter cake was rinsed with *tert*-butyl methyl ether (2 x 4L). The combined filtrates (containing the mesylate intermediate) were transferred to a reactor and diluted with dimethyl sulfoxide (16 L). To the mixture was added phthalimide (1198 g, 8.14 mol). The mixture was stirred until a solution was obtained, then potassium carbonate (1023 g, 7.4 mol) was added and the mixture was stirred and heated to 70 °C until the reaction was completed (2 h). The mixture was cooled to 20 °C and diluted with 2-methyltetrahydrofuran (16 L), followed by the addition of deionized water (21 L). The phases were separated and the upper organic phase was washed with deionized water (10 L) and saturated aqueous sodium chloride (2 x 1 L). The organic phase was diluted with toluene (16 L) and concentrated under reduced pressure to approximately 10 L volume. The solid was isolated by filtration and the filter cake was rinsed with toluene (2 x 2 L). The resulting solid was dried to afford compound (*S*)-6-bromo-2-(4-(3-(1,3-dioxoisoindolin-2-yl)propyl)-2,2-dimethylpyrrolidin-1-yl)nicotinamide as an off-white solid (3393 g, 6.99 mol, 94 % yield).

¹H NMR (400 MHz, DMSO-d₆) δ 7.91 - 7.80 (m, 4H), 7.80 - 7.71 (m, 1H), 7.42 - 7.36 (m, 1H), 7.34 (d, *J* = 7.7 Hz, 1H), 7.29 - 7.09 (m, 3H), 6.67 (d, *J =* 7.7 Hz, 1H), 3.59 (t, *J* = 6.9 Hz, 2H), 3.23 (t, *J =* 10.4 Hz, 1H), 3.16 (dd, *J =* 10.2, 7.4 Hz, 1H), 2.30 (s, 2H), 2.28 - 2.13 (m, 1H), 1.90 (dd, *J =* 12.0, 5.6 Hz, 1H), 1.71 - 1.53 (m, 2H), 1.51 (s, 3H), 1.48 (s, 3H), 1.47 - 1.23 (m, 3H).

### Step 9: Synthesis of perfluorophenyl 6-fluoropyridine-2-sulfonate

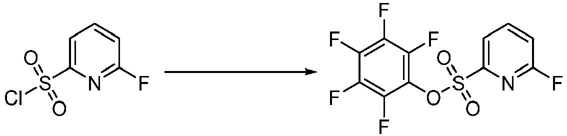

To a solution of 2,3,4,5,6-pentafluorophenol (33.21 g, 180.4 mmol) in iPrOAc (175 mL) was added aq KHCO₃ (approximately 90.30 mL of 20 %w/v, 180.4 mmol). The mixture was stirred at ambient temperature for 10 min. A solution of 6-fluoropyridine-2-sulfonyl chloride (35.29 g, 180.4 mmol) in iPrOAc (25 mL) was then added while keeping the temperature below 20 °C. The reaction mixture was allowed to stir at ambient temperature for 1 hr to complete the reaction. The aqueous layer was removed, and the organic layer was washed with water (50 mL), dried over Na₂SO₄, then concentrated by rotary evaporation to remove most of solvent and precipitate a white solid. Heptane (70 mL) was added and the slurry was stirred at ambient temperature. The solid was collected by filtration, rinsed with heptane (30 mL), and dried under vacuum to give 58.76 g of perfluorophenyl 6-fluoropyridine-2-sulfonate as a white solid. The mother liquor was concentrated by rotary evaporation, then 1 mL (iPrOAc)/ 20 mL (Heptane) was added and stirred at ambient temperature overnight. The resulting solid was collected and dried under vacuum oven at ambient temperature overnight with a N₂ bleed to give 0.87 g as a second crop. In total, 59.63 g (96% yield) of perfluorophenyl 6-fluoropyridine-2-sulfonate was obtained. ¹H NMR (400 MHz, DMSO-*d*₆) δ 8.46 (q, *J =* 7.7 Hz, 1H), 8.20 (dd, *J* = 7.5, 2.0 Hz, 1H), 7.84 (dd, *J* = 8.3, 2.2 Hz, 1H). ¹⁹F NMR (376 MHz, DMSO-d6) δ -64.25 (s, 1F), -152.20 - -152.69 (m, 2F), -154.84 (t, *J =* 23.3 Hz, 2F), -161.00 - -161.74 (m, 1F).

### Step 10: Synthesis of (S)-6-bromo-2-(4-(3-(1,3-dioxoisoindolin-2-yl)propyl)-2,2-dimethylpyrrolidin-1-yl)-N-((6-fluoropyridin-2-yl)sulfonyl)nicotinamide

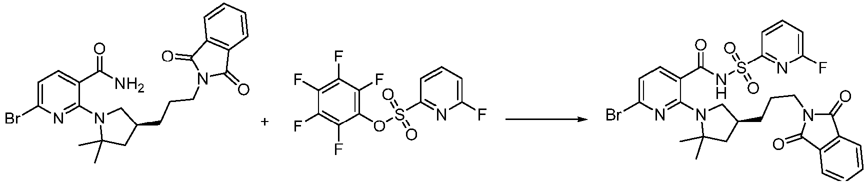

A solution of lithium *tert*-butoxide (1,905.13 g, 2.20 equiv, 20 % w/w in THF) was added to a mixture of (S)-6-bromo-2-(4-(3-(1,3-dioxoisoindolin-2-yl)propyl)-2,2-dimethylpyrrolidin-1-yl)nicotinamide (1,050g, 1.00 equiv) and perfluorophenyl 6-fluoropyridine-2-sulfonate (890.93g, 1.20 equiv) in 2-MeTHF (10.5 L, 10.0 vol), keeping the internal temperature below -2 °C. The mixture was stirred at -10 °C until the reaction was complete (typically 0.5 h). A solution of aq HCl (5.25 L, 5 vol, 1 N) was added while keeping the internal temperature < 20 °C. 2-MeTHF (5.0 L, 5 vol) was then added to the reactor at ambient temperature and then the phases were allowed to separate. The lower aqueous layer was discarded. The organic phase was washed with water (5 vol) then concentrated to 5 volumes under vacuum keeping the temperature below 50 °C. 2-MeTHF (10.0 L, 10 vol) was added and again the mixture concentrated to 5 volumes. 2-MeTHF (5 vol) was added and the heterogenous mixture was heated to 70 °C with agitation until complete dissolution occurred. The mixture was then cooled linearly to 45 °C over 6 h then held at 45 °C for 6 h. The mixture was then cooled to 25 °C over 2 h then heptane (10.0 L, 10 vol) was added to the mixture over 3 h. The mixture was drained from the reactor and the solids were isolated. The reactor and filter cake were then washed twice with a mixture of 2-MeTHF (2.0 L, 2 vol) and heptane (2.0 L, 2 vol). The solids were dried under vacuum at 45 °C to afford (*S*)-6-bromo-2-(4-(3-(1,3-dioxoisoindolin-2-yl)propyl)-2,2-dimethylpyrrolidin-1-yl)-*N*-((6-fluoropyridin-2-yl)sulfonyl)nicotinamide. ¹H NMR (400 MHz, DMSO-*d*₆) δ 13.05 (s, 1H), 8.37 (q, *J =* 7.8 Hz, 1H), 8.11 (dd, *J* = 7.4*,* 1.9 Hz, 1H), 7.95 - 7.88 (m, 2H), 7.88 - 7.81 (m, 2H), 7.59 (dd, *J* = 8.1*,* 2.4 Hz, 2H), 6.78 (d, *J =* 7.9 Hz, 1H), 3.59 (t, *J =* 6.8 Hz, 2H), 2.45 (dd, *J =* 8.8, 3.9 Hz, 2H), 2.17 (s, 1H), 1.87 (dd, *J =* 11.9, 5.5 Hz, 1H), 1.56 (dddd, *J =* 22.3, 16.7, 8.9, 4.1 Hz, 2H), 1.47 (s, 3H), 1.44 (s, 3H), 1.35 (t, *J =* 12.1 Hz, 1H), 1.21 (ddd, *J =* 13.3, 10.5, 5.4 Hz, 1H), 1.00 (dtd, *J =* 14.1, 9.4, 5.7 Hz, 1H).

### Step 11: Synthesis of (S)-2-((3-(1-(6-bromo-3-(((6-fluoropyridin-2-yl)sulfonyl)carbamoyl)pyridin-2-yl)-5,5-dimethylpyrrolidin-3-yl)propyl)carbamoyl)benzoic acid

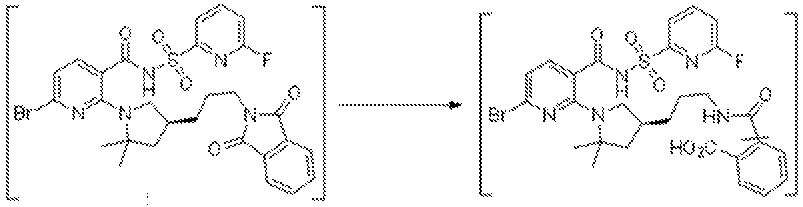

The reaction solution containing (*S*)-6-bromo-2-(4-(3-(1,3-dioxoisoindolin-2-yl)propyl)-2,2-dimethylpyrrolidin-1-yl)-N-((6-fluoropyridin-2-yl)sulfonyl)nicotinamide from the preceding step was cooled and maintained below 10 °C when a solution of LiOH•H₂O (284 g, 6.77 mol; 3 equiv) in water (2.19 L; 2 volumetric equiv) was added. The biphasic mixture was stirred at 5 - 15 °C until the reaction was completed (about 2 h). While maintaining the reaction temperature below 10 °C, 2 M HCl (5.64 L, 11.3 mol; 5 equiv) was added dropwise over ~1 h. The pH of the aqueous phase was about 2. The phases were separated then the organic phase was concentrated to a minimum volume removing most of the 2-MeTHF (40 °C/150 - 70 torr). The reaction mixture was advanced to the next step without any further processing.

### Step 12: Synthesis of (S)-2-(4-(3-aminopropyl)-2,2-dimethylpyrrolidin-1-yl)-6-bromo-N-((6-fluoropyridin-2-yl)sulfonyl)nicotinamide

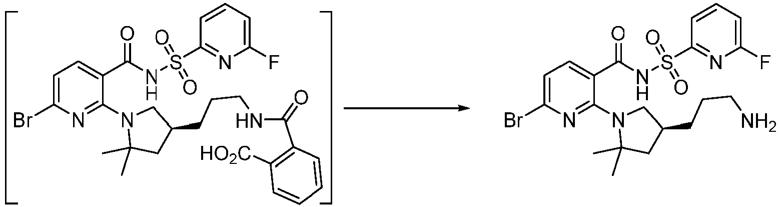

The concentrate containing (*S*)-2-((3-(1-(6-bromo-3-(((6-fluoropyridin-2-yl)sulfonyl)carbamoyl)pyridin-2-yl)-5,5-dimethylpyrrolidin-3-yl)propyl)carbamoyl)benzoic acid from the preceding step was diluted with CH₃CN (6.56 L; 6 volumetric equiv) and water (3.83 L; 2 volumetric equiv) then oxalic acid (508 g, 5.64 mol; 2.5 equiv) was added and the resultant solution was heated at 60 °C until the reaction was complete (about at least 4 h). The solution was cooled to 0 - 10 °C then a solution of K₂CO₃ (2.18 kg, 15.8 mol; 7 equiv) in water (3.83 L; 3.5 volumetric equiv) was added dropwise while maintaining the reaction temperature below 10 °C. The solid was collected by filtration. The damp filter-cake was washed consecutively with water (2 x 2.2 L; 2 volumetric equiv) and then *i*-PrOH (2 x 600 mL; 0.5 volumetric equiv), air-dried with suction, and vacuum-dried (50 °C/30 torr) to afford (S)-2-(4-(3-aminopropyl)-2,2-dimethylpyrrolidin-1-yl)-6-bromo-*N*-((6-fluoropyridin-2-yl)sulfonyl)nicotinamide (959 g; 83% for 3 steps; >98% AUC) as a fine, white powder. ¹H NMR (400 MHz, DMSO-d₆) δ 8.09 (q, *J =* 7.9 Hz, 1H), 7.83 (dd, *J =* 7.5, 2.2 Hz, 1H), 7.67 (s, 3H), 7.39 (d, *J* = 7.6 Hz, 1H), 7.23 (dd, *J =* 8.2,2.4 Hz, 1H), 6.58 (d, *J =* 7.6 Hz, 1H), 3.20 - 2.99 (m, 2H), 2.81 (td, *J* = 7.2, 4.7 Hz, 2H), 2.08 (dh, *J =* 15.3, 7.0 Hz, 1H), 1.84 (dd, *J =* 11.8, 5.7 Hz, 1H), 1.54 (q, *J =* 7.6 Hz, 2H), 1.48 (s, 3H), 1.47 (s, 3H), 1.37 (t, *J* = 11.9 Hz, 1H), 1.26 (ddd, *J* = 29.1, 13.8, 7.4 Hz, 2H).

### Step 13: Synthesis of (14S)-8-bromo-12,12-dimethyl-2λ⁶-thia-3,9,11,18,23-pentaazatetracyclo[17.3.1.1^{11,14}.0^{5,10}]tetracosa-1(23),5,7,9,19,21-hexaene-2,2,4-trione

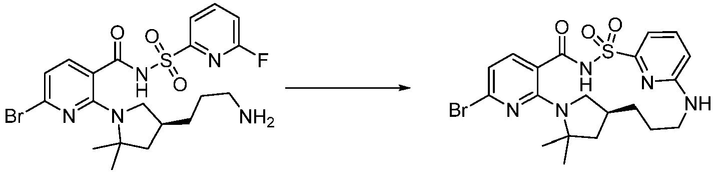

A mixture of (*S*)-2-(4-(3-aminopropyl)-2,2-dimethylpyrrolidin-1-yl)-6-bromo-*N-*((6-fluoropyridin-2-yl)sulfonyl)nicotinamide (950 g, 1.85 mol) and Na₂CO₃ (392 g, 3.69 mol; 2 equiv) in DMSO (7.60 L; 8 volumetric equiv) was heated at 85 °C until the reaction was completed (~6 h). The suspension was cooled to <15 °C and diluted with MeTHF (19.0 L; 20 volumetric equiv). Water (13.3 L) was added slowly while maintaining the reaction temperature <15 °C. While maintaining the reaction temperature <15 °C, 2 M HCl (4.62 L, 9.24 mol; 5 equiv) was added (pH ~2). The phases were separated and the organic phase was washed twice with water (9.50 L; 10 volumetric equiv) containing NaCl (190 g; 2 wt%). The organic phase was concentrated to a minimum volume (45 °C/180 torr) and chased with i-PrOAc (2-3 x 500 mL) to remove the MeTHF. The concentrate was backfilled with *i*-PrOAc (3.800 L; 4 volumetric equiv) and agitated at 45 °C until crystallization occurred. (The mixture may be seeded with (14*S*)-8-bromo-12,12-dimethyl-2λ⁶-thia-3,9,11,18,23-pentaazatetracyclo[17.3.1.1^{11,14}.0^{5,10}]tetracosa-1(23),5,7,9,19,21-hexaene-2,2,4-trione if necessary). The suspension was aged with agitation for at least 30 min and then allowed to cool to 20 °C. After aging at 20 °C for at least 2 h, the solid was collected by filtration. The filter-cake was washed with 1:1 *i*-PrOAc/MTBE (500-mL), air-dried with suction, and vacuum-dried (40 - 55 °C/<100 torr/N₂ bleed) to afford (14*S*)-8-bromo-12,12-dimethyl-2λ⁶-thia-3,9,11,18,23-pentaazatetracyclo[17.3.1.1^{11,14}.0^{5,10}]tetracosa-1(23),5,7,9,19,21-hexaene-2,2,4-trione •0.8 *i*-PrOAc (830 g; 78% yield corrected for *i*-PrOAc solvate) as a white powder with a slight yellow tint.

A second crop was obtained by concentrating the filtrate to ~400 mL total volume. The mixture was then seeded and aged at 15 - 20 °C. The solid was collected by filtration. The filter-cake was washed successively with 1:1 *i*-PrOAc/MTBE (200 mL) and MTBE (100 mL), air-dried with suction, and vacuum-dried (55 °C/<100 torr/N₂ bleed) to afford (14*S*)-8-bromo-12,12-dimethyl-2λ⁶-thia-3,9,11,18,23-pentaazatetracyclo[17.3.1.1^{11,14}.0^{5,10}]tetracosa-1(23),5,7,9,19,21-hexaene-2,2,4-trione•0.67 *i-*PrOAc (113 g; 11% corrected yield) as a pale yellow solid. 1H NMR (400 MHz, Chloroform-*d*) δ 9.13 (s, 1H), 7.66 (d, *J =* 7.9 Hz, 1H), 7.54 (dd, *J =* 8.4, 7.3 Hz, 1H), 7.43 (d, *J =* 7.3 Hz, 1H), 6.79 (d, *J =* 7.9 Hz, 1H), 6.56 (d, *J =* 8.4 Hz, 1H), 4.99 (hept, *J =* 6.3 Hz, 1H), 4.57 (d, *J* = 8.8 Hz, 1H), 4.02 - 3.85 (m, 1H), 3.27 - 3.09 (m, 2H), 2.96 (t, *J =* 10.2 Hz, 1H), 2.35 (p, *J =* 9.5 Hz, 1H), 2.02 (s, 3H), 1.95 (dd, *J =* 12.1, 6.7 Hz, 1H), 1.72 - 1.59 (m, 6H), 1.58 (s, 3H), 1.55 (s, 3H), 1.43 (d, *J* = 40.1 Hz, 1H), 1.23 (d, *J =* 6.3 Hz, 5H).

### Part B. Synthesis of vanzacaftor free form A

A reactor was equipped with an overhead stirrer, reflux condenser, N₂ bubble line and outlet, and a temperature probe. A mixture of (14*S*)-8-bromo-12,12-dimethyl-2λ⁶-thia-3,9,11,18,23-pentaazatetracyclo[17.3.1.1^{11,14}.0^{5,10}]tetracosa-1(23),5,7,9,19,21-hexaene-2,2,4-trione (120 g of 86% w/w with IPAc [103.2 g (14*S*)-8-bromo-12,12-dimethyl-2λ⁶-thia-3,9,11,18,23-pentaazatetracyclo[17.3.1.1^{11,14}.0^{5,10}]tetracosa-1(23),5,7,9,19,21-hexaene-2,2,4-trione], 0.21 mol, 1 equiv), 3-(2-(dispiro[2.0.2⁴.1³]heptan-7-yl)ethoxy)-1*H*-pyrazole (42.6 g, 0.21 mol, 1 equiv), 325 mesh K₂CO₃ (63.4 g, 0.46 mol, 2.2 equiv), CuI (3.3 g, 17.2 mmol, 0.083 equiv) and BuOAc (740 mL) were charged into a reactor. The mixture was stirred at ambient temperature. Then DMF (300 mL, 2.9 vol) and *N,N'*-dimethylcyclohexane-1,2-diamine (14.6 g or 16.2 ml, 0.1 mol, 0.49 equiv) were charged to the reactor and the mixture was purged with three N₂/vacuum/N₂ cycles. The mixture was then heated to 120 °C for 4 h, then allowed to cool to ambient temperature. 10% aq w/v oxalic acid (860 mL, 0.96 mol, 4.6 equiv) was added dropwise and the mixture stirred for at least 1 h. The mixture was then filtered to remove suspended solids. The removed solids were washed with (2 x 120 mL). The layers from the filtrate were separated. The organic layer was washed with 8% aq. w/v trisodium citrate (600 mL). Brine was added as necessary to aid phase separation. The organic layer was washed with 1:1 v/v water/brine (400 mL). The organic layer was filtered through a pad of Celite. The filter pad was washed with IPAc (150 mL). The filtrate was concentrated, then 800 mL of 1-PrOH (7.8 vol) was added and the mixture concentrated. This step was repeated one more time. Toluene (800 mL) was added and the mixture concentrated. This step was repeated one more time to afford a thick slurry. The crude mixture was concentrated to a volume of 300 mL (2.9 vol) of toluene. After stirring the slurry overnight, the solid was collected by filtration and washing the solid with toluene (2 x 100 mL, 0.97 vol). The solid was dried under vacuum with a nitrogen bleed at 50 °C until the loss on drying was no more than 1.0% to afford vanzacaftor as a white/off-white solid (107.0 g, 83%, 94.5% (AUC) HPLC purity.

Recrystallization: vanzacaftor Form A [22.2 g, 94.6% (AUC) vanzacaftor Form A was suspended in toluene (440 mL, 20 vol based on vanzacaftor Form A) and the mixture heated to reflux. After holding at reflux for 2 h, the mixture was allowed to cool to ambient over 8 h. After stirring at ambient temperature overnight, the solid was collected by filtration washing the solid with toluene (40 mL, 1.8 vol). The solid was dried under vacuum with a nitrogen bleed at 50 °C until the loss on drying was no more than 1.0% to afford vanzacaftor Form A as a white/off-white solid (18.8 g, 84%, 96.8% (AUC) HPLC purity).

Second Recrystallization: vanzacaftor Form A [17.5 g, 97.0% (AUC) vanzacaftor Form A] was suspended in toluene (350 mL, 20 vol based on vanzacaftor Form A) and the mixture heated to reflux. After holding at reflux for no less than 2 h, the mixture was allowed to cool to ambient temperature over 8 h. After stirring at ambient temperature overnight, the solid was collected by filtration washing the solid with toluene (40 mL, 1.8 vol). The solid was dried under vacuum with a nitrogen bleed at 50 °C until the loss on drying was no more than 1.0% to afford vanzacaftor Form A (free form) as a white/off-white solid (15.7 g, 89%, 98.4% (AUC) HPLC purity).

### Part C. Synthesis of vanzacaftor calcium salt hydrate Form A

Vanzacaftor calcium salt hydrate Form A was prepared by charging 0.2 mmol of vanzacaftor free Form A and 0.1 mmol of Ca(OMe)₂ dry powder with IPA at ~45 mg/mL and spiked with ~10% of water and heated to 70 °C. Initially, all solids dissolved. After 5 min, white solid precipitated out. The resulting slurry was stirred for 4 d at room temperature. The solid was isolated as vanzacaftor calcium salt hydrate Form A by vacuum filtration and dried under vacuum at 40 °C for overnight (~ 78% isolated yield).

An alternative method of preparing vanzacaftor calcium salt hydrate Form A utilized 10 g of vanzacaftor free Form A charged with 63 mL IPA and 7 mL water. The slurry was heated to 55-65 °C. The mixture was charged with 1.1 equiv of NaOH. The mixture was stirred until the solution turned homogeneous. The solution was then cooled to 25 °C and seeded with 0.1 g of vanzacaftor sodium salt hydrate Form A. The slurry was stirred for 18 h. The solution was then heated to 45 °C. The slurry was seeded with 0.1 g of vanzacaftor calcium salt hydrate Form A. A solution of 0.55 equiv CaCl₂, 9 mL IPA, and 1 mL water were added over a 5 h period of time. The resulting slurry was stirred for 2 h. The slurry was cooled to 20 °C over a 5 h period of time. The resulting solids were collected by vacuum filtration and the resulting wet cake was washed with 50 mL of water. The washed wet cake was allowed to air-dry for 1 h. The air-dried wet cake was transferred to a vacuum oven at 45 °C with a slight nitrogen bleed for 20 h to yield crystalline vanzacaftor calcium salt hydrate Form A (8.5 g, 82% isolated yield).

### Part D. Synthesis of vanzacaftor calcium salt hydrate Form D

Approximately 25 mg of vanzacaftor calcium salt hydrate Form A was charged with 0.5 mL of EtOH:water 67:33 w/w. The slurry was heated to 65 °C for 8 d. The resulting solid collected by vacuum filtration was vanzacaftor calcium salt hydrate Form D.

Alternatively, vanzacaftor calcium salt hydrate Form D was prepared from 89 g of vanzacaftor sodium hydrate Form A charged with 1080 mL IPA and 120 mL water. The slurry was heated to 55-65 °C. The slurry was charged with 18 g of vanzacaftor calcium salt hydrate Form D seed. The slurry was wet-milled as a solution of 0.55 equiv CaCl₂, 81 mL IPA and 9 mL water was added over a 5 h period of time. The wet mill was allowed to run until the X-ray powder diffraction confirmed that the slurry was all vanzacaftor calcium salt hydrate Form D. The resulting solids were collected by vacuum filtration and wet cake was washed with 350 mL of water. The washed wet cake was allowed to air-dry for 1 h. The air-dried wet cake was transferred to a vacuum oven at 45 °C with a slight nitrogen bleed for 20 h to yield crystalline vanzacaftor calcium salt hydrate Form D (83.15 g, 90.6% isolated yield).

### Example 2: Synthesis of (R)-1-(2,2-Difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide (tezacaftor)

The synthesis of tezacaftor is described in United States Patent Publication US 2009/0131492. The production of solid *d*ispersions of tezacaftor is described in International Patent Publications WO 2011/119984 and WO 2015/160787.

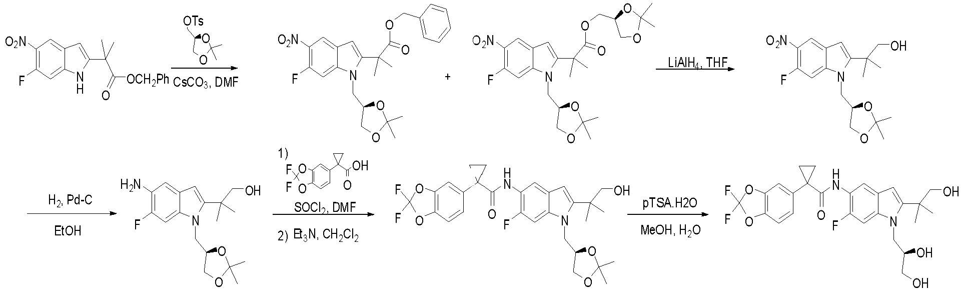

### Step 1: (R)-Benzyl 2-(1-((2,2-dimethyl-1,3-dioxolan-4-yl)methyl)-6-fluoro-5-nitro-1H-indol-2-yl)-2-methylpropanoate and ((S)-2,2-dimethyl-1,3-dioxolan-4-yl)methyl 2-(1-(((R)-2,2-dimethyl-1,3-dioxolan-4-yl)methyl)-6-fluoro-5-nitro-1H-indol-2-yl)-2-methylpropanoate

Cesium carbonate (8.23 g, 25.3 mmol) was added to a mixture of benzyl 2-(6-fluoro-5-nitro-1*H*-indol-2-yl)-2-methylpropanoate (3.0 g, 8.4 mmol) and (S)-(2,2-dimethyl-1,3-dioxolan-4-yl)methyl 4-methylbenzenesulfonate (7.23 g, 25.3 mmol) in DMF (*N,N-*dimethylformamide) (17 mL). The reaction was stirred at 80 °C for 46 hours under a nitrogen atmosphere. The mixture was then partitioned between ethyl acetate and water. The aqueous layer was extracted with ethyl acetate. The combined ethyl acetate layers were washed with brine, dried over MgSO₄, filtered, and concentrated. The crude product, a viscous brown oil which contains both of the products shown above, was taken directly to the next step without further purification. (*R*)-Benzyl 2-(1-((2,2-dimethyl-1,3-dioxolan-4-yl)methyl)-6-fluoro-5-nitro-1*H*-indol-2-yl)-2-methylpropanoate, ESI-MS *m*/*z* calc. 470.2, found 471.5 (M+1)⁺. Retention time 2.20 minutes. ((*S*)-2,2-Dimethyl-1,3-dioxolan-4-yl)methyl 2-(1-(((*R*)-2,2-dimethyl-1,3-dioxolan-4-yl)methyl)-6-fluoro-5-nitro-1*H*-indol-2-yl)-2-methylpropanoate, ESI-MS *m*/*z* calc. 494.5, found 495.7 (M+1)⁺. Retention time 2.01 minutes.

### Step 2: (R)-2-(1-((2,2-dimethyl-1,3-dioxolan-4-yl)methyl)-6-fluoro-5-nitro-1H-indol-2-yl)-2-methylpropan-1-ol

The crude reaction mixture obtained in step 1 was dissolved in THF (42 mL) and cooled in an ice-water bath. LiAlH₄ (16.8 mL of 1 M solution, 16.8 mmol) was added drop-wise. After the addition was complete, the mixture was stirred for an additional 5 minutes. The reaction was quenched by adding water (1 mL), 15% NaOH solution (1 mL) and then water (3 mL). The mixture was filtered over Celite, and the solids were washed with THF and ethyl acetate. The filtrate was concentrated and purified by column chromatography (30-60% ethyl acetate- hexanes) to obtain (*R*)-2-(1-((2,2-dimethyl-1,3-dioxolan-4-yl)methyl)-6-fluoro-5-nitro-1*H*-indol-2-yl)-2-methylpropan-1-ol as a brown oil (2.68g, 87 % over 2 steps). ESI-MS *m*/*z* calc. 366.4, found 367.3 (M+1)⁺. Retention time 1.68 minutes. 1H NMR (400 MHz, DMSO-*d*₆) δ 8.34 (d, *J =* 7.6 Hz, 1H), 7.65 (d, *J =* 13.4 Hz, 1H), 6.57 (s, 1H), 4.94 (t, *J =* 5.4 Hz, 1H), 4.64 - 4.60 (m, 1H), 4.52 - 4.42 (m, 2H), 4.16 - 4.14 (m, 1H), 3.76 - 3.74 (m, 1H), 3.63 - 3.53 (m, 2H), 1.42 (s, 3H), 1.38 - 1.36 (m, 6H) and 1.19 (s, 3H) ppm. (DMSO is dimethylsulfoxide).

### Step 3: (R)-2-(5-amino-1-((2,2-dimethyl-1,3-dioxolan-4-yl)methyl)-6-fluoro-1H-indol-2-yl)-2-methylpropan-1-ol

(*R*)-2-(1-((2,2-dimethyl-1,3-dioxolan-4-yl)methyl)-6-fluoro-5-nitro-1*H*-indol-2-yl)-2-methylpropan-1-ol (2.5 g, 6.82 mmol) was dissolved in ethanol (70 mL) and the reaction was flushed with N₂. Then Pd-C (250 mg, 5% wt) was added. The reaction was flushed with nitrogen again and then stirred under H₂ (atm). After 2.5 hours only partial conversion to the product was observed by LCMS. The reaction was filtered through Celite and concentrated. The residue was re-subjected to the conditions above. After 2 hours LCMS indicated complete conversion to product. The reaction mixture was filtered through Celite. The filtrate was concentrated to yield the product (1.82 g, 79 %). ESI-MS *m*/*z* calc. 336.2, found 337.5 (M+1)⁺. Retention time 0.86 minutes. 1H NMR (400 MHz, DMSO-*d*6) δ 7.17 (d, *J =* 12.6 Hz, 1H), 6.76 (d, *J =* 9.0 Hz, 1H), 6.03 (s, 1H), 4.79 - 4.76 (m, 1H), 4.46 (s, 2H), 4.37 - 4.31 (m, 3H), 4.06 (dd, *J =* 6.1, 8.3 Hz, 1H), 3.70 - 3.67 (m, 1H), 3.55 - 3.52 (m, 2H), 1.41 (s, 3H), 1.32 (s, 6H) and 1.21 (s, 3H) ppm.

### Step 4: (R)-1-(2,2-difluorobenzo[d][1,3]dioxol-5-yl)-N-(1-((2,2-dimethyl-1,3-dioxolan-4-yl)methyl)-6-fluoro-2-(1-hydroxy-2-methylpropan-2-yl)-1H-indol-5-yl)cyclopropanecarboxamide

DMF (3 drops) was added to a stirring mixture of 1-(2,2-difluorobenzo[*d*] [1,3] dioxol-5-yl)cyclopropanecarboxylic acid (1.87 g, 7.7 mmol) and thionyl chloride (1.30 mL, 17.9 mmol). After 1 hour a clear solution had formed. The solution was concentrated under vacuum and then toluene (3 mL) was added and the mixture was concentrated again. The toluene step was repeated once more and the residue was placed on high vacuum for 10 minutes. The acid chloride was then dissolved in dichloromethane (10 mL) and added to a mixture of (*R*)-2-(5-amino-1-((2,2-dimethyl-1,3-dioxolan-4-yl)methyl)-6-fluoro-1*H*-indol-2-yl)-2-methylpropan-1-ol (1.8 g, 5.4 mmol) and triethylamine (2.24 mL, 16.1 mmol) in dichloromethane (45 mL). The reaction was stirred at room temperature for 1 hour. The reaction was washed with 1N HCl solution, saturated NaHCO₃ solution and brine, dried over MgSO₄ and concentrated to yield the product (3g, 100%). ESI-MS *m*/*z* calc. 560.6, found 561.7 (M+1)⁺. Retention time 2.05 minutes. ¹H NMR (400 MHz, DMSO-*d*₆) δ 8.31 (s, 1H), 7.53 (s, 1H), 7.42 - 7.40 (m, 2H), 7.34 - 7.30 (m, 3H), 6.24 (s, 1H), 4.51 - 4.48 (m, 1H), 4.39 - 4.34 (m,2H), 4.08 (dd, *J =* 6.0, 8.3 Hz, 1H), 3.69 (t*, J =* 7.6 Hz, 1H), 3.58 - 3.51 (m, 2H), 1.48 - 1.45 (m, 2H), 1.39 (s, 3H), 1.34 - 1.33 (m, 6H), 1.18 (s, 3H) and 1.14 - 1.12 (m, 2H) ppm.

### Step 5: (R)-1-(2,2-difluorobenzo[d][1,3]dioxol-5-yl)-N-(1-(2,3-dihydroxypropyl)-6-fluoro-2-(1-hydroxy-2-methylpropan-2-yl)-1H-indol-5-yl)cyclopropanecarboxamide (tezacaftor)

(*R*)-1-(2,2-difluorobenzo[*d*][1,3]dioxol-5-yl)-*N*-(1-((2,2-dimethyl-1,3-dioxolan-4-yl)methyl)-6-fluoro-2-(1-hydroxy-2-methylpropan-2-yl)-1*H*-indol-5-yl)cyclopropane carboxamide (3.0 g, 5.4 mmol) was dissolved in methanol (52 mL). Water (5.2 mL) was added followed by p-TsOH.H₂O (p-toluenesulfonic acid hydrate) (204 mg, 1.1 mmol). The reaction was heated at 80 °C for 45 minutes. The solution was concentrated and then partitioned between ethyl acetate and saturated NaHCO₃ solution. The ethyl acetate layer was dried over MgSO₄ and concentrated. The residue was purified by column chromatography (50-100 % ethyl acetate - hexanes) to yield the product. (1.3 g, 47 %, ee >98% by SFC). ESI-MS *m*/*z* calc. 520.5, found 521.7 (M+1)⁺. Retention time 1.69 minutes. ¹H NMR (400 MHz, DMSO-d₆) δ 8.31 (s, 1H), 7.53 (s, 1H), 7.42 - 7.38 (m, 2H), 7.33 - 7.30 (m, 2H), 6.22 (s, 1H), 5.01 (d, *J =* 5.2 Hz, 1H), 4.90 (t, *J =* 5.5 Hz, 1H), 4.75 (t, *J =* 5.8 Hz, 1H), 4.40 (dd, *J* = 2.6, 15.1 Hz, 1H), 4.10 (dd, *J* = 8.7, 15.1 Hz, 1H), 3.90 (s, 1H), 3.65 - 3.54 (m, 2H), 3.48 - 3.33 (m, 2H), 1.48 - 1.45 (m, 2H), 1.35 (s, 3H), 1.32 (s, 3H) and 1.14 - 1.11 (m, 2H) ppm.

### Example 3: Preparation of a Solid Dispersion of Amorphous Tezacaftor

A solvent system of DCM and MeOH was formulated according to the ratio 80 wt% DCM / 20 wt% MeOH, in an appropriately sized container, equipped with a magnetic stirrer and stir plate. Into this solvent system, hypromellose polymer (HPMC, E15 grade) and tezacaftor were added according to the ratio 20 wt% hypromellose / 80 wt% tezacaftor. The resulting mixture contained 12.5 wt% solids. The actual amounts of ingredients and solvents used to generate this mixture are recited in the table below:

### Solid spray dispersion ingredients for amorphous tezacaftor:

| | Units | Batch |
|---|---|---|
| Tezacaftor | g | 2400 |
| HPMC | g | 600 |
| **Total Solids** | **g** | **3000** |
| DCM | g | 16800 |
| MeOH | g | 4200 |
| **Total Solvents** | **g** | **21000** |
| **Total Spray Solution Weight** | **g** | **24000** |

The mixture was mixed until it was substantially homogenous and all components were substantially dissolved.

A spray drier, Anhydro MS-35 Spray Drier, fitted with two fluid 0.8 mm nozzle (Schlick series 970/0 S4), was used under normal spray drying mode, following the dry spray process parameters recited in the table below.

### Processing parameters to generate solid spray dispersion of amorphous tezacaftor:

| **Parameter** | **Value** |
|---|---|
| Process Gas Flow Rate | 34 kg/hr |
| Nozzle Gas Flow Rate | 4.2 kg/hr |
| Feed Flow Rate | 2 kg/hr |
| Inlet Temperature | 96-108 °C |
| Outlet Temperature | 40 °C |
| Vacuum Dryer Temperature | 45 °C |
| Vacuum Drying Time | 24-72 hours |

A high efficiency cyclone separated the wet product from the spray gas and solvent vapors. The wet product was transferred into trays and placed in vacuum dryer for drying to reduce residual solvents to a level of less than about 3000 ppm for MeOH and less than 600 ppm of DCM and to generate dry dispersion of amorphous tezacaftor, containing <0.02% MeOH and <0.06% DCM.

### Example 4: Synthesis of N-(2-(tert-butyl)-5-hydroxy-4-(2-(methyl-d₃)propan-2-yl-1,1,1,3,3,3-d₆)phenyl)-4-oxo-1,4-dihydroquinoline-3-carboxamide (Deutivacaftor)

The synthesis of deutivacaftor is described in PCT Publication No. WO 2019/109021 and U.S. Patent 9,512,079.

### Synthesis of deutivacaftor

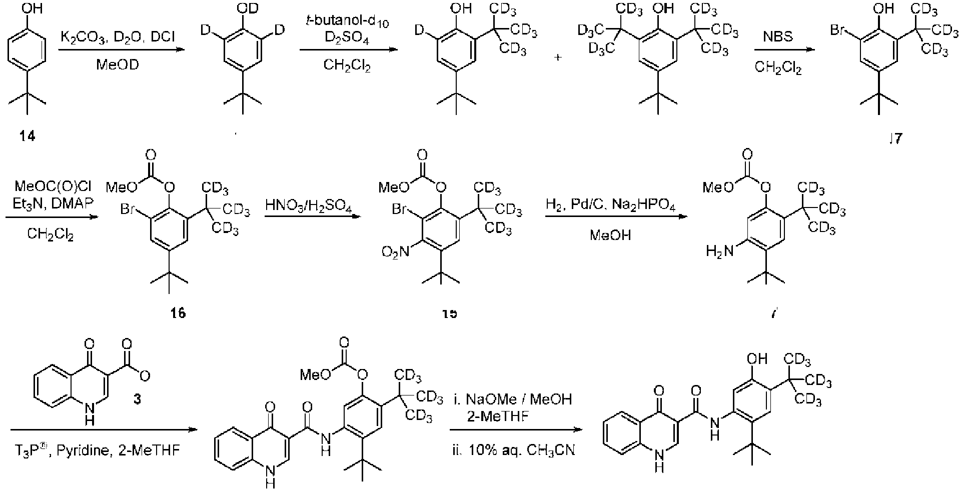

### Procedure for the synthesis of 4-(tert-butyl)phen-2,6-d₂-ol-d

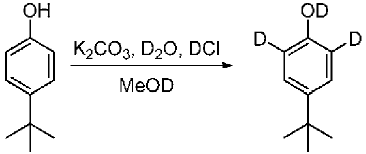

To a 5 L round bottom flask equipped with overhead stirrer was charged 4-*tert*butylphenol (14, 503.2 g), K₂CO₃ (46.3 g), D₂O (1949 g, 1761 mL, 3.5 vol), and MeOD (409 g, 503 mL, 1.0 vol). The mixture was heated to reflux under a nitrogen atmosphere. The reaction mixture was aged at reflux for 16 hours. The reaction mixture was cooled to room temperature and sampled for conversion (%D incorporation). The reaction was cooled to 5 °C and 35% DCI solution (90 g, 71.2 mL) was added. The reaction mixture was aged at 5 °C for 2 hours. The resultant slurry was filtered and the cake washed with D₂O (836 g, 755 mL, 1.5 vol) This process was repeated until the target %D incorporation is met (normally two exchanges required). The wet cake is dried in a vacuum oven with a nitrogen bleed at 40 °C until a constant weight is obtained. Yield of 4-(*tert*-butyl)phen-2,6-*d*₂-ol-d (19) is 506 g of a white solid (98%) with a purity of 99.6% and %D incorporation of 99 3%

### Procedure for the synthesis of 4-(tert-butyl)-2-(2-(methyl-d₃)propan-2-yl-1,1,1,3,3,3-d₆)phen-6-d-ol-d and 4-(tert-butyl)-2,6-bis(2-(methyl-d₃)propan-2-yl-1,1,1,3,3,3-d₆)phenol-d

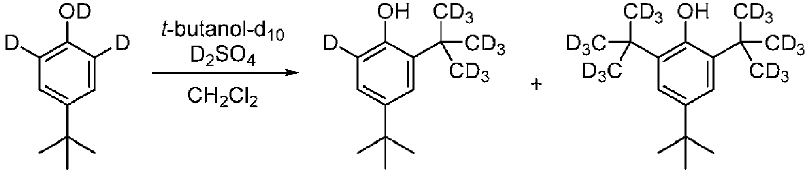

4-(*tert*-butyl)phen-2,6-*d*₂-ol-d (101.8 g, 0.66 mol, 1.0 equiv.) was dissolved in DCM (400 mL) in a 2 L reactor. *i*-Butanol-d₁₀ (43.0 g, 0.51 mol, 0.77 equiv.) was dissolved in DCM (100 mL) in a 250 mL flask. The solution of *tert*-butanol-d₆ was charged to the 2 L reactor at room temperature The reaction mixture was cooled to -5 °C. D₂SO₄ (51.1 g, 0.51 mol, 0.77 equiv.) was charged dropwise via an addition funnel while maintaining a temperature range of -4 to -2 °C. The reaction mixture was stirred at -2 °C for 3-4 hours. Upon complete conversion the reaction was quenched by adding water (28 mL) and warmed to 18-20 °C. The bottom aqueous layer was drained and discarded. The DCM layer was treated with sat. aq. NaHCO₃ solution (approximately 200 mL), adjusting the pH to 6-8. NaCl (sat.) solution (400 mL) was charged to the mixture. The resulting solution was stirred for 5 min, and settled for 5 min. The lower DCM layer was drained into a 1 L flask. The aqueous layer was discarded. The DCM solution was concentrated to minimal volume and n-heptane (200 mL) was charged. The solution was concentrated to minimal volume and n-heptane charged to a final volume of 800 mL. 0.5 N NaOH solution 600 mL (6 vol) was charged to the reactor and the resulting mixture was stirred for 5 min, and settled for at least 5 min. The aqueous layer was drained and discarded. 10 N NaOH solution 300 mL (3 vol) was charged to the reactor and the resulting mixture was stirred for 5 min, and settled for at least 5 min. The aqueous layer was drained and discarded. 1.0 N NaOH solution 300 mL (3 vol) was charged to the reactor and the resulting mixture was stirred for 5 min, and settled for at least 5 min. The aqueous layer was drained and discarded. The remaining n-heptane solution was concentrated to dryness to afford the desired product, 4-(*tert-*butyl)-2-(2-(methyl-*d*₃)propan-2-yl-1,1,1,3,3,3-*d*₆)phen-6-d-ol-d (18) as a clear oil, 104.5 g, which was carried forward into the next step without further purification.

### Procedure for the synthesis of 2-bromo-4-(tert-butyl)-6-(2-(methyl-d₃)propan-2-yl-1,1,1,3,3,3-d₆)phenol

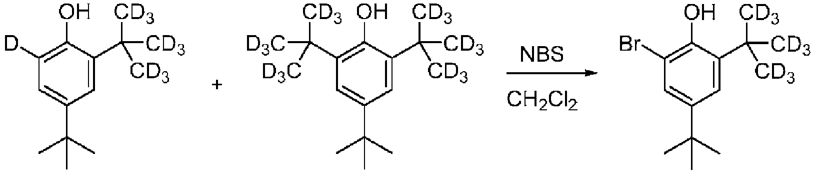

4-(*tert*-butyl)-2-(2-(methyl-d₃)propan-2-yl-1,1,1,3,3,3-*d*₆)phen-6-d-ol-d (100 g, 0.462 mol, 1.0 equiv.) was dissolved in DCM (800 mL, 7 vol) in a 2 L reactor and the solution was stirred. The batch was cooled down to 0 ± 3 °C. To the batch was charged portion-wise *N-*bromosuccinimide (84.4 g, 0.462 mol, 1.0 equiv) over 30 min. The batch was stirred at 0 ± 2 °C for at least 30 minutes The batch was then heated to 20 ± 2 °C over a period of 2 hours and stirred at 20 ± 2 °C for at least 12 hours. Upon complete conversion, sat. aq. NaHCO₃ solution (500 mL, 5 vol) was charged and the batch stirred for at least 10 minutes. The agitation was stopped to allow the phases to separate for at least 5 minutes and the DCM layer was drained, followed by removal of the aqueous layer. The DCM layer was charged back to the vessel. To the batch was charged sat. aq. NaHCO₃ bicarbonate solution (500 mL, 5 vol), and the batch was stirred for at least 10 minutes. The agitation was stopped to allow the phases to separate for at least 5 minutes and the DCM layer was drained, followed by removal of the aqueous layer The DCM layer was charged back to the vessel and diluted with an additional DCM (300 mL, 3 vol). The batch was distilled (removal of 300 mL) and checked by KF to achieve dryness The resulting clear yellow solution of 17 was carried forward into the next step without further purification.

### Procedure for the synthesis of 2-bromo-4-(tert-butyl)-6-(2-(methyl-d₃)propan-2-yi-1,1,1,3,3,3-d₆)phenyl methyl carbonate

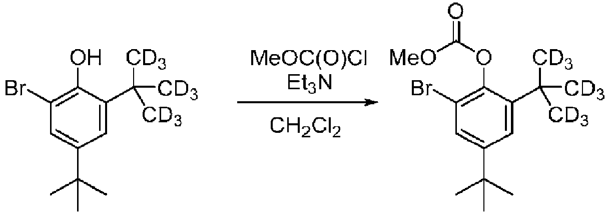

To a clean reactor was charged the DCM solution of 4-(*tert*-butyl)-2-(2-(methyl-d₃)propan-2-yl-1,1,1,3,3,3-d₆)phen-6-d-ol-d (136 g, 0.462 mol, 1.0 equiv.) followed by additional DCM (130 mL, 1 vol), and this solution was stirred. To the batch was charged 4-(dimethylamino)pyridine (2.8 g, 0.023 mol, 0.05 equiv) and triethylamine (70.1 g, 0.693 mol, 1.5 equiv). The batch was cooled to 0 ± 3 °C. To the batch was charged drop-wise methyl chloroformate (48.0 g, 0.508 mol, 1.1 equiv) over 40 minutes while maintaining a batch temperature < 5 °C. The batch was stirred at 3 ± 2 °C for at least 30 minutes, and then warmed to 20 ± 2 °C over a period of 1 hour. Upon complete conversion, 1 N HCl (400 mL, 3 vol) was charged. The batch was stirred for at least 10 minutes, and then the layers were allowed to separate for at least 5 minutes. The lower organic layer was drained followed by the aqueous layer (1^{st} aqueous layer). The organic layer was charged back to the reactor, along with 1 N HCl solution (400 mL, 3 vol). The batch was stirred for at least 10 minutes, and then the layers were allowed to separate for at least 5 minutes. The lower organic layer was drained. The first aqueous layer was charged to the reactor, along with DCM (300 mL, 2.2 vol) The batch was stirred for at least 10 minutes, and then the layers were allowed to separate for at least 5 minutes. The lower organic layer was drained and combined with the 1^{st} organic layer, followed by removal of the aqueous layer. Charge the vessel with the contents of both organic layers. The reactor was charged with water (500 mL, 3 7 vol). The batch was stirred for at least 10 minutes, and then the layers were allowed to separate for at least 5 minutes. The lower organic layer was drained, followed by the aqueous layer. The organic layer was charged back to the reactor, along DCM (400 mL, 3 vol). The batch was distilled to remove 800 mL and checked by KF to ensure dryness. The resulting clear yellow solution of 16 was telescoped into the next step without further purification.

### Procedure for the synthesis of 2-bromo-4-(tert-butyl)-6-(2-(methyl-d₃)propan-2-yi-1,1,1,3,3,3-d₆)-3-nitrophenyl methyl carbonate

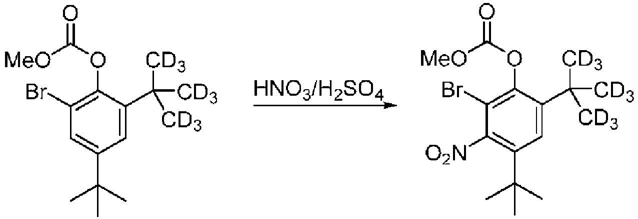

To a reactor was charged 2-bromo-4-(*tert*-butyl)-6-(2-(methyl-d₃)propan-2-yl-1,1,1,3,3,3-d₆)phenyl methyl carbonate and then the solution was cooled to 0 °C. Sulfuric acid (4.9 equiv) and nitric acid (100%, 2.0 equiv) was charged while maintaining a temperature of not more than 5 °C. The reaction was stirred at 0 °C for 2 hours until complete conversion. The reaction was then quenched with water (88 vol) and diluted with DCM (1.7 vol). The layers were separated and the upper aqueous layer was extracted with DCM (2.8 vol). After separating the layers, the organic layers were combined, returned to the reactor, and washed with sodium bicarbonate (7.4% w/w, 6.8 vol). After separating the layers, the organic layer was returned to the reactor and washed with sodium chloride (23% w/w, 3.8 vol) After separating the layers, the organic layer was returned to the reactor and concentrated to minimal volume. Methanol (1.2 vol) was charged, followed by concentration to minimal volume Methanol (1.2 vol) was charged, followed by concentration to minimal volume Methanol (1 7 vol) was charged, and the slurry was heated to reflux for 30 min and then cooled slowly over 4 hours to 5 °C. The solid product (15) was filtered and the cake washed with cold methanol (1.0 vol) The solid 2-bromo-4-(*tert*-butyl)-6-(2-(methyl-d₃)propan-2-yl-1,1,1,3,3,3-d₆)-3-nitrophenyl ethyl carbonate (15) was dried under vacuum at 40 - 50 °C to yield an off- white solid, 99.9% purity and 99% D incorporation.

### Procedure for the synthesis of 5-amino-4-(tert-butyl)-2-(2-(methyl-d₃)propan-2-yl-1,1,1,3,3,3-d₆)phenyl methyl carbonate

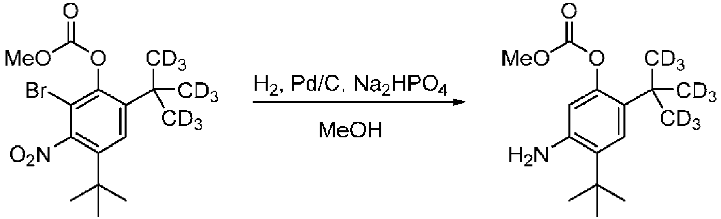

Charge 5 wt% (50 - 65 wt% wet, JM Type 37) of 5% Pd/C to a reactor Charge (4.0 vol) Methanol. Close the system. Purge with N_{2(g)} at 2.0 Bar. Activate with H_{2(g)} at 2.0 Bar. Charge the vessel to 2.0 Bar with H_{2 (g)} at 25 °C +/- 5 °C. Stir for not less than 2 hours while maintaining a temperature of 25 °C +/- 5 °C. Vent and purge with N2_{(g)} at 2.0 Bar. Charge compound 15 (1.0 eq) to a reactor, together with Na₂HPO₄ (2.3 eq). Charge (11.0 vol) Methanol. Close the system. Purge with N_{2(g)} at 2.0 Bar. Activate with H2_{(g)} at 2.0 Bar. Charge the vessel to 2.0 Bar with H_{2(g)} at 25 °C +/- 5 °C. Stir for about 24 hours while maintaining a reaction temperature of 25 °C +/- 5 °C Upon complete conversion, dilute reaction mixture by adding 7.7 vol of MeOH. Heat reaction mixture to 35.0 °C +/- 5 °C. Filter off catalyst and Na₂HPO₄. Wash the reactor and filter cake with Methanol (4.0 vol), and filter, combining with the initial filtrate Check Pd content and if needed perform resin treatment (resin treatment is: Charge SPM-32 resin (5 wt%). Stir the resin treated solution for not less than 3 hours at 35.0 °C +/- 5 °C. Filter off resin.)

Wash the reactor and filter cake with Methanol (2.0 vol), and filter, combining with the initial filtrate). Charge Norit CASP active carbon (3 wt%). Stir for not less than 3 hours at 35.0 °C +/- 5 °C Filter off active carbon. Wash the reactor and filter cake with methanol (2.0 vol), and filter, combining with the initial filtrate. Distill under vacuum at not more than 50 °C to 8.0 vol. Charge water (2.0 vol) while maintaining a temperature of 45 °C +/- 5 °C. Cool the resultant slurry to 0 °C +/- 5 °C over 2 hours. Hold and stir the slurry at 0 °C +/- 5 °C for not less than 1 hour. Filter and wash the cake with 2.0 volumes methanol / water (8.2) at 0 °C +/- 5 °C. Dry 5-amino-4-(*tert*-butyl)-2-(2-(methyl-d₃)propan-2-yl-1,1,1,3,3,3-d₆)phenyl methyl carbonate (7) under vacuum at not more than 40 °C to give a yield of a white solid, >99.5% purity.

### Procedure for the synthesis of 4-(tert-butyl)-2-(2-(methyl-d₃)propan-2-yl-1,1,1,3,3,3-d₆)-5-(4-oxo-1,4-dihydroquinoline-3-carboxamido)phenyl methyl carbonate

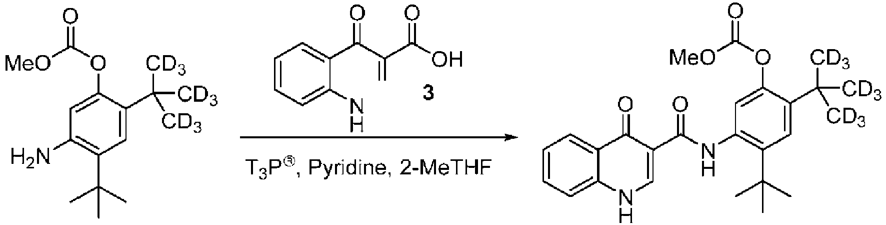

The procedure for the conversion of compound 7 into compound 8 may be performed according to the analogous procedure for compound 5 in Example 1 of PCT Publication No. WO 2019/109021.

### Procedure for the synthesis of N-(2-(tert-butyl)-5-hydroxy-4-(2-(methyl-d₃)propan-2- yl-1,1,1,3,3,3-d₆)phenyl)-4-oxo-1,4-dihydroquinoline-3-carboxamide (Deutivacaftor)

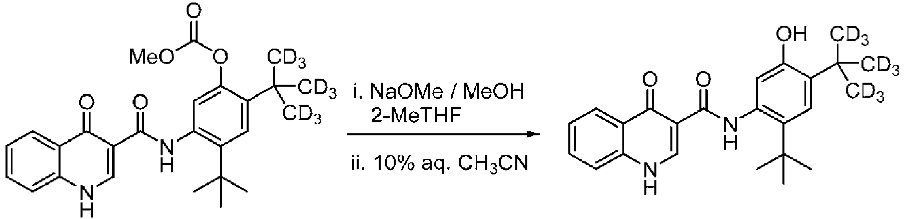

The procedure for the conversion of 4-(*tert*-butyl)-2-(2-(methyl-d₃)propan-2-yl-1,1,1,3,3,3-d₆)-5-(4-oxo-1,4-dihydroquinoline-3-carboxamido)phenyl methyl carbonate into deutivacaftor may be performed according to the analogous procedure for the synthesis of Compound 1 in Example 1 of PCT Publication No. WO 2019/109021.

### Example 5: Preparation of a Solid Dispersion of Deutivacaftor

Solid dispersions of a non-deuterated analog of deutivacaftor (ivacaftor) and methods of preparing such dispersions are disclosed in PCT Publication No. WO 2007/079139,. These same solid dispersions are suitable for use with deutivacaftor. Alternatively, a spray-dried dispersion of deutivacaftor may be prepared using the method described below.

A mixture of deutivacaftor, HPMCAS, and SLS (80 wt% deutivacaftor/19.5 wt% HPMCAS/0.5 wt% SLS) was dissolved in a methyl ethyl ketone/water mixture to give a total solids load of 11%. The resulting solution was spray dried using a Buchi spray drier under the following conditions:
Nozzle diameter: 1.52 mm
Outlet temperature:
   NOR: 50 - 60 °C
   DSL: 40 - 70 °C
Condenser temperature:
   NOR: -5.5 - 0 °C
   DSL: -5.5 - 5.5 °C

The resulting wet spray dried dispersion was further dried under the following conditions to give a deutivacaftor spray dried dispersion.
Jacket inlet temperature:
   NOR: ≤ 80 °C for 2 hours, then ≤ 110 °C for ≤ 4 days
   DSL: ≤ 80 °C for 2 hours, then ≤ 110 °C for ≤ 11 days or ≤ 120 °C for ≤ 1 day
Nitrogen flow rate (NOR: 2.5 - 8.0 m³/hr; Range: 2.5 - 8.0 m³/hr)
Vacuum pressure (NOR: ≤ -0.90 barg; Range: ≤ -0.80 barg)

### Example 6: Preparation of Exemplary Formulation 1

### Formulation 1

| **Component** | **Component Function** | **Content (% w/w)** | **Content (mg/unit dose)** |
|---|---|---|---|
| Crystalline vanzacaftor calcium salt hydrate Form D | Active Ingredient | 2.0 | 4.2 |
| Tezacaftor SDD (800 mg/g)^{a} | Active Ingredient | 9.5 | 20.0 |
| Deutivacaftor SDD (800 mg/g)^{b} | Active Ingredient | 29.8 | 62.5 |
| Mannitol, NF Mannogen EZ spray dried | Diluent/Flavor | 12.1 | 25.4 |
| Lactose Monohydrate, NF Fast Flo 316 | Diluent/Flavor | 36.3 | 76.3 |
| Sucralose Powder, NF Emprove | Sweetener | 2.3 | 4.8 |
| Croscarmellose Sodium (Ac-Di-Sol SD-711) | Disintegrant | 6.0 | 12.6 |
| Colloidal Silicon Dioxide, NF Cab-o-Sil | Flow Aid | 1.0 | 2.1 |
| Magnesium Stearate | Lubricant | 1.0 | 2.1 |
| Total | | **100.0** | **210.0** |

| | | | |
|---|---|---|---|
| ^{a} Tezacaftor SDD Composition: 80/20 wt% tezacaftor/HPMC ^{b} Deutivacaftor SDD Composition: 80/19.5/0.5 wt% deutivacaftor/HPMCAS/SLS | | | |

Vanzacaftor calcium salt hydrate Form D, tezacaftor SDD, deutivacaftor SDD, colloidal silicon dioxide, and sucralose were weighed, sieved, and blended. Separately, lactose monohydrate, mannitol, and croscarmellose were weighed, sieved, and blended. The two mixtures were then blended together. Magnesium stearate was weighed, sieved, and blended with the resulting mixture. The resulting blend was then compressed into granules, the granules were filled into HPMC capsules/sachets, and the filled capsules/sachets were packaged. The target weight of each granule was 7.0 mg with an average target weight of ten granules at 70.0 mg.

### Example 7: Preparation of Exemplary Formulation 2

| **Component** | **Component Function** | **Content (% w/w)** | **Content (mg/unit dose)** |
|---|---|---|---|
| Crystalline vanzacaftor calcium salt hydrate Form D | Active Ingredient | 2.0 | 8.5 |
| Tezacaftor SDD (800 mg/g)^{a} | Active Ingredient | 9.5 | 40.0 |
| Deutivacaftor SDD (800 mg/g)^{b} | Active Ingredient | 29.8 | 125.0 |
| Mannitol, NF Mannogen EZ spray dried | Diluent/Flavor | 12.1 | 50.8 |
| Lactose Monohydrate, NF Fast Flo 316 | Diluent/Flavor | 36.3 | 152.5 |
| Sucralose Powder, NF Emprove | Sweetener | 2.3 | 9.6 |
| Croscarmellose Sodium (Ac-Di-Sol SD-711) | Disintegrant | 6.0 | 25.2 |
| Colloidal Silicon Dioxide, NF Cab-o-Sil | Flow Aid | 1.0 | 4.2 |
| Magnesium Stearate | Lubricant | 1.0 | 4.2 |
| **Total** | | **100.0** | **420.0** |

| | | | |
|---|---|---|---|
| ^{c} Tezacaftor SDD Composition: 80/20 wt% tezacaftor/HPMC ^{d} Deutivacaftor SDD Composition: 80/19.5/0.5 wt% deutivacaftor/HPMCAS/SLS | | | |

The granules were prepared according to the procedure for Example 6.

### Example 8: Preparation of Exemplary Formulation 3

| **Component** | **Component Function** | **Content (% w/w)** | **Content (mg/unit dose)** |
|---|---|---|---|
| Crystalline vanzacaftor calcium salt hydrate Form D | Active Ingredient | 2.0 | 12.7 |
| Tezacaftor SDD (800 mg/g)^{a} | Active Ingredient | 9.5 | 60.0 |
| Deutivacaftor SDD (800 mg/g)^{b} | Active Ingredient | 29.8 | 187.5 |
| Mannitol, NF Mannogen EZ spray dried | Diluent/Flavor | 12.1 | 76.2 |
| Lactose Monohydrate, NF Fast Flo 316 | Diluent/Flavor | 36.3 | 228.8 |
| Sucralose Powder, NF Emprove | Sweetener | 2.3 | 14.4 |
| Croscarmellose Sodium (Ac-Di-Sol SD-711) | Disintegrant | 6.0 | 37.8 |
| Colloidal Silicon Dioxide, NF Cab-o-Sil | Flow Aid | 1.0 | 6.3 |
| Magnesium Stearate | Lubricant | 1.0 | 6.3 |
| **Total** | | **100.0** | **630.0** |

| | | | |
|---|---|---|---|
| ^{e} Tezacaftor SDD Composition: 80/20 wt% tezacaftor/HPMC ^{f} Deutivacaftor SDD Composition: 80/19.5/0.5 wt% deutivacaftor/HPMCAS/SLS | | | |

The granules were prepared according to the procedure for Example 6.

## Claims

1. A pharmaceutical composition, formulated into a unit dosage form comprising a plurality of granules, wherein each of the granules comprises:
a. crystalline vanzacaftor calcium salt hydrate Form D;
b. a solid dispersion comprising amorphous tezacaftor and a first polymer;
c. a solid dispersion comprising amorphous deutivacaftor and a second polymer;
d. one or more fillers;
e. a disintegrant;
f. a sweetener;
g. a glidant; and
h. a lubricant; and
wherein the unit dosage form comprises crystalline vanzacaftor calcium salt hydrate Form D in an amount ranging from 1 mg to 12.7 mg, amorphous tezacaftor in an amount ranging from 10 mg to 50 mg, and amorphous deutivacaftor in an amount ranging from 15 mg to 150 mg.

2. The pharmaceutical composition of claim 1, wherein the unit dosage form comprises 4.2 mg of crystalline vanzacaftor calcium salt hydrate Form D, 16 mg of amorphous tezacaftor, and 50 mg of amorphous deutivacaftor.

3. The pharmaceutical composition of claim 1, wherein the unit dosage form comprises:
(a) 6.4 mg of crystalline vanzacaftor calcium salt hydrate Form D, 24 mg of amorphous tezacaftor, and 75 mg of amorphous deutivacaftor;
(b) 8.5 mg of crystalline vanzacaftor calcium salt hydrate Form D, 32 mg of amorphous tezacaftor, and 100 mg of amorphous deutivacaftor; or
(c) 12.7 mg of crystalline vanzacaftor calcium salt hydrate Form D, 48 mg of amorphous tezacaftor, and 150 mg of amorphous deutivacaftor.

4. The pharmaceutical composition of any one of claims 1 to 3, wherein the first polymer in the solid dispersion comprising tezacaftor is HPMC; and/or
wherein the second polymer in the solid dispersion comprising deutivacaftor is HPMCAS.

5. The pharmaceutical composition of any one of claims 1 to 4, wherein the solid dispersion comprising tezacaftor and the first polymer comprises 70 to 90 percent of amorphous tezacaftor by weight of the dispersion, optionally 80 percent of amorphous tezacaftor by weight of the dispersion and 20 percent of HPMC by weight of the dispersion; and/or
wherein the solid dispersion comprising deutivacaftor and the second polymer comprises 70 to 90 percent of amorphous deutivacaftor, optionally 80 percent of amorphous deutivacaftor by weight of the dispersion, 19.5 percent of HPMCAS by weight of the dispersion, and 0.5 percent SLS by weight of the dispersion.

6. The pharmaceutical composition of any one of claims 1 to 5, wherein the one or more fillers comprise mannitol; optionally wherein the mannitol is present in an amount of 10 to 14 percent by weight of the composition.

7. The pharmaceutical composition of any one of claims 1 to 6, wherein the one or more fillers comprise lactose; optionally wherein the lactose is present in an amount of 35 to 40 percent by weight of the composition.

8. The pharmaceutical composition of any one of claims 1 to 7, wherein the filler is a binary filler comprising mannitol and lactose; optionally wherein the binary filler comprises:
(a) mannitol and lactose in a ratio by weight selected from: 1 to 5, 1 to 4, 1 to 3, 1 to 2, and 1 to 1.5; or
(b) mannitol and lactose in a ratio of 1 to 3 by weight.

9. The pharmaceutical composition of any one of claims 1 to 8, wherein the disintegrant is croscarmellose sodium; optionally wherein the croscarmellose sodium is present in an amount of from 1 to 8 percent by weight, optionally 6 percent by weight of the pharmaceutical composition.

10. The pharmaceutical composition of any one of claims 1 to 9, wherein the sweetener is sucralose; optionally wherein the sucralose is present in an amount of 1 to 2 percent by weight of the pharmaceutical composition.

11. The pharmaceutical composition of any one of claims 1 to 10, wherein the lubricant is magnesium stearate; optionally wherein the magnesium stearate is present in an amount of 0.5 to 2.0 percent by weight of the composition.

12. The pharmaceutical composition of any one of claims 1 to 11, wherein the glidant is colloidal silicon dioxide; optionally wherein the colloidal silicon dioxide is present in an amount of 0.5 to 1.5 percent by weight of the composition.

13. A pharmaceutical composition, formulated into a unit dosage form comprising a plurality of granules, wherein each of the granules comprises:
a. 1 to 3 percent crystalline vanzacaftor calcium salt hydrate Form D by weight of the composition,
b. 8 to 11 percent of a solid dispersion by weight of the composition, wherein the solid dispersion comprises 80 percent amorphous tezacaftor and 20 percent HPMC by weight of the solid dispersion,
c. 25 to 35 percent by of a solid dispersion by weight of the composition, wherein the solid dispersion comprises 80 percent amorphous deutivacaftor, 19.5 percent HPMCAS and 0.5 percent SLS by weight of the solid dispersion,
d. 10 to 14 percent mannitol by weight of the composition,
e. 30 to 40 percent lactose by weight of the composition,
f. 1.5 to 2.5 percent of sucralose by weight of the composition,
g. 5 to 7 percent croscarmellose sodium by weight of the composition,
h. 0.5 to 1.5 percent colloidal silicon dioxide by weight of the composition, and
i. 0.5 to 2.0 percent magnesium stearate by weight of the composition.

14. The pharmaceutical composition of claim 13, wherein the mannitol and lactose are present in a ratio of 1 to 3 by weight.

15. The pharmaceutical composition of claim 13 comprising:
a. 2 percent crystalline vanzacaftor calcium salt hydrate Form D by weight of the composition,
b. 9.5 percent of a solid dispersion by weight of the composition, wherein the solid dispersion comprises 80 percent amorphous tezacaftor and 20 percent HPMC by weight of the solid dispersion,
c. 29.8 percent by of a solid dispersion by weight of the composition, wherein the solid dispersion comprises 80 percent amorphous deutivacaftor, 19.5 percent HPMCAS and 0.5 percent SLS by weight of the solid dispersion,
d. 12.1 percent mannitol by weight of the composition,
e. 36.3 percent lactose by weight of the composition,
f. 2.3 percent of sucralose by weight of the composition,
g. 6 percent croscarmellose sodium by weight of the composition,
h. 1 percent colloidal silicon dioxide by weight of the composition, and
i. 1 percent magnesium stearate by weight of the composition.

16. The pharmaceutical composition of any one of claims 13 to 15, wherein the unit dosage form comprises 0.1 to 12.7 mg of crystalline vanzacaftor calcium salt hydrate Form D, 10 to 50 mg of amorphous tezacaftor, and 15 to 150 mg of amorphous deutivacaftor.

17. The pharmaceutical composition of any one of claims 13 to 16, wherein the unit dosage form comprises 4.2 mg of crystalline vanzacaftor calcium salt hydrate Form D, 16 mg of amorphous tezacaftor, and 50 mg of amorphous deutivacaftor.

18. The pharmaceutical composition of any one of claims 13 to 16, wherein the unit dosage form comprises:
(a) 6.4 mg of crystalline vanzacaftor calcium salt hydrate Form D, 24 mg of amorphous tezacaftor, and 75 mg of amorphous deutivacaftor;
(b) 8.5 mg of crystalline vanzacaftor calcium salt hydrate Form D, 32 mg of amorphous tezacaftor, and 100 mg of amorphous deutivacaftor; or
(c) 12.7 mg of crystalline vanzacaftor calcium salt hydrate Form D, 48 mg of amorphous tezacaftor, and 150 mg of amorphous deutivacaftor.

19. The pharmaceutical composition of any one of claims 1 to 18, wherein the unit dose form comprises:
(a) from 18 to 60 granules; or
(b) 30 granules.

20. The pharmaceutical composition of any one of claims 1 to 19, wherein the granule has a shape that is cylinder-like, oval-like, cone-like, sphere-like, ellipsis-like, polygon-like or combinations thereof, wherein the granule has as its longest dimension or diameter a length of 1.5 mm to 4.0 mm, optionally a length of 2 mm.

21. The pharmaceutical composition of any one of claims 1 to 20, for use in a method of treating or lessening the severity of CFTR-mediated disease in a pediatric patient, wherein the CFTR mediated disease is cystic fibrosis.

22. The pharmaceutical composition for use of claim 21, wherein the pharmaceutical composition is administered once daily.

23. The pharmaceutical composition for use of claim 21 or claim 22, wherein the patient weighs:
(a) 14 or more kilograms; or
(b) less than 14 kilograms.

24. The pharmaceutical composition for use of any one of claims 21 to 23, wherein the patient:
(a) has at least one mutation selected from 3141del9, E822K, G1069R, L967S, R117L, S912L, 546insCTA, F191V, G1244E, L997F, R117P, S945L, A46D, F311del, G1249R, L1077P, R170H, S977F, A120T, F311L, G1349D, L1324P, R258G, S1159F, A234D, F508C, H139R, L1335P, R334L, S1159P, A349V, F508C;S1251N, H199Y, L1480P, R334Q, S1251N, A455E, F508del*, H939R, M152V, R347H, S1255P, A554E, F575Y, H1054D, M265R, R347L, T338I, A1006E, F1016S, H1085P, M952I, R347P, T1036N, A1067T, F1052V, H1085R, M952T, R352Q, T1053I, D110E, F1074L, H1375P, M1101K, R352W, V201M, D110H, F1099L, I148T, P5L, R553Q, V232D, D192G, G27R, I175V, P67L, R668C, V456A, D443Y, G85E, I336K, P205S, R751L, V456F, D443Y;G576A;R668C, G126D, I502T, P574H, R792G, V562I, D579G, G178E, I601F, Q98R, R933G, V754M, D614G, G178R, I618T, Q237E, R1066H, V1153E, D836Y, G194R, I807M, Q237H, R1070Q, V1240G, D924N, G194V, I980K, Q359R, R1070W, V1293G, D979V, G314E, I1027T, Q1291R, R1162L, W361R, D1152H, G463V, I1139V, R31L, R1283M, W1098C, D1270N, G480C, I1269N, R74Q, R1283S, W1282R, E56K, G551D, I1366N, R74W, S13F, Y109N, E60K, G551S, K1060T, R74W;D1270N, S341P, Y161D, E92K, G576A, L15P, R74W;V201M, S364P, Y161S, E116K, G576A;R668C, L165S, R74W;V201M;D1270N, S492F, Y563N, E193K, G622D, L206W, R75Q, S549N, Y1014C, E403D, G628R, L320V, R117C, S549R, Y1032C, E474K, G970D, L346P, R117G, S589N, E588V, G1061R, L453S, R117H, S737F; wherein D443Y;G576A;R668C, F508C;S1251N, G576A;R668C, R74W;D1270N, R74W;V201M, and R74W;V201M;D1270N are complex or compound mutations where a single allele of the *CFTR* gene has multiple mutations, which exist independently of the presence of mutations on the other allele; or
(b) is homozygous for the F508del mutation of the *CFTR* gene; or
(c) has a heterozygous CFTR genotype and has one F508del mutation.

## Patentansprüche

1. Pharmazeutische Zusammensetzung, formuliert zu einer Einheitsdosierungsform, die eine Vielzahl von Granulatkörnern umfasst, wobei jedes der Granulatkörner umfasst:
a. kristallines Vanzacaftor-Calciumsalz-Hydrat Form D;
b. eine feste Dispersion, die amorphes Tezacaftor und ein erstes Polymer umfasst;
c. eine feste Dispersion, die amorphes Deutivacaftor und ein zweites Polymer umfasst;
d. einen oder mehrere Füllstoffe;
e. ein Sprengmittel;
f. ein Süßungsmittel;
g. ein Gleitmittel; und
h. ein Schmiermittel; und
wobei die Einheitsdosierungsform kristallines Vanzacaftor-Calciumsalz-Hydrat Form D in einer Menge in dem Bereich von 1 mg bis 12,7 mg, amorphes Tezacaftor in einer Menge in dem Bereich von 10 mg bis 50 mg und amorphes Deutivacaftor in einer Menge in dem Bereich von 15 mg bis 150 mg umfasst.

2. Pharmazeutische Zusammensetzung nach Anspruch 1, wobei die Einheitsdosierungsform 4,2 mg kristallines Vanzacaftor-Calciumsalz-Hydrat Form D, 16 mg amorphes Tezacaftor und 50 mg amorphes Deutivacaftor umfasst.

3. Pharmazeutische Zusammensetzung nach Anspruch 1, wobei die Einheitsdosierungsform umfasst:
(a) 6,4 mg kristallines Vanzacaftor-Calciumsalz-Hydrat Form D, 24 mg amorphes Tezacaftor und 75 mg amorphes Deutivacaftor;
(b) 8,5 mg kristallines Vanzacaftor-Calciumsalz-Hydrat Form D, 32 mg amorphes Tezacaftor und 100 mg amorphes Deutivacaftor; oder
(c) 12,7 mg kristallines Vanzacaftor-Calciumsalz-Hydrat Form D, 48 mg amorphes Tezacaftor und 150 mg amorphes Deutivacaftor.

4. Pharmazeutische Zusammensetzung nach einem der Ansprüche 1 bis 3, wobei das erste Polymer in der festen Dispersion, die Tezacaftor umfasst, HPMC ist; und/oder wobei das zweite Polymer in der festen Dispersion, die Deutivacaftor umfasst, HPMCAS ist.

5. Pharmazeutische Zusammensetzung nach einem der Ansprüche 1 bis 4, wobei die feste Dispersion, die Tezacaftor und das erste Polymer umfasst, 70 bis 90 Gew.-% an amorphem Tezacaftor, bezogen auf das Gewicht der Dispersion, gegebenenfalls 80 Gew.-% an amorphem Tezacaftor, bezogen auf das Gewicht der Dispersion, und 20 Gew.-% HPMC, bezogen auf das Gewicht der Dispersion, umfasst; und/oder
wobei die feste Dispersion, die Deutivacaftor und das zweite Polymer umfasst, 70 bis 90 Prozent an amorphem Deutivacaftor, gegebenenfalls 80 Prozent an amorphem Deutivacaftor, bezogen auf das Gewicht der Dispersion, 19,5 Prozent HPMCAS, bezogen auf das Gewicht der Dispersion, und 0,5 Prozent SLS, bezogen auf das Gewicht der Dispersion, umfasst.

6. Pharmazeutische Zusammensetzung nach einem der Ansprüche 1 bis 5, wobei der eine oder die mehreren Füllstoffe Mannit umfassen; wobei das Mannit gegebenenfalls in einer Menge von 10 bis 14 Gew.-% der Zusammensetzung vorhanden ist.

7. Pharmazeutische Zusammensetzung nach einem der Ansprüche 1 bis 6, wobei der eine oder die mehreren Füllstoffe Lactose umfassen; gegebenenfalls wobei die Lactose in einer Menge von 35 bis 40 Gew.-% der Zusammensetzung vorhanden ist.

8. Pharmazeutische Zusammensetzung nach einem der Ansprüche 1 bis 7, wobei der Füllstoff ein binärer Füllstoff ist, der Mannit und Lactose umfasst; wobei der binäre Füllstoff gegebenenfalls umfasst:
(a) Mannit und Lactose in einem Gewichtsverhältnis ausgewählt aus: 1 zu 5, 1 zu 4, 1 zu 3, 1 zu 2 und 1 zu 1,5; oder
b) Mannit und Lactose in einem Gewichtsverhältnis von 1 zu 3.

9. Pharmazeutische Zusammensetzung nach einem der Ansprüche 1 bis 8, wobei das Sprengmittel Croscarmellose-Natrium ist; wobei das Croscarmellose-Natrium gegebenenfalls in einer Menge von 1 bis 8 Gew.-%, gegebenenfalls 6 Gew.-%, der pharmazeutischen Zusammensetzung vorhanden ist.

10. Pharmazeutische Zusammensetzung nach einem der Ansprüche 1 bis 9, wobei das Süßungsmittel Sucralose ist; wobei die Sucralose gegebenenfalls in einer Menge von 1 bis 2 Gew.-% der pharmazeutischen Zusammensetzung vorhanden ist.

11. Pharmazeutische Zusammensetzung nach einem der Ansprüche 1 bis 10, wobei das Gleitmittel Magnesiumstearat ist; wobei das Magnesiumstearat gegebenenfalls in einer Menge von 0,5 bis 2,0 Gew.-% der Zusammensetzung vorhanden ist.

12. Pharmazeutische Zusammensetzung nach einem der Ansprüche 1 bis 11, wobei das Gleitmittel kolloidales Siliciumdioxid ist; wobei das kolloidale Siliciumdioxid gegebenenfalls in einer Menge von 0,5 bis 1,5 Gew.-% der Zusammensetzung vorhanden ist.

13. Pharmazeutische Zusammensetzung, formuliert zu einer Einheitsdosierungsform, die eine Vielzahl von Granulatkörnern umfasst, wobei jedes der Granulatkörner umfasst:
a. 1 bis 3 Gew.-% kristallines Vanzacaftor-Calciumsalz-Hydrat Form D, bezogen auf das Gewicht der Zusammensetzung,
b. 8 bis 11 Gew.-% an einer festen Dispersion, bezogen auf das Gewicht der Zusammensetzung, wobei die feste Dispersion 80 Gew.-% an amorphem Tezacaftor und 20 Gew.- % HPMC, bezogen auf das Gewicht der festen Dispersion, umfasst,
c. 25 bis 35 Gew.-% an einer festen Dispersion, bezogen auf das Gewicht der Zusammensetzung, wobei die feste Dispersion 80 Gew.-% an amorphem Deutivacaftor, 19,5 Gew.-% HPMCAS und 0,5 Gew.-% SLS, bezogen auf das Gewicht der festen Dispersion, umfasst,
d. 10 bis 14 Gew.-% Mannit, bezogen auf das Gewicht der Zusammensetzung,
e. 30 bis 40 Gew.-% Lactose, bezogen auf das Gewicht der Zusammensetzung,
f. 1,5 bis 2,5 Gew.-% Sucralose, bezogen auf das Gewicht der Zusammensetzung,
g. 5 bis 7 Gew.-% Croscarmellose-Natrium, bezogen auf das Gewicht der Zusammensetzung,
h. 0,5 bis 1,5 Gew.-% kolloidales Siliciumdioxid, bezogen auf das Gewicht der Zusammensetzung, und
i. 0,5 bis 2,0 Gew.-% Magnesiumstearat, bezogen auf das Gewicht der Zusammensetzung.

14. Pharmazeutische Zusammensetzung nach Anspruch 13, wobei das Mannit und die Lactose in einem Gewichtsverhältnis von 1 zu 3 vorliegen.

15. Pharmazeutische Zusammensetzung nach Anspruch 13, umfassend:
a. 2 Prozent an kristallinem Vanzacaftor-Calciumsalz-Hydrat Form D, bezogen auf das Gewicht der Zusammensetzung,
b. 9,5 Gew.-% an einer festen Dispersion, bezogen auf das Gewicht der Zusammensetzung, wobei die feste Dispersion 80 Gew.-% an amorphem Tezacaftor und 20 Gew.- % HPMC, bezogen auf das Gewicht der festen Dispersion, umfasst,
c. 29,8 Gew.-% an einer festen Dispersion, bezogen auf das Gewicht der Zusammensetzung, wobei die feste Dispersion 80 Gew.-% an amorphem Deutivacaftor, 19,5 Gew.-% HPMCAS und 0,5 Gew.-% SLS, bezogen auf das Gewicht der festen Dispersion, umfasst,
d. 12,1 Gew.-% Mannit, bezogen auf das Gewicht der Zusammensetzung,
e. 36,3 Gew.-% Lactose, bezogen auf das Gewicht der Zusammensetzung,
f. 2,3 Gew.-% Sucralose, bezogen auf das Gewicht der Zusammensetzung,
g. 6 Gew.-% Croscarmellose-Natrium, bezogen auf das Gewicht der Zusammensetzung,
h. 1 Gew.-% an kolloidalem Siliciumdioxid, bezogen auf das Gewicht der Zusammensetzung, und
i. 1 Gew.-% Magnesiumstearat, bezogen auf das Gewicht der Zusammensetzung.

16. Pharmazeutische Zusammensetzung nach einem der Ansprüche 13 bis 15, wobei die Einheitsdosierungsform 0,1 bis 12,7 mg kristallines Vanzacaftor-Calciumsalz-Hydrat Form D, 10 bis 50 mg amorphes Tezacaftor und 15 bis 150 mg amorphes Deutivacaftor umfasst.

17. Pharmazeutische Zusammensetzung nach einem der Ansprüche 13 bis 16, wobei die Einheitsdosierungsform 4,2 mg kristallines Vanzacaftor-Calciumsalz-Hydrat Form D, 16 mg amorphes Tezacaftor und 50 mg amorphes Deutivacaftor umfasst.

18. Pharmazeutische Zusammensetzung nach einem der Ansprüche 13 bis 16, wobei die Einheitsdosierungsform umfasst:
(a) 6,4 mg kristallines Vanzacaftor-Calciumsalz-Hydrat Form D, 24 mg amorphes Tezacaftor und 75 mg amorphes Deutivacaftor;
(b) 8,5 mg kristallines Vanzacaftor-Calciumsalz-Hydrat Form D, 32 mg amorphes Tezacaftor und 100 mg amorphes Deutivacaftor; oder
(c) 12,7 mg kristallines Vanzacaftor-Calciumsalz-Hydrat Form D, 48 mg amorphes Tezacaftor und 150 mg amorphes Deutivacaftor.

19. Pharmazeutische Zusammensetzung nach einem der Ansprüche 1 bis 18, wobei die Einheitsdosisform umfasst:
(a) von 18 bis 60 Granulatkörner; oder
(b) 30 Granulatkörner.

20. Pharmazeutische Zusammensetzung nach einem der Ansprüche 1 bis 19, wobei das Granulatkorn eine Form aufweist, die zylinderartig, oval, konusartig, kugelartig, ellipsenartig, polygonal oder Kombinationen davon ist, wobei das Granulatkorn als seine längste Abmessung oder seinen Durchmesser eine Länge von 1,5 mm bis 4,0 mm, gegebenenfalls eine Länge von 2 mm, aufweist.

21. Pharmazeutische Zusammensetzung nach einem der Ansprüche 1 bis 20 zur Verwendung bei einem Verfahren zur Behandlung oder Verringerung der Schwere einer CFTRvermittelten Erkrankung bei einem pädiatrischen Patienten, wobei die CFTR-vermittelte Erkrankung zystische Fibrose ist.

22. Pharmazeutische Zusammensetzung zur Verwendung nach Anspruch 21, wobei die pharmazeutische Zusammensetzung einmal täglich verabreicht wird.

23. Pharmazeutische Zusammensetzung zur Verwendung nach Anspruch 21 oder Anspruch 22, wobei der Patient wiegt:
(a) 14 oder mehr Kilogramm; oder
(b) weniger als 14 Kilogramm.

24. Pharmazeutische Zusammensetzung zur Verwendung nach einem der Ansprüche 21 bis 23, wobei der Patient:
(a) wenigstens eine Mutation ausgewählt aus 3141del9, E822K, G1069R, L967S, R117L, S912L, 546insCTA, F191V, G1244E, L997F, R117P, S945L, A46D, F311del, G1249R, L1077P, R170H, S977F, A120T, F311L, G1349D, L1324P, R258G, S1159F, A234D, F508C, H139R, L1335P, R334L, S1159P, A349V, F508C;S1251N, H199Y, L1480P, R334Q, S1251N, A455E, F508del*, H939R, M152V, R347H, S1255P, A554E, F575Y, H1054D, M265R, R347L, T338I, A1006E, F1016S, H1085P, M952I, R347P, T1036N, A1067T, F1052V, H1085R, M952T, R352Q, T1053I, D110E, F1074L, H1375P, M1101K, R352W, V201M, D110H, F1099L, I148T, P5L, R553Q, V232D, D192G, G27R, I175V, P67L, R668C, V456A, D443Y, G85E, I336K, P205S, R751L, V456F, D443Y;G576A;R668C, G126D, I502T, P574H, R792G, V562I, D579G, G178E, I601F, Q98R, R933G, V754M, D614G, G178R, I618T, Q237E, R1066H, V1153E, D836Y, G194R, I807M, Q237H, R1070Q, V1240G, D924N, G194V, I980K, Q359R, R1070W, V1293G, D979V, G314E, I1027T, Q1291R, R1162L, W361R, D1152H, G463V, I1139V, R31L, R1283M, W1098C, D1270N, G480C, I1269N, R74Q, R1283S, W1282R, E56K, G551D, I1366N, R74W, S13F, Y109N, E60K, G551S, K1060T, R74W;D1270N, S341P, Y161D, E92K, G576A, L15P, R74W;V201M, S364P, Y161S, E116K, G576A;R668C, L165S, R74W;V201M;D1270N, S492F, Y563N, E193K, G622D, L206W, R75Q, S549N, Y1014C, E403D, G628R, L320V, R117C, S549R, Y1032C, E474K, G970D, L346P, R117G, S589N, E588V, G1061R, L453S, R117H, S737F aufweist; wobei D443Y;G576A;R668C, F508C;S1251N, G576A;R668C, R74W;D1270N, R74W;V201M und R74W;V201M;D1270N komplexe oder verbundene Mutationen sind, bei denen ein einzelnes Allel des CFTR-Gens mehrere Mutationen aufweist, die unabhängig von dem Vorhandensein von Mutationen auf dem anderen Allel vorliegen; oder
(b) für die F508del-Mutation des CFTR-Gens homozygot ist; oder
(c) einen heterozygoten CFTR-Genotyp aufweist und eine F508del-Mutation aufweist.

## Revendications

1. Composition pharmaceutique, formulée dans une forme posologique unitaire comprenant une pluralité de granules, chacun des granules comprenant :
a. la forme D de sel de calcium de vanzacaftor cristallin hydraté ;
b. une dispersion solide comprenant du tézacaftor amorphe et un premier polymère ;
c. une dispersion solide comprenant du deutivacaftor amorphe et un deuxième polymère ;
d. une ou plusieurs charges ;
e. un agent désintégrant ;
f. un édulcorant ;
g. un agent glissant ; et
h. un lubrifiant ; et
la forme posologique unitaire comprenant la forme D de sel de calcium de vanzacaftor cristallin hydraté en une quantité comprise entre 1 mg et 12,7 mg, du tézacaftor amorphe en une quantité comprise entre 10 mg et 50 mg et du deutivacaftor amorphe en une quantité comprise entre 15 mg et 150 mg.

2. Composition pharmaceutique selon la revendication 1, la forme posologique unitaire comprenant 4,2 mg de forme D de sel de calcium de vanzacaftor cristallin hydraté, 16 mg de tézacaftor amorphe et 50 mg de deutivacaftor amorphe.

3. Composition pharmaceutique selon la revendication 1, la forme posologique unitaire comprenant :
(a) 6,4 mg de forme D de sel de calcium de vanzacaftor cristallin hydraté, 24 mg de tézacaftor amorphe et 75 mg de deutivacaftor amorphe ;
(b) 8,5 mg de forme D de sel de calcium de vanzacaftor cristallin hydraté, 32 mg de tézacaftor amorphe et 100 mg de deutivacaftor amorphe ; ou
(a) 12,7 mg de forme D de sel de calcium de vanzacaftor cristallin hydraté, 48 mg de tézacaftor amorphe et 150 mg de deutivacaftor amorphe.

4. Composition pharmaceutique selon l'une quelconque des revendications 1 à 3, dans laquelle le premier polymère dans la dispersion solide comprenant du tézacaftor est le HPMC ; et/ou
dans laquelle le deuxième polymère dans la dispersion solide comprenant du deutivacaftor est le HPMCAS.

5. Composition pharmaceutique selon l'une quelconque des revendications 1 à 4, dans laquelle la dispersion solide comprenant du tézacaftor et le premier polymère comprend 70 à 90 pour cent de tézacaftor amorphe en poids de la dispersion, facultativement 80 pour cent de tézacaftor amorphe en poids de la dispersion et 20 pour cent de HPMC en poids de la dispersion ; et/ou
dans laquelle la dispersion solide comprenant du deutivacaftor et le deuxième polymère comprend 70 à 90 pour cent de deutivacaftor amorphe, facultativement 80 pour cent de deutivacaftor amorphe en poids de la dispersion, 19,5 pour cent de HPMCAS en poids de la dispersion et 0,5 pour cent de SLS en poids de la dispersion.

6. Composition pharmaceutique selon l'une quelconque des revendications 1 à 5, dans laquelle la ou les charges comprennent du mannitol ; facultativement, le mannitol étant présent en une quantité de 10 à 14 pour cent en poids de la composition.

7. Composition pharmaceutique selon l'une quelconque des revendications 1 à 6, dans laquelle la ou les charges comprennent du lactose ; facultativement, le lactose étant présent en une quantité de 35 à 40 pour cent en poids de la composition.

8. Composition pharmaceutique selon l'une quelconque des revendications 1 à 7, dans laquelle la charge est une charge binaire comprenant du mannitol et du lactose ; facultativement, la charge binaire comprenant :
(a) du mannitol et du lactose dans un rapport pondéral choisi parmi : 1 à 5, 1 à 4, 1 à 3, 1 à 2, et 1 à 1,5 ; ou
(b) du mannitol et du lactose dans un rapport pondéral de 1 à 3.

9. Composition pharmaceutique selon l'une quelconque des revendications 1 à 8, dans laquelle l'agent désintégrant est la croscarmellose sodique ; facultativement, la croscarmellose sodique étant présente en une quantité de 1 à 8 pour cent en poids, facultativement 6 pour cent en poids de la composition pharmaceutique.

10. Composition pharmaceutique selon l'une quelconque des revendications 1 à 9, dans laquelle l'édulcorant est le sucralose ; facultativement, le sucralose étant présent en une quantité de 1 à 2 pour cent en poids de la composition pharmaceutique.

11. Composition pharmaceutique selon l'une quelconque des revendications 1 à 10, dans laquelle le lubrifiant est le stéarate de magnésium ; facultativement, le stéarate de magnésium étant présent en une quantité de 0,5 à 2,0 pour cent en poids de la composition.

12. Composition pharmaceutique selon l'une quelconque des revendications 1 à 11, dans laquelle l'agent glissant est le dioxyde de silicium colloïdal ; facultativement, le dioxyde de silicium colloïdal étant présent en une quantité de 0,5 à 1,5 pour cent en poids de la composition.

13. Composition pharmaceutique, formulée dans une forme posologique unitaire comprenant une pluralité de granules, chacun des granules comprenant :
a. 1 à 3 pour cent de forme D de sel de calcium de vanzacaftor cristallin hydraté en poids de la composition,
b. 8 à 11 pour cent d'une dispersion solide en poids de la composition, la dispersion solide comprenant 80 pour cent de tézacaftor amorphe et 20 pour cent de HPMC en poids de la dispersion solide,
c. 25 à 35 pour cent d'une dispersion solide en poids de la composition, la dispersion solide comprenant 80 pour cent de deutivacaftor amorphe, 19,5 pour cent de HPMCAS et 0,5 pour cent de SLS en poids de la dispersion solide,
d. 10 à 14 pour cent de mannitol en poids de la composition,
e. 30 à 40 pour cent de lactose en poids de la composition,
f. 1,5 à 2,5 pour cent de sucralose en poids de la composition,
g. 5 à 7 pour cent de croscarmellose sodique en poids de la composition,
h. 0,5 à 1,5 pour cent de dioxyde de silicium colloïdal en poids de la composition, et
i. 0,5 à 2,0 pour cent de stéarate de magnésium en poids de la composition.

14. Composition pharmaceutique selon la revendication 13, dans laquelle le mannitol et le lactose sont présents dans un rapport pondéral de 1 à 3.

15. Composition pharmaceutique selon la revendication 13 comprenant :
a. 2 pour cent de forme D de sel de calcium de vanzacaftor cristallin hydraté en poids de la composition,
b. 9,5 pour cent d'une dispersion solide en poids de la composition, la dispersion solide comprenant 80 pour cent de tezacaftor amorphe et 20 pour cent de HPMC en poids de la dispersion solide,
c. 29,8 pour cent d'une dispersion solide en poids de la composition, la dispersion solide comprenant 80 pour cent de deutivacaftor amorphe, 19,5 pour cent de HPMCAS et 0,5 pour cent de SLS en poids de la dispersion solide,
d. 12,1 pour cent de mannitol en poids de la composition,
e. 36,3 pour cent de lactose en poids de la composition,
f. 2,3 pour cent de sucralose en poids de la composition,
g. 6 pour cent de croscarmellose sodique en poids de la composition,
h. 1 pour cent de dioxyde de silicium colloïdal par poids de la composition, et
i. 1 pour cent de stéarate de magnésium en poids de la composition.

16. Composition pharmaceutique selon l'une quelconque des revendications 13 à 15, la forme posologique unitaire comprenant 0,1 à 12,7 mg de forme D de sel de calcium de vanzacaftor cristallin hydraté, 10 à 50 mg de tézacaftor amorphe, et 15 à 150 mg de deutivacaftor amorphe.

17. Composition pharmaceutique selon l'une quelconque des revendications 13 à 16, la forme posologique unitaire comprenant 4,2 mg de forme D de sel de calcium de vanzacaftor cristallin hydraté, 16 mg de tézacaftor amorphe, et 50 mg de deutivacaftor amorphe.

18. Composition pharmaceutique selon l'une quelconque des revendications 13 à 16, la forme posologique unitaire comprenant :
(a) 6,4 mg de forme D de sel de calcium de vanzacaftor cristallin hydraté, 24 mg de tézacaftor amorphe et 75 mg de deutivacaftor amorphe ;
(b) 8,5 mg de forme D de sel de calcium de vanzacaftor cristallin hydraté, 32 mg de tézacaftor amorphe et 100 mg de deutivacaftor amorphe ; ou
(a) 12,7 mg de forme D de sel de calcium de vanzacaftor cristallin hydraté, 48 mg de tézacaftor amorphe et 150 mg de deutivacaftor amorphe.

19. Composition pharmaceutique selon l'une quelconque des revendications 1 à 18, la forme posologique unitaire comprenant :
(a) de 18 à 60 granules ; ou
(b) 30 granules.

20. Composition pharmaceutique selon l'une quelconque des revendications 1 à 19, le granule ayant une forme qui est de type cylindrique, ovale, conique, sphérique, elliptique, polygonale ou des combinaisons de celles-ci, le granule ayant comme plus grande dimension ou diamètre une longueur de 1,5 mm à 4,0 mm, facultativement une longueur de 2 mm.

21. Composition pharmaceutique selon l'une quelconque des revendications 1 à 20, pour une utilisation dans une méthode de traitement ou de diminution de la sévérité d'une maladie médiée par CFTR chez un patient pédiatrique, la maladie médiée par CFTR étant la mucoviscidose.

22. Composition pharmaceutique selon la revendication 21, la composition pharmaceutique étant administrée une fois par jour :

23. Composition pharmaceutique selon la revendication 21 ou la revendication 22, le patient pesant :
(a) au moins 14 kilogrammes ; ou
(b) moins de 14 kilogrammes.

24. Composition pharmaceutique selon l'une quelconque des revendications 21 à 23, le patient :
(a) présentant au moins une mutation choisie parmi 3141del9, E822K, G1069R, L967S, R117L, S912L, 546insCTA, F191V, G1244E, L997F, R117P, S945L, A46D, F311del, G1249R, L1077P, R170H, S977F, A120T, F311L, G1349D, L1324P, R258G, S1159F, A234D, F508C, H139R, L1335P, R334L, S1159P, A349V, F508C;S1251N, H199Y, L1480P, R334Q, S1251N, A455E, F508del*, H939R, M152V, R347H, S1255P, A554E, F575Y, H1054D, M265R, R347L, T338I, A1006E, F1016S, H1085P, M952I, R347P, T1036N, A1067T, F1052V, H1085R, M952T, R352Q, T1053I, D110E, F1074L, H1375P, M1101K, R352W, V201M, D110H, F1099L, I148T, P5L, R553Q, V232D, D192G, G27R, I175V, P67L, R668C, V456A, D443Y, G85E, I336K, P205S, R751L, V456F, D443Y;G576A;R668C, G126D, I502T, P574H, R792G, V562I, D579G, G178E, I601F, Q98R, R933G, V754M, D614G, G178R, I618T, Q237E, R1066H, V1153E, D836Y, G194R, I807M, Q237H, R1070Q, V1240G, D924N, G194V, I980K, Q359R, R1070W, V1293G, D979V, G314E, I1027T, Q1291R, R1162L, W361R, D1152H, G463V, I1139V, R31L, R1283M, W1098C, D1270N, G480C, I1269N, R74Q, R1283S, W1282R, E56K, G551D, I1366N, R74W, S13F, Y109N, E60K, G551S, K1060T, R74W;D1270N, S341P, Y161D, E92K, G576A, L15P, R74W;V201M, S364P, Y161S, E116K, G576A;R668C, L165S, R74W;V201M;D1270N, S492F, Y563N, E193K, G622D, L206W, R75Q, S549N, Y1014C, E403D, G628R, L320V, R117C, S549R, Y1032C, E474K, G970D, L346P, R117G, S589N, E588V, G1061R, L453S, R117H, S737F ; où D443Y;G576A;R668C, F508C;S1251N, G576A;R668C, R74W;D1270N, R74W;V201M et R74W;V201M;D1270N sont des mutations complexes ou composées dans lesquelles un allèle unique du gène CFTR présente des mutations multiples, qui existent indépendamment de la présence de mutations sur l'autre allèle ; ou
(b) étant homozygote pour la mutation F508del du gène CFTR ; ou
(c) ayant un génotype CFTR hétérozygote et présentant une mutation F508del.
